# EUROPEAN PATENT APPLICATION

(11) **EP 4 180 445 A1**
(43) Date of publication of application: **17.05.2023**
(21) Application number: 21838780.1
(22) Date of filing: 05.07.2021
(51) Int. Cl.: C07J 63/00, A61K 31/58, A61P 31/16, A61P 3/04, A61P 1/16, A61P 3/00

(54) **PENTACYCLIC TRITERPENOID GLYCOSIDE COMPOUND, AND PREPARATION METHOD THEREFOR AND USE THEREOF**

(30) Priority: 07.07.2020 CN 202010647960
(71) Applicant: Shanghai Institute of Materia Medica, Chinese Academy of Sciences, Pudong, Shanghai 201203 (CN)
(72) Inventor: LIU, Hong, Shanghai 201203 (CN); WANG, Jiang, Shanghai 201203 (CN); LIU, Yichu, Shanghai 201203 (CN); YU, Changyue, Shanghai 201203 (CN); WANG, Yibing, Shanghai 201203 (CN); JIANG, Hualiang, Shanghai 201203 (CN); CHEN, Kaixian, Shanghai 201203 (CN)
(74) Representative: Lavoix
(86) International application number: PCT/CN2021/104599
(87) International publication number: WO 2022/007765

(57) **Abstract**

The present invention provides a pentacyclic triterpenoid glycoside compound, and a preparation method therefor and a use thereof. Specifically, the present invention provides a compound as shown in formula I. The definition of each group is described in the description. The compound can be used for preparing a medicament for treating metabolic diseases such as diabetes and viral diseases caused by influenza virus, coronavirus and the like.

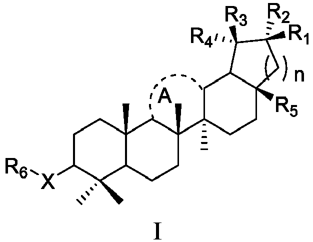

## Description

### TECHNICAL FIELD

The invention relates to the field of pharmaceutical chemistry and medicine, and specifically to a class of pentacyclic triterpenoid glycoside compound, preparation method therefor, and the use thereof in treating metabolic diseases and anti-virus, in particular to the use in the preparation of medicine for treating diabetes, influenza, pneumonia induced by coronavirus and other diseases.

### BACKGROUND OF THE INVENTION

Diabetes Mellitus (DM) is a chronic, systemic and metabolic disease caused by the longterm joint action of genetic factors and environmental factors. It is characterized by an increase in plasma glucose levels. It is a disease that affects normal physiological activities due to the metabolic disorder of sugar, fat and protein caused by insulin secretion insufficiency or dysfunction of insulin (insulin resistance). The complications of diabetes can be classified into acute complications and chronic complications. Among them, acute complications include diabetes ketoacidosis, diabetes hyperosmolar coma, various acute infections and lactic acidosis, etc.. In addition, hypoglycemia during the treatment of diabetes is also one of the most common acute complications. Chronic complications include diabetes ophthalmopathy, diabetes nephropathy, diabetes psychosis, diabetes cardio cerebral limb macro angiopathy, diabetes foot and skin lesions, etc. The main clinical manifestations of diabetes are polydipsia, polyuria, polyphagia and weight loss.

Diabetes is classified into insulin-dependent diabetes mellitus (IDDM, or type I diabetes) and non insulin-dependent diabetes mellitus, which are the most common, accounting for more than 90% of diabetes patients. The exact etiology and pathogenesis of type I diabetes are still not very clear. The etiology is jointly involved by genetic and environmental factors, mainly due to the damage of pancreatic islet b cells in the body, which leads to the inability to produce insulin in the body. Patients need insulin injection every day to control the insulin level in their blood. Type II diabetes is a kind of metabolic syndrome due to inability to control the blood glucose level in the body. Its main characteristics are hyperglycemia, insulin resistance and insulin secretion deficiency. The main etiology of type II diabetes is insulin resistance, which makes the body unable to use insulin effectively, or the reduction of insulin secretion make the insulin level unable meet the need of body. Because the patients with this type of diabetes can secrete insulin, they generally do not need to use insulin treatment. Diet adjustment or oral hypoglycemic drugs are enough to control blood glucose level.

Pentacyclic triterpenoid compounds are secondary plant metabolites found in a variety of plant organs, and the content of which in some species is up to 30% of their dry weight. Although the molecular mechanism is still unclear, triterpenoid compounds are generally considered as important components of plant defense systems against pathogens and herbivores. It has been reported that some glycoside pentacyclic triterpenoid compound have the same or higher *in vitro* anti influenza virus activity as oseltamivir. Mechanism studies show that these compounds closely bind to hemagglutinin (HA) protein, thus destroying the interaction between hemagglutinin and sialic acid receptor and preventing viruses from entering host cells. Such process hardly lead to drug resistance. At the same time, studies have shown that pentacyclic triterpenoid compounds can bind to the N protein of the coronavirus, prevent the assembly of the virus, and achieve the anti-coronavirus effect. At the same time, the N protein of coronavirus is highly conservative and plays an important role in immunity. Therefore, compounds targeting N protein are potential antiviral small molecule compounds.

For saccharide medicine, oxygen glycosides and nitrogen glycosides are easy to be oxidized and metabolized *in vivo,* while carbon glycosides show good metabolic stability both *in vivo* and *in vitro,* which makes carbon glycosides widely concerned by scientists. Therefore, pentacyclic triterpenoid carbon glycoside compounds have a very bright prospect in the research and development of hypoglycemic drugs and antiviral drugs.

In conclusion, there is an urgent need to develop new pentacyclic triterpenoid carbon glycosides in this field.

### SUMMARY OF THE INVENTION

The object of the present invention is to provide a pentacyclic triterpenoid carbon glycoside derivative compound shown in general formula I or pharmaceutically acceptable salts, racemates, R-isomers or S-isomers thereof, or the mixtures thereof.

The first aspect of the invention provides a pentacyclic triterpenoid carbon glycoside derivative compound with the structure shown in the following general formula I, or racemates, R-isomers, S-isomers, pharmaceutically acceptable salts thereof, or the mixtures thereof: 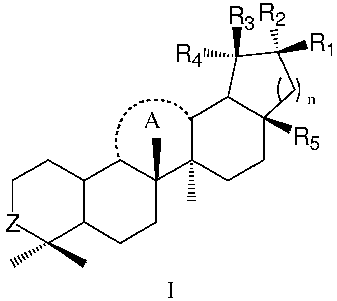 wherein,
ring A is selected from the group consisting of 6-membered saturated carbon ring or unsaturated carbon ring;
R₁, R₂ and R₃ are each independently selected from the group consisting of hydrogen, and methyl; R₄ is selected from the group consisting of hydrogen, and isopropenyl;
Rs is selected from the group consisting of hydrogen, deuterium, tritium, halogen, cyano, amino, nitro, hydroxyl, sulfydryl, aldehyde group, carboxyl, sulfonyl, phosphate group, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, substituted or unsubstituted C₁-C₆ amido, and Y-R₇; wherein,
Y is selected from the group consisting of -(CH₂)ₘCHR₉-, carbonyl, -CONH-, and -COO-;
wherein m is 0 or 1;
R₇ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₂-C₁₀ ester group, substituted or unsubstituted C₅-C₉ furanosyl, and substituted or unsubstituted C₅-C₉ pyranosyl;
Z is selected from the group consisting of carbonyl, -CH(R₉)₂, C=N-Rs, C=N-NH-Rs, and -X-R₆;
Rs is selected from the group consisting of hydrogen, deuterium, tritium, halogen, cyano, amino, nitro, hydroxyl, sulfydryl, aldehyde group, carboxyl, sulfonyl, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, and substituted or unsubstituted C₁-C₆ amido;
R₉ is selected from the group consisting of hydrogen, deuterium, tritium, halogen, cyano, amino, nitro, hydroxyl, and sulfydryl;
R₆ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
X is selected from the group consisting of -CHR₉-, carbonyl, S, -NHC(O)-R₈, -NHS(O)₂-R₈, - NHC(O)NH-R₈, - NHC(S)NH-R₈, -COO-, and -O-S(O)₂-R₈;
n is 1 or 2;
unless otherwise specified, the "substituted" in the above formulas means that the hydrogen atom on the corresponding group, or the hydroxyl group on the glycosyl ring is substituted by one or more substituents selected from the group consisting of deuterium, tritium, halogen, hydroxyl, carboxyl, sulfydryl, benzyl, C₁-C₁₂ alkoxycarbonyl, C₁-C₆ aldehyde group, amino, C₁-C₆ amide, nitro, cyano, unsubstituted or halogenated C₁-C₆ alkyl, C₂-C₁₀ alkenyl, C₁-C₆ alkoxy, C₁-C₆ alkyl-amino, C₆-C₁₀ aryl, five or six membered heteroaryl, five or six membered non-aromatic heterocyclyl, -O-(C₆-C₁₀ aryl), -O-(five or six membered heteroaryl), C₁-C₁₂ alkaminocarbonyl, substituted or unsubstituted C₂-C₁₀ acyl, sulfonyl (-SO₂-OH), phosphoryl (-PO₃-OH), C₅-C₉ furanosyl, and C₅-C₉ pyranosyl.

In another preferred embodiment, the compound of formula I has the structure shown in the following general formula II: 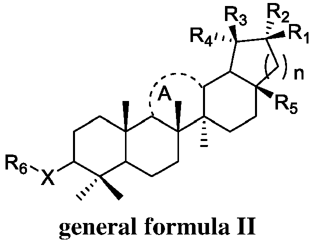 wherein,
Rs is selected from the group consisting of hydrogen, deuterium, tritium, halogen, cyano, amino, nitro, hydroxyl, sulfydryl, aldehyde group, carboxyl, sulfonyl, phosphate group, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, and substituted or unsubstituted C₁-C₆ amido;
X is selected from the group consisting of -CHR₉-, and carbonyl;
R₆ is selected from the group consisting of substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
R₉ is selected from the group consisting of hydrogen, deuterium, tritium, halogen, cyano, amino, nitro, hydroxyl, and sulfydryl;
ring A is selected from the group consisting of 6-membered saturated carbon ring or unsaturated carbon ring;
n is 1 or 2.

In another preferred embodiment, the compound of formula I has the structure shown in the following general formula III: 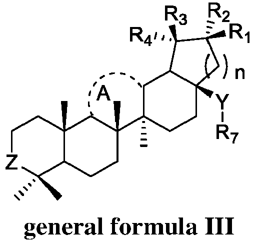 wherein,
R₁, R₂ and R₃ are each independently selected from the group consisting of hydrogen, and methyl;
R₄ is selected from the group consisting of hydrogen, and isopropenyl;
R₇ is selected from the group consisting of hydrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
Y is selected from the group consisting of -(CH₂)ₘCHR₉-, and carbonyl;
m is 0 or 1;
R₉ is selected from the group consisting of hydrogen, deuterium, tritium, halogen, cyano, amino, nitro, hydroxyl, and sulfydryl;
Z is selected from the group consisting of carbonyl, -CH(R₉)₂, =N-R₈, and -X-R₆;
R₉ is selected from the group consisting of hydrogen, halogen, cyano, amino, nitro, hydroxyl, and sulfydryl;
X is selected from the group consisting of -CHR₉-, carbonyl, S, -NHC(O)-R₈, -NHS(O)₂-R₈, - NHC(O)NH-R₈, -NHC(S)NH-R₈, -COO-, and -O-S(O)₂-R₈;
R₆ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
ring A is selected from the group consisting of 6-membered saturated carbon ring or unsaturated carbon ring;
n is 1 or 2.

In another preferred embodiment, the compound of formula I has the structure shown in the following general formulas IV, V, and VI:

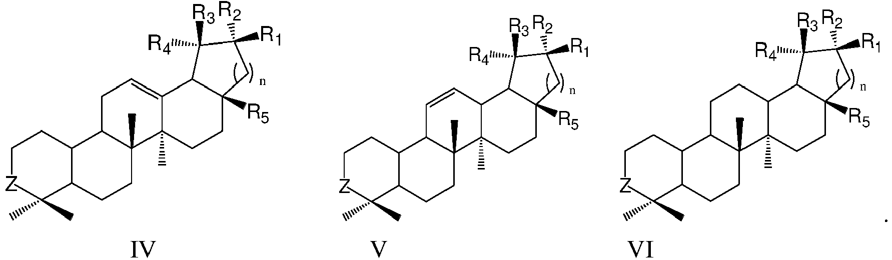

In another preferred embodiment, R₅ is selected from the group consisting of hydrogen, halogen, hydroxyl, carboxyl, substituted or unsubstituted C₁-C₆ alkyl, substituted or unsubstituted C₁-C₆ alkoxy, substituted or unsubstituted C₂-C₁₀ acyl, substituted or unsubstituted C₂-C₁₀ ester group, and substituted or unsubstituted C₁-C₆ amido.

In another preferred embodiment, R₉ is selected from the group consisting of hydrogen, hydroxyl, aldehyde group, sulfydryl, and -X-R₆;
X is selected from the group consisting of -CHR₉-, carbonyl, S, -NHC(O)-R₈, -NHS(O)₂-R₈, - NHC(O)NH-R₈, -NHC(S)NH-R₈, -COO-, and -O-S(O)₂-R₈;
R₆ is selected from the group consisting of substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl.

In another preferred embodiment, the pentacyclic triterpenoid carbon glycoside compounds are the compounds described in the example.

The second aspect of the invention provides a preparation method of the compound according to the first aspect of the invention, wherein the preparation method includes the following scheme 1 or scheme 2: 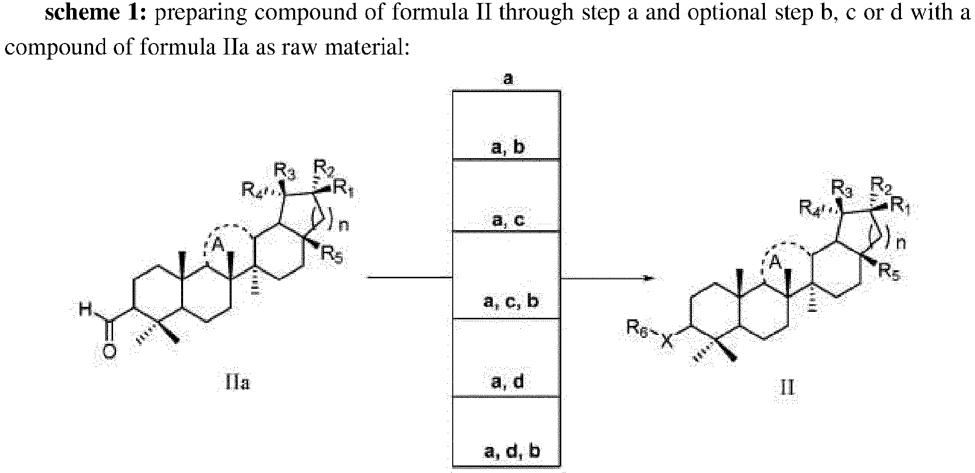 wherein, each group is defined as described in the first aspect of the invention;
step a: reacting compound IV with compound III to obtain the product of step a;
step b: reducing the product of step a to obtain the product of step b;
step c: alkylating the product of step b to obtain the product of step c;
step d: reacting the product of step c with Des Martin reagent to obtain a compound of formula II;
wherein the structure of compound IV is:

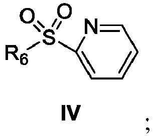

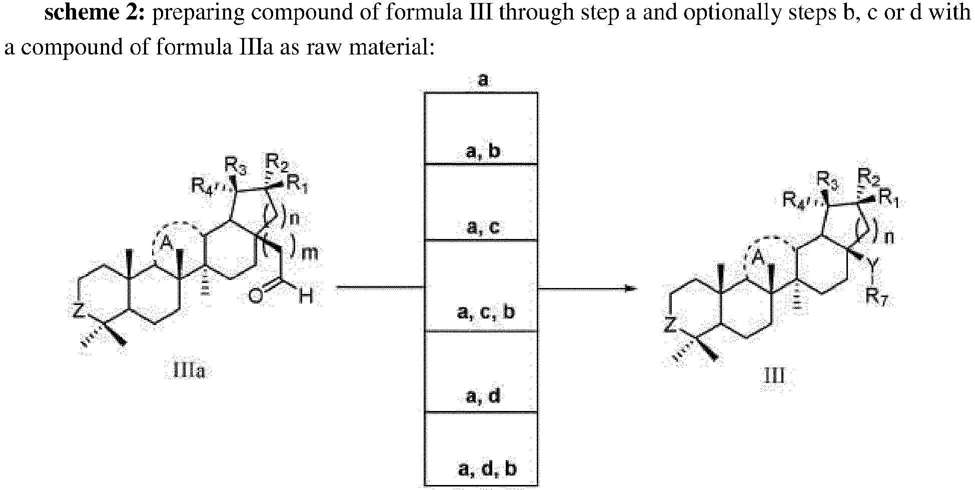

The third aspect of the invention provides a pharmaceutical composition, which contains the therapeutically effective amount of one or more of the compound of formula I, or pharmaceutical salts, racemates, R-isomers and S-isomers thereof according to the first aspect of the invention, as well as one or more pharmaceutical carriers, excipients, adjuvants, accessories and/or diluents.

The fourth aspect of the invention provides a use of a compound of formula I and formula II, racemates, R-isomers, S-isomers or pharmaceutical salts thereof according to the first aspect of the invention for preparing a medicament for treating or preventing metabolic diseases related to diabetes and viral diseases; preferably, the diseases are selected from the group consisting of diabetes, influenza, obesity, liver fibrosis, metabolic diseases, and viral diseases.

It should be understood that, within the scope of the present invention, the above technical features of the present invention and the technical features specifically described in the following descriptions (such as the examples) can be combined with each other to form a new or preferred technical solution. Due to space limitations, they will not be repeated herein.

### BRIEF DESCRIPTION OF THE DRAWINGS

Figure 1 shows the OGTT experimental data of ICR mice of A2, A4, A6, A10, A25, A43, A44, A47 and A67.
Figure 2 shows good *in vivo* hypoglycemic effect of A25 and A43.
Figure 3 shows the EC₅₀ and the *in vitro* effect of promoting GLP secretion of A64 and the positive drug ANT777.
Figure 4 shows that the compounds of the invention show equivalent virus inhibition rate and low cytotoxicity to the positive drug ribavirin, of which the virus inhibition rate is similar to that of the positive drug ribavirin, but the cytotoxicity is lower than that of ribavirin.
Figure 5 shows the experimental data of the affinity to SARS Cov-2 N protein of A102, A104, A106, A108, A112, A114, A116, A117, A120, A121, A122, A123, A124, A125, A126, and A127.
Figure 6 shows the experimental data of the affinity of compounds A102, A114 and A116 to N protein.
Figures 7 and 8 show the SARS Cov-2 virus replication inhibition experimental data of A102, A104, A114, A117, A118, A119, A120, A121, A122, A123, A124, A125, A126, A127, A128, and A129.
Figures 9 and 10 show the good PK properties of compound A104 in mice.

### DETAILED DESCRIPTION OF THE INVENTION

After extensive and in-depth research, the inventor accidentally discovered a class of pentacyclic triterpenoid carbon glycoside compounds with novel structure and excellent oral hypoglycemic activity or antiviral activity for the first time. On this basis, the invention is completed.

### Definitions

In the present invention, the halogen refers to F, Cl, Br, and I.

In the invention, unless otherwise specified, the terms used have the general meaning known to those skilled in the art.

In the invention, the term "C1-C6 alkyl" refers to linear or branched alkyl group with 1 to 6 carbon atoms, including methyl, ethyl, propyl, isopropyl, butyl, isobutyl, sec-butyl, *tert-butyl,* pentyl and hexyl etc.; preferably ethyl, propyl, isopropyl, butyl, isobutyl, sec-butyl and *tert-butyl.*

In the invention, the term "C1-C6 alkoxy" refers to linear or branched alkoxy group with 1 to 6 carbon atoms, including methoxy, ethoxy, propoxy, isopropoxy, butoxy, etc..

In the invention, the term "C2-C6 alkenyl" refers to linear or branched alkenyl group containing a double bond with 2 to 6 carbon atoms, including vinyl, propenyl, butenyl, isobutenyl, pentenyl, hexenyl, etc. unlimitedly.

In the present invention, the term "C2-C6 alkynyl" refers to a linear or branched alkynyl group containing one triple bond with 2 to 6 carbon atoms, including acetynyl, proynyl, butyryl, isobutyryl, pentynyl, hexynyl etc. unlimitedly.

In the present invention, the term "C3-C10 cycloalkyl" refers to cycloalkyl group with 3 to 10 carbon atoms on the ring, including cyclopropyl, cyclobutyl, cyclopentyl, cyclohexyl, cycloheptyl, cyclooctyl, cyclodecyl, etc. unlimitedly. The terms "C3-C8 cycloalkyl", "C3-C7 cycloalkyl" and "C3-C6 cycloalkyl" have similar meanings.

In the present invention, the term "C3-C10 cycloalkenyl" refers to cycloalkenyl group with 3 to 10 carbon atoms on the ring, including cyclopropenyl, cyclobutenyl, cyclopentenyl, cyclohexenyl, cyclohexenyl, cyclooctenyl, cyclodecyl, etc. unlimitedly. The term "C3-C7 cycloalkenyl" has a similar meaning.

In the invention, the term "C1-C12 alkoxycarbonyl" refers to alkoxycarbonyl group with 1 to 12 carbon atoms on the alkyl chain, including methoxycarbonyl, ethoxycarbonyl, propoxycarbonyl, isopropoxycarbonyl, tert-butoxycarbonyl, benzyloxycarbonyl, etc. unlimitedly.

In the present invention, the term "C1-C12 alkaminocarbonyl" refers to alkaminocarbonyl group with 1 to 12 carbon atoms on the alkyl chain, including methylaminocarbonyl, ethylaminocarbonyl, propylaminocarbonyl, isopropylaminocarbonyl, tert-butylaminocarbonyl, benzylaminocarbonyl, dimethylaminocarbonyl, etc. unlimitedly.

In the present invention, the term "C5-C9 furanosyl" refers to furanosyl group with 5 to 9 carbon atoms, wherein the 1 position of the glycosyl group is connected to the main chain, including ribofuranosyl, desoxyribofuranosyl, galactofuranosyl, etc. unlimitedly.

In the present invention, the term "C5-C9 pyranosyl" refers to a pyranosyl with 5 to 9 carbon atoms, wherein the 1 position of the glycosyl group is connected to the main chain, including glucopyranosyl, glucopyranuronyl, rhamnopyranosyl, galactopyranosyl, mannopyranosyl, xylpyranosyl, etc.

In the present invention, the terms "aromatic ring" or "aryl" have the same meaning, preferably "aryl" is "C6-C12 aryl" or "C6-C10 aryl". The term "C6-C12 aryl" refers to aromatic cylic group with 6 to 12 carbon atoms without heteroatoms on the ring, such as phenyl, naphthyl, etc. The term "C6-C10 aryl" has a similar meaning.

In the present invention, the terms "aromatic heterocyclic ring" or "heteroaryl" have the same meaning, which refer to heteroaromatic group containing one to more heteroatoms. The heteroatoms referred herein include oxygen, sulfur and nitrogen. For example, furanyl, thiophenyl, pyridinyl, pyrazolyl, pyrrolyl, N-alkyl pyrrolyl, pyrimidinyl, pyrazinyl, imidazolyl, tetrazolyl, etc. The heteroaryl ring can be fused on aryl, heterocyclic ring or cycloalkyl ring, wherein the ring connected with the parent structure is the heteroaryl ring. The heteroaryl group may be optionally substituted or unsubstituted.

In the present invention, the term "3-12 membered heterocyclyl" refers to the saturated or unsaturated 3-12 membered cyclic group containing 1-3 heteroatoms selected from oxygen, sulfur and nitrogen on the ring, such as dioxolanyl, etc. The term "3-7 membered heterocyclyl" has a similar meaning.

In the present invention, the term "substituted" means that one or more hydrogen atoms on a specific group are substituted by a specific substituent. The specific substituent is the substituent described correspondingly in the preceding text, or the substituent in each example. Unless otherwise specified, a substituented group can have substituent selected from a specific group at any substitutable site of the group, and the substituents can be the same or different in each position. A cyclic substituent, such as heterocyclyl, can be attached to another ring, such as a cycloalkyl, to form a spiro bicyclic system, for example, two rings have a common carbon atom. Those skilled in the art should understand that the combinations of substituents expected by the invention are those stable or chemically achievable. The substituents are, for example (but not limited to) C1-8 alkyl, C2-8 alkenyl, C2-8 alkynyl, C3-8 cycloalkyl, 3- to 12- membered heterocyclyl, aryl, heteroaryl, halogen, hydroxyl, carboxyl(-COOH), C1-8 aldehyde group, C2-10 acyl, C2-10 ester group, C1-C12 alkoxycarbonyl, amino, alkoxy, C1-10 sulfonyl, etc.

### Pentacyclic triterpenoid carbon glycoside compound

The invention provides a pentacyclic triterpenoid carbon glycoside derivative compound with the structure shown in the following general formula I or general formula II, or racemates, R-isomers, S-isomers, pharmaceutically acceptable salts thereof or the mixtures thereof: 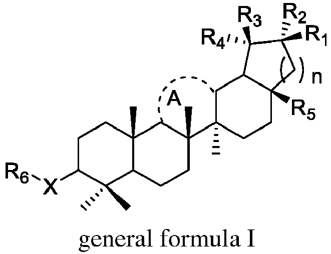 wherein,
R₁, R₂ and R₃ are each independently selected from the group consisting of hydrogen, and methyl; R₄ is selected from the group consisting of hydrogen, and isopropenyl;
R₅ is selected from the group consisting of hydrogen, deuterium, tritium, halogen, cyano, amino, nitro, hydroxyl, sulfydryl, aldehyde group, carboxyl, sulfonyl, phosphate group, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, substituted or unsubstituted C₁-C₆ amido;
R₆ is selected from the group consisting of substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
X is selected from the group consisting of -CHR₉-, and carbonyl;
R₉ is selected from the group consisting of hydrogen, deuterium, tritium, halogen, cyano, amino, nitro, hydroxyl, and sulfydryl;
ring A is selected from the group consisting of 6-membered saturated carbon ring or unsaturated carbon ring;
n is 1 or 2. 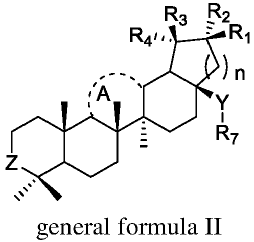
wherein,
R₁, R₂ and R₃ are each independently selected from the group consisting of hydrogen, and methyl; R₄ is selected from the group consisting of hydrogen, and isopropenyl;
R₇ is selected from the group consisting of substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
Y is selected from the group consisting of -(CH₂)ₘCHR₉-, and carbonyl;
m is 0 or 1;
R₉ is selected from the group consisting of hydrogen, deuterium, tritium, halogen, cyano, amino, nitro, hydroxyl, and sulfydryl;
Z is selected from the group consisting of carbonyl, -CH(R₈)₂, =N-Rs;
R₈ is selected from the group consisting of hydrogen, deuterium, tritium, halogen, cyano, amino, nitro, hydroxyl, sulfydryl, aldehyde group, carboxyl, sulfonyl, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, and substituted or unsubstituted C₁-C₆ amido;
ring A is selected from the group consisting of 6-membered saturated carbon ring or unsaturated carbon ring;
n is 1 or 2.

In a more preferred embodiment of the invention, the compounds of general formula I and general formula II of the invention are preferably the following specific compounds:

| Number | Structure | Name |
|---|---|---|
| A1 | 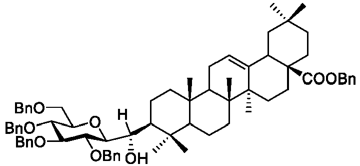 | (3S,5S,8R,9R,10S,14R,17R,18S)-28-O-Benzyloleanolicacid-3-(1'R)-(hydroxyl)methyl-2",3",4",6"-O-tetrabenzyl-β-D-glucopyranoside |
| A2 | 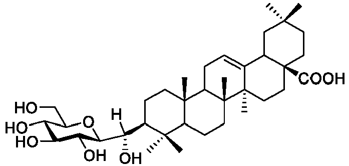 | (3S,5S,8R,9R,10S,14R,17R,18S)-Oleanolicacid-3-(1'R)-(hydroxyl)methyl-β-D-glucopyranoside |
| A3 | 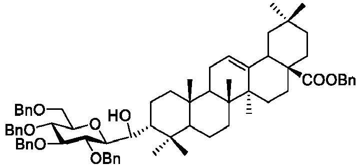 | (3R,5S,8R,9R,10S,14R,17R,18S)-28-O-Benzyloleanolicacid-3-(1'R)-(hydroxyl)methyl-2",3",4",6"-O-tetrabenzyl-β-D-glucopyranoside |
| A4 | 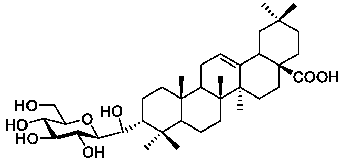 | (3R,5S,8R,9R,10S,14R,17R,18S)-Oleanolicacid-3-(1''R)-(hydroxyl)methyl-β-D-glucopyranoside |
| A5 | 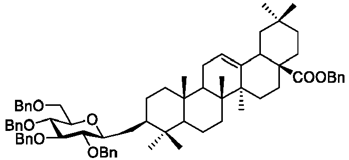 | (3S,5S,8R,9R,10S,14R,17R,18S)-28-O-Benzyloleanolicacid-3-methyl-2",3",4",6"-O-tetrabenzyl-β-D-glucopyranoside |
| A6 | 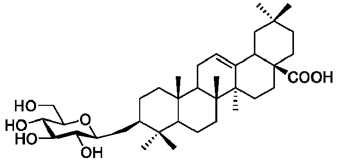 | (3S,5S,8R,9R,10S,14R,17R,18S)-Oleanolicacid-methyl-β-D-glucopyranoside |
| A7 | 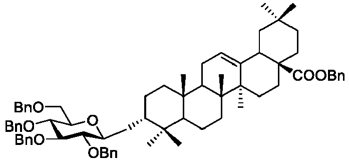 | (3R,5S,8R,9R,10S,14R,17R,18S)-28-O-Benzyloleanolicacid-3-methyl-2", 3 ",4", 6"-O-tetrabenzyl-β-D-glucopyranoside |
| A8 | 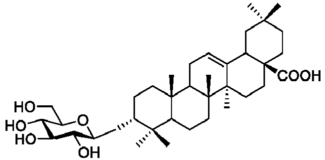 | (3R,5S,8R,9R,10S,14R,17R,18S)-Oleanolicacid-3-methyl-β-D-glucopyranoside |
| A9 | 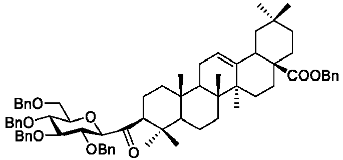 | (3S,5S,8R,9R, 10S, 14R, 17R, 18S)-28-O-Benzyloleanolicacid-3-carbonyl-2",3",4",6"-O-tetrabenzyl-β-D-glucopyranoside |
| A10 | 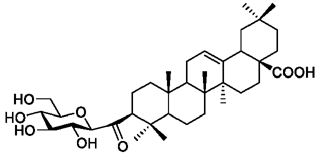 | (3S,5S,8R,9R,10S,14R,17R,18S)-Oleanolicacid-3-carbonyl-β-D-glucopyranoside |
| A11 | 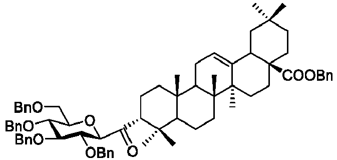 | (3R,5S,8R,9R,10S,14R,17R,18S)-28-O-Benzyloleanolicacid-3-carbonyl-2",3",4",6"-O-tetrabenzyl-β-D-glucopyranoside |
| A12 | 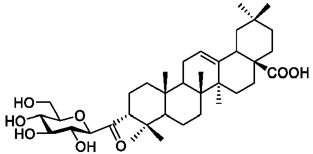 | (3R,5S,8R,9R,10S,14R,17R,18S)-Oleanolicacid-3-carbonyl-β-D-glucopyranoside |
| A13 | 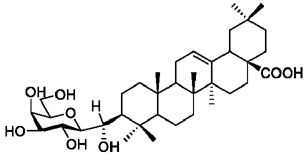 | (3S,5S,8R,9R,10S,14R,17R,18S)-Oleanolicacid-3-(1'R)-(hydroxyl)methyl-β-D-galactopyrano side |
| A14 | 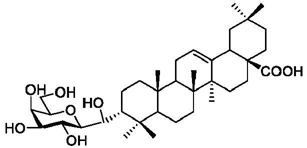 | (3R,5S,8R,9R,10S,14R,17R,18S)-Oleanolicacid-3-(1'R)-(hydroxyl)methyl-β-D-galactopyrano side |
| A15 | 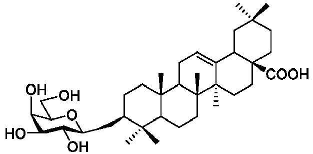 | (3S,5S,8R,9R,10S,14R,17R,18S)-Oleanolicacid-3-methyl-β-D-galactopyrano side |
| A16 | 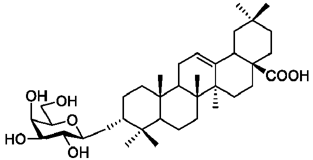 | (3R,5S,8R,9R,10S,14R,17R,18S)-Oleanolicacid-3-methyl-β-D-galactopyrano side |
| A17 | 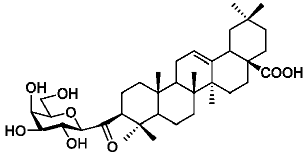 | (3S,5S,8R,9R,10S,14R,17R, 18S)-Oleanolicacid-3-carbonyl-β-D-galactopyrano side |
| A18 | 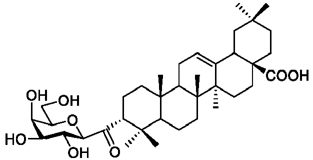 | (3R,5S,8R,9R,10S,14R,17R,18S)-Oleanolicacid-3-carbonyl-β-D-galactopyrano side |
| A19 | 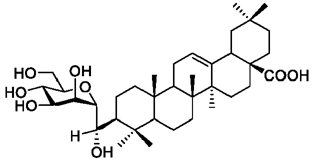 | (3S,5S,8R,9R,10S,14R,17R,18S)-Oleanolicacid-3-(1'S)-(hydroxyl)methyl-α-D-mannopyranoside |
| A20 | 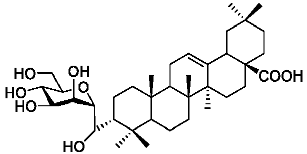 | (3R,5S,8R,9R,10S,14R,17R,18S)-Oleanolicacid-3-(1'S)-(hydroxyl)methyl-α-D-mannopyranoside |
| A21 | 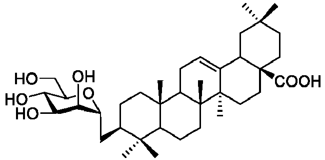 | (3S,5S,8R,9R,10S,14R,17R,18S)-Oleanolicacid-3-methyl-α-D-mannopyranoside |
| A22 | 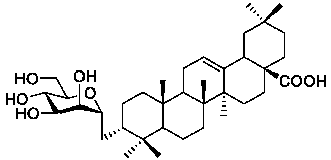 | (3R,5S,8R,9R,10S,14R,17R,18S)-Oleanolicacid-3-methyl-α-D-mannopyranoside |
| A23 | 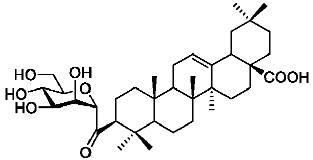 | (3S,5S,8R,9R,10S,14R,17R,18S)-Oleanolicacid-3-carbonyl-α-D-mannopyranoside |
| A24 | 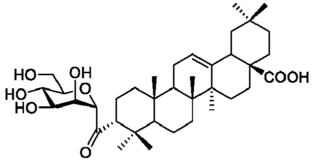 | (3R,5S,8R,9R,10S,14R,17R,18S)-Oleanolicacid-3-carbonyl-α-D-mannopyranoside |
| A25 | 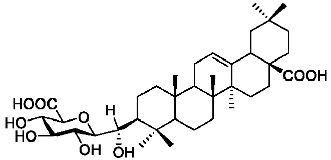 | (3S,5S,8R,9R,10S,14R,17R,18S)-Oleanolicacid-3-(1'R)-(hydroxyl)methyl-β-D-glucurouopyranoside |
| A26 | 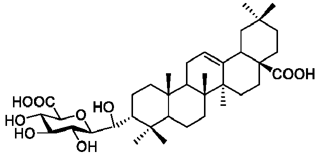 | (3R,5S,8R,9R,10S,14R,17R,18S)-Oleanolicacid-3-(1'R)-(hydroxyl)methyl-β-D-glucurouopyranoside |
| A27 | 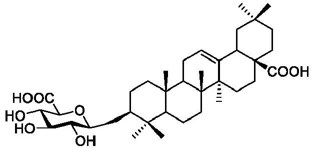 | (3S,5S,8R,9R,10S,14R,17R,18S)-Oleanolicacid-3-methyl-β-D-glucurouopyranoside |
| A28 | 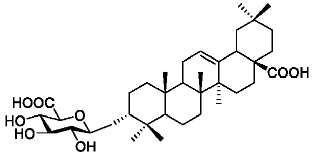 | (3R,5S,8R,9R,10S,14R,17R,18S)-Oleanolicacid-3-methyl-β-D-glucurouopyranoside |
| A29 | 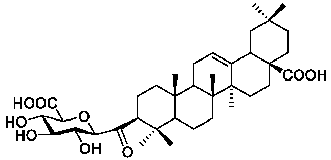 | (3S,5S,8R,9R,10S,14R,17R,18S)-Oleanolicacid-3-carbonyl-β-D-glucurouopyranoside |
| A30 | 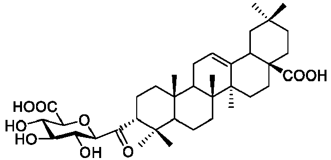 | (3R,5S,8R,9R,10S,14R,17R,18S)-Oleanolicacid-3-carbonyl-β-D-glucurouopyranoside |
| A31 | 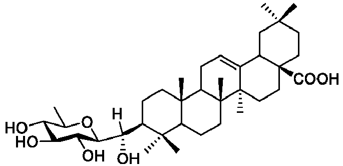 | (3S,5S,8R,9R,10S,14R,17R,18S)-Oleanolicacid-3-(1'R)-(hydroxyl)methyl-6"-deoxy-β-D-glucopyranoside |
| A32 | 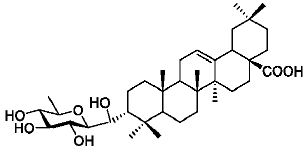 | (3R,5S,8R,9R,10S,14R,17R,18S)-Oleanolicacid-3-(1'R)-(hydroxyl)methyl-6"-deoxy-β-D-glucopyranoside |
| A33 | 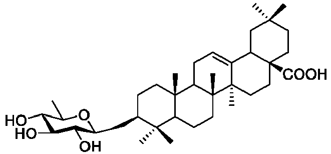 | (3S,5S,8R,9R,10S,14R,17R,18S)-Oleanolicacid-3-methyl-6"-deoxy-β-D-glucopyranoside |
| A34 | 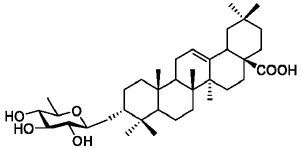 | (3R,5S,8R,9R,10S,14R,17R,18S)-Oleanolicacid-3-methyl-6"-deoxy-β-D-glucopyranoside |
| A35 | 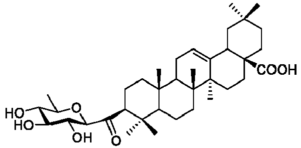 | (3S,5S,8R,9R,10S,14R,17R,18S)-Oleanolicacid-3-carbonyl-6"-deoxy-β-D-glucopyranoside |
| A36 | 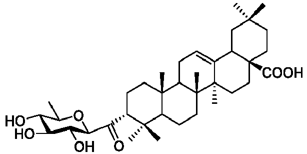 | (3R,5S,8R,9R,10S,14R,17R,18S)-Oleanolicacid-3-carbonyl-6"-deoxy-β-D-glucopyranoside |
| A37 | 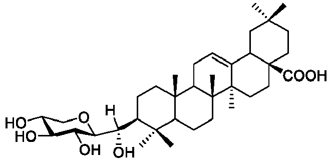 | (3S,5S,8R,9R,10S,14R,17R,18S)-Oleanolicacid-3-(1'R)-(hydroxyl)methyl-β-D-xylopyranoside |
| A38 | 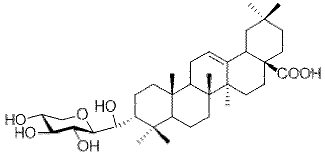 | (3R,5S,8R,9R,10S,14R,17R,18S)-Oleanolicacid-3-(1'R)-(hydroxyl)methyl-β-D-xylopyranoside |
| A39 | 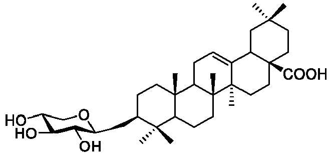 | (3S,5S,8R,9R,10S,14R,17R,18S)-Oleanolicacid-3-methyl-β-D-xylopyranoside |
| A40 | 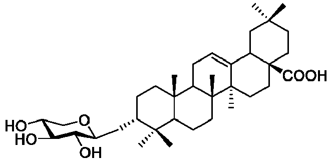 | (3R,5S,8R,9R,10S,14R,17R,18S)-Oleanolicacid-3-methyl-β-D-xylopyranoside |
| A41 | 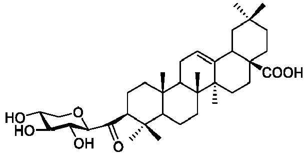 | (3S,5S,8R,9R,10S,14R,17R,18S)-Oleanolicacid-3-carbonyl-β**-**D-xylopyranoside |
| A42 | 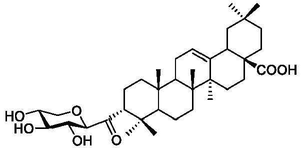 | (3R,5S,8R,9R,10S,14R,17R,18S)-Oleanolicacid-3-carbonyl-β-D-xylopyranoside |
| A43 | 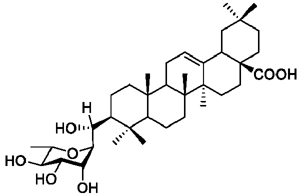 | (3S,5S,8R,9R,10S,14R,17R,18S)-Oleanolicacid-3-(1'S)-(hydroxyl)methyl-α-L-rhamnopyranoside |
| A44 | 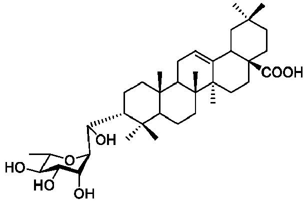 | (3R,5S,8R,9R,10S,14R,17R,18S)-Oleanolicacid-3-(1'S)-(hydroxyl)methyl-α-L-rhamnopyranoside |
| A45 | 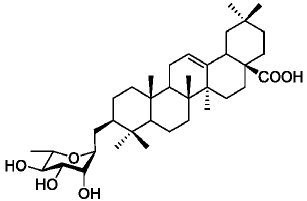 | (3S,5S,8R,9R,10S,14R,17R,18S)-Oleanolicacid-3-(1'S)-methyl-α-L-rhamnopyranoside |
| A46 | 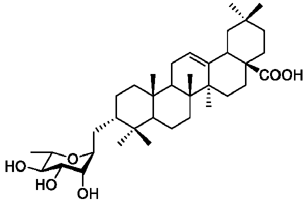 | (3R,5S,8R,9R,10S,14R,17R,18S)-Oleanolicacid-3-(1'S)-methyl-α-L-rhamnopyranoside |
| A47 | 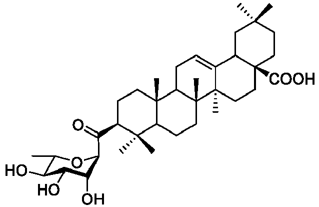 | (3S,5S,8R,9R,10S,14R,17R,18S)-Oleanolicacid-3-(1'S)-carbonyl-α-L-rhamnopyranoside |
| A48 | 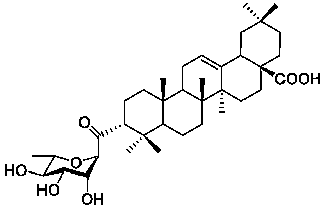 | (3R,5S,8R,9R,10S,14R,17R,18S)-Oleanolicacid-3-1'S)-carbonyl-α-L-rhamnopyranoside |
| A49 | 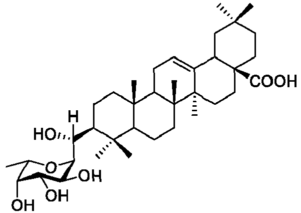 | (3S,5S,8R,9R,10S,14R,17R,18S)-Oleanolicacid-3-(1'S)-(hydroxyl)methyl-α-L-fucopyranoside |
| A50 | 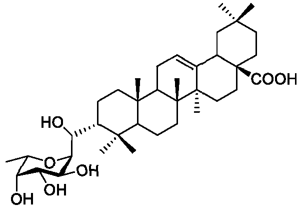 | (3R,5S,8R,9R,10S,14R,17R,18S)-Oleanolicacid-3-(1'S)-(hydroxyl)methyl-α-L-fucopyranoside |
| A51 | 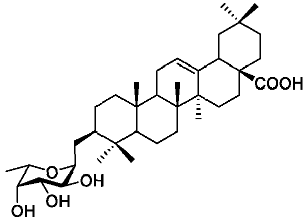 | (3S,5S,8R,9R,10S,14R,17R,18S)-Oleanolicacid-3-(1'S)-methyl-α-L-fucopyranoside |
| A52 | 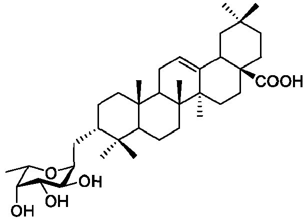 | (3R,5S,8R,9R,10S,14R,17R,18S)-Oleanolicacid-3-(1'S)-methyl-α-L-fucopyranoside |
| A53 | 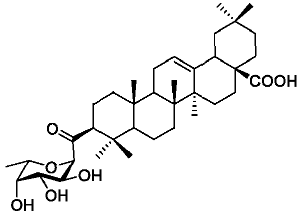 | (3S,5S,8R,9R,10S,14R,17R,18S)-Oleanolicacid-3-(1'S)-carbonyl-α-L-fucopyranoside |
| A54 | 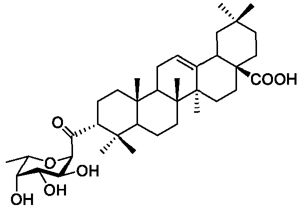 | (3R,5S,8R,9R,10S,14R,17R,18S)-Oleanolicacid-3-(1'S)-carbonyl-α-L-fucopyranoside |
| A55 | 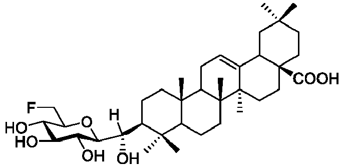 | (3S,5S,8R,9R,10S,14R,17R,18S)-Oleanolicacid-3-(1'R)-(hydroxyl)methyl-6"-deoxy-6"-fluoro-β-D-glucopyranoside |
| A56 | 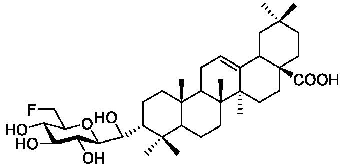 | (3R,5S,8R,9R,10S,14R,17R,18S)-Oleanolicacid-3-(1'R)-(hydroxyl)methyl-6"-deoxy-6"-fluoro-β-D-glucopyranoside |
| A57 | 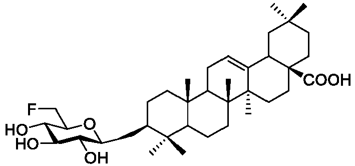 | (3S,5S,8R,9R,10S,14R,17R,18S)-Oleanolicacid-3-methyl-6"-deoxy-6"-fluoro-β-D-glucopyranoside |
| A58 | 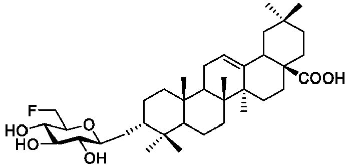 | (3R,5S,8R,9R,10S,14R,17R,18S)-Oleanolicacid-3-methyl-6"-deoxy-6"-fluoro-β-D-glucopyranoside |
| A59 | 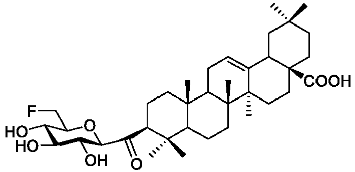 | (3S,5S,8R,9R,10S,14R,17R,18S)-Oleanolicacid-3-carbonyl-6"-deoxy-6"-fluoro-β-D-glucopyranoside |
| A60 | 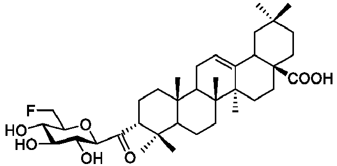 | (3R,5S,8R,9R,10S,14R,17R,18S)-Oleanolicacid-3-carbonyl-6"-deoxy-6"-fluoro-β-D-glucopyranoside |
| A61 | 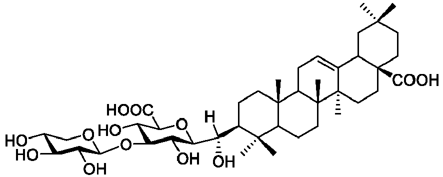 | (3S,5S,8R,9R,10S,14R,17R,18S)-Oleanolicacid-3-(1'R)-(hydroxyl)methyl-β-D-xylopyranosyl(1→3)-β-D-glucurouopyranoside |
| A62 | 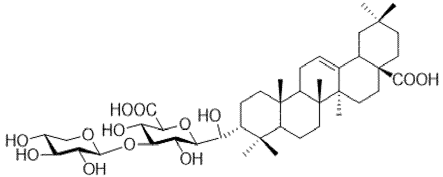 | (3R,5S,8R,9R,10S,14R,17R,18S)-Oleanolicacid-3-(1'R)-(hydroxyl)methyl-β-D-xylopyranosyl(1→3)-β-D-glucurouopyranoside |
| A63 | 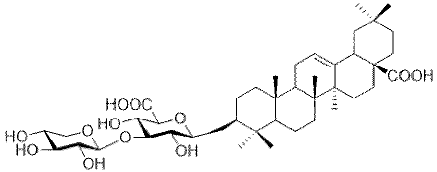 | (3S,5S,8R,9R,10S,14R,17R,18S)-Oleanolicacid-3-methyl-β-D-xylopyranosyl(1→3)-β-D-glucurouopyranoside |
| A64 | 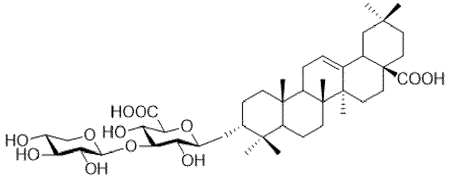 | (3R,5S,8R,9R,10S,14R,17R,18S)-Oleanolicacid-3-methyl-β-D-xylopyranosyl(1→3)-β-D-glucurouopyranoside |
| A65 | 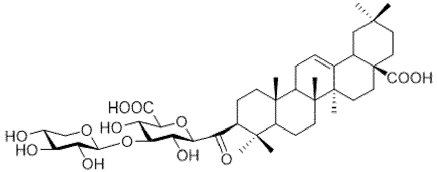 | (3S,5S,8R,9R,10S,14R,17R,18S)-Oleanolicacid-3-carbonyl-β**-**D-xylopyranosyl(1→3)-β-D-glucurouopyranoside |
| A66 | 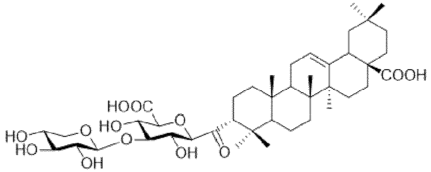 | (3R,5S,8R,9R,10S,14R,17R,18S)-Oleanolicacid-3-carbonyl-β-D-xylopyranosyl(1→3)-β-D-glucurouopyranoside |
| A67 | 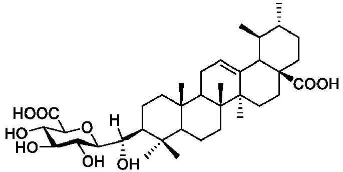 | (3S,5S,8R,9R,10S,14R,17R,18S,1 9S,20R)-Ursolicacid-3-(1'R)-(hydroxyl)methyl-(3-D-glucurouopyranoside |
| A68 | 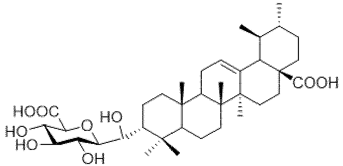 | (3R,5S,8R,9R,10S,14R,17R,18S,1 9S,20R)-Ursolicacid-3-(1'R)-(hydroxyl)methyl-β-D-glucurouopyranoside |
| A69 | 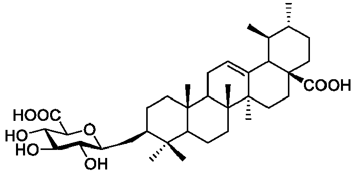 | (3S,5S,8R,9R,10S,14R,17R,18S,1 9S,20R)-Ursolicacid-3-methyl-β-D-glucurouopyranoside |
| A70 | 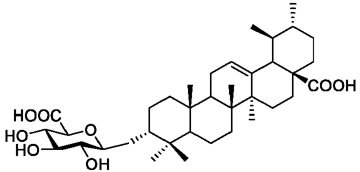 | (3R,5S,8R,9R,10S,14R,17R,18S,1 9S,20R)-Ursolicacid-3-methyl-β-D-glucurouopyranoside |
| A71 | 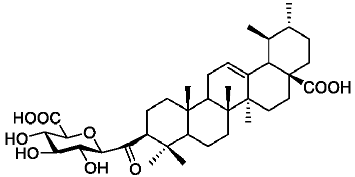 | (3S,5S,8R,9R,10S,14R,17R,18S,1 9S,20R)-Ursolicacid-3-carbonyl-β-D-glucurouopyranoside |
| A72 | 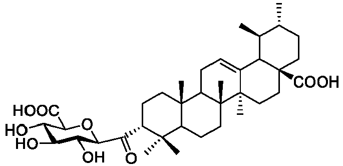 | (3R,5S,8R,9R,10S,14R,17R,18S,1 9S,20R)-Ursolicacid-3-carbonyl-β-D-glucurouopyranoside |
| A73 | 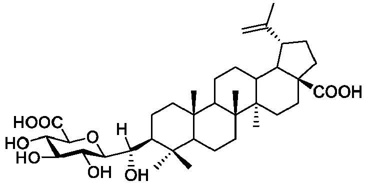 | (3S,5S,8R,9R,10S,13R,14R,17S,1 8R,19R)-Betulinicacid-3-(1'R)-(hydroxyl)methyl-β-D-glucurouopyranoside |
| A74 | 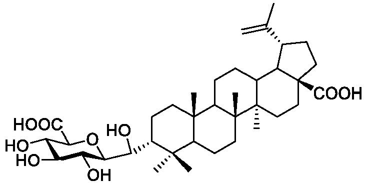 | (3S,5S,8R,9R,10S,13R,14R,17S,1 8R,19R)-Betulinicacid-3-(1'R)-(hydroxyl)methyl-β-D-glucurouopyranoside |
| A75 | 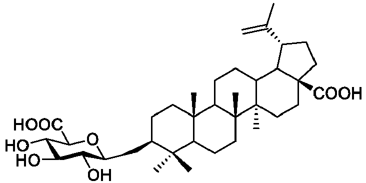 | (3S,5S,8R,9R,10S,13R,14R,17S,1 8R,19R)-Betulinicacid-3-methyl-β-D-glucurouopyranoside |
| A76 | 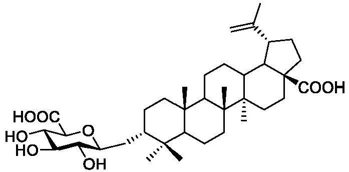 | (3S,5S,8R,9R,10S,13R,14R,17S,1 8R,19R)-Betulinicacid-3-methyl-β-D-glucurouopyranoside |
| A77 | 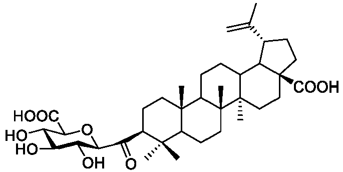 | (3S,5S,8R,9R,10S,13R,14R,17S,1 8R,19R)-Betulinicacid-3-carbonyl-β-D-glucurouopyranoside |
| A78 | 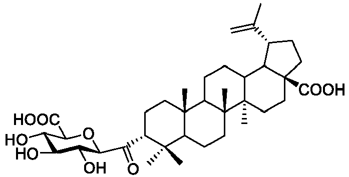 | (3S,5S,8R,9R,10S,13R,14R,17S,1 8R,19R)-Betulinicacid-3-carbonyl-β-D-glucurouopyranoside |
| A79 | 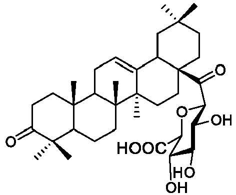 | (5S,8R,9R,10S,14R,17R,18S)-3-Carbonyloleanolicacid-28-β-D-glucurouopyranoside |
| A80 | 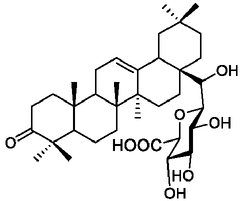 | (5S,8R,9R,10S,14R,17R,18S)-3-Carbonyloleanolicacid-17-(1'R)-(hydroxyl)methyl-β-D-glucurouopyranoside |
| A81 | 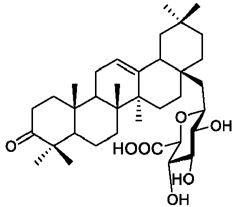 | (5S,8R,9R,10S,14R,17R,18S)-3-Carbonyloleanolicacid-17-methyl-β-D-glucurouopyranoside |
| A82 | 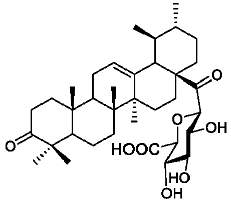 | (5S,8R,9R,10S,14R,17R,18S19S, 20R)-3-Carbonylursolicacid-28-β-D-glucurouopyranoside |
| A83 | 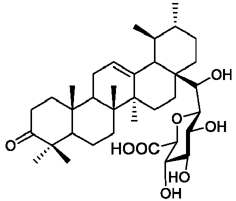 | (5S,8R,9R,10S,14R,17R,18S19S, 20R)-3-Carbonylursolicacid-17-(1'R)-(hydroxyl)methyl-β-D-glucurouopyranoside |
| A84 | 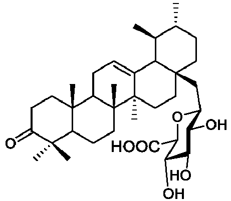 | (5S,8R,9R,10S,14R,17R,18S,19S, 20R)-3-Carbonylursolicacid-17-methyl-β-D-glucurouopyranoside |
| A85 | 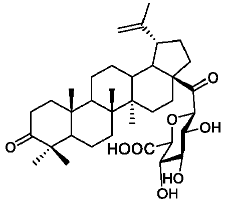 | (5S,8R,9R,10S,13R,14R,17S,18R, 19R)-3-Carbonylbetulinicacid-28-β-D-glucurouopyranoside |
| A86 | 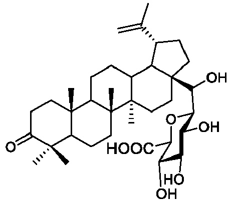 | (5S,8R,9R,10S,13R,14R,17S,18R, 19R)-3-Carbonylbetulinicacid-17-(1'R)-(hydroxyl)methyl-β-D-glucurouopyranoside |
| A87 | 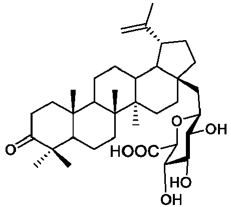 | (5S,8R,9R,10S,13R,14R,17S,18R, 19R)-3-Carbonylbetulinicacid-17-methyl-β-D-glucurouopyranoside |
| A88 | 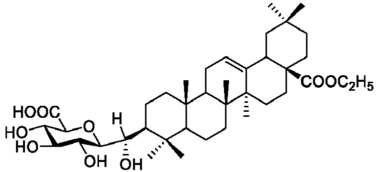 | (3S,5S,8R,9R,10S,14R,17R,18S)-28-O-Ethyloleanolicacid-3-(1'R)-(hydroxyl)methyl-β-D-glucurouopyranoside |
| A89 | 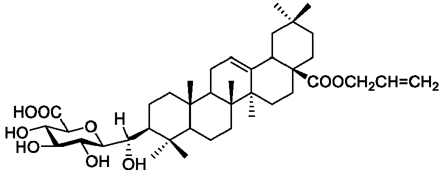 | (3S,5S,8R,9R,10S,14R,17R,18S)-28-O-Allyloleanolicacid-3-(1'R)-(hydroxyl)methyl-β-D-glucurouopyranoside |
| A90 | 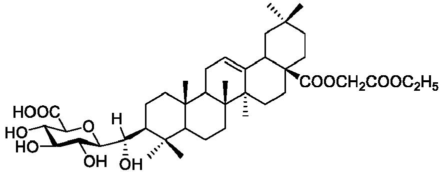 | (3S,5S,8R,9R,10S,14R,17R,18S)-28-O-(O-Ethyl)carboxymethyloleanolicaci d-3-(1'R)-(hydroxyl)methyl-β-D-glucurouopyranoside |
| A91 | 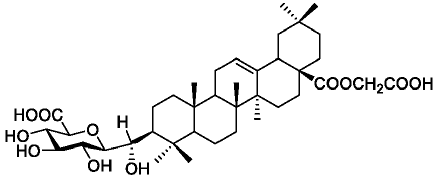 | (3S,5S,8R,9R,10S,14R,17R,18S)-28-O-Acetoxyoleanolicacid-3-(1'R)-(hydroxyl)methyl-β-D-glucurouopyranoside |
| A92 | 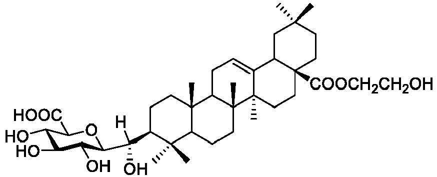 | (3S,5S,8R,9R,10S,14R,17R,18S)-28-O-Hydroxyethyloleanolicacid-3-(1'R)-(hydroxyl)methyl-β-D-glucurouopyranoside |
| A93 | 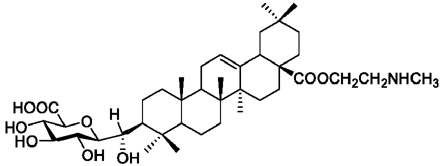 | (3S,5S,8R,9R,10S,14R,17R,18S)-28-O-(N-Methyl)aminoethyloleanolicacid-3-(1'R)-(hydroxyl)methyl-β-D-glucurouopyranoside |
| A94 | 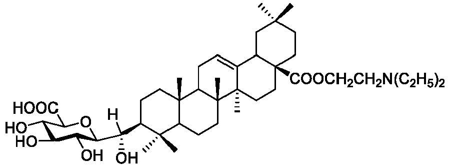 | (3S,5S,8R,9R,10S,14R,17R,18S)-28-O-(N,N-Diethyl)aminoethyloleanolicacid-3-(1'R)-(hydroxyl)methyl-β-D-glucurouopyranoside |
| A95 | 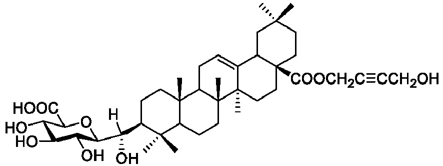 | (3S,5S,8R,9R,10S,14R,17R,18S)-28-O-Hydroxylbut-2‴ynyloleanolicacid-3-(1'R)-(hydroxyl)methyl-β-D-glucurouopyranoside |
| A96 | 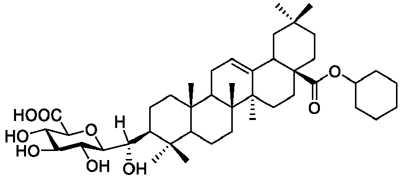 | (3S,5S,8R,9R,10S,14R,17R,18S)-28-O-Cyclohexyloleanolicacid-3-(1'R)-(hydroxyl)methyl-β-D-glucurouopyranoside |
| A97 | 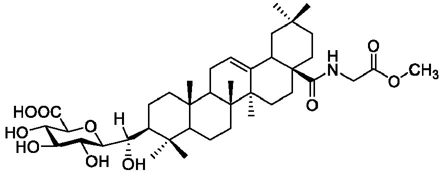 | (3S,5S,8R,9R,10S,14R,17R,18S)-28-(O-Methyl)carboxymethylaminoolea nolicacid-3-(1'R)-(hydroxyl)methyl-β-D-glucurouopyranoside |
| A98 | 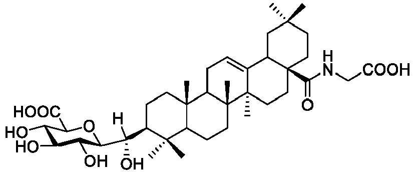 | (3S,5S,8R,9R,10S,14R,17R,18S)-28-Carboxymethylaminooleanolicaci d-3-(1'R)-(hydroxyl)methyl-β-D-glucurouopyranoside |
| A99 | 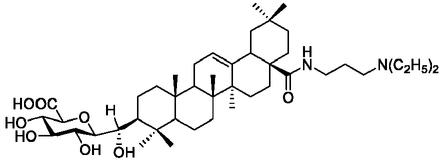 | (3S,5S,8R,9R,10S,14R,17R,18S)-28-(N,N-Diethyl)aminopropylaminooleano licacid-3-(1'R)-(hydroxyl)methyl-β-D-glucurouopyranoside |
| A100 | 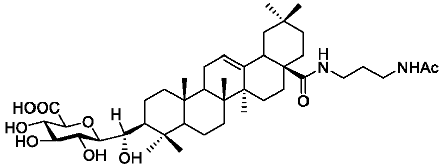 | (3S,5S,8R,9R,10S,14R,17R,18S)-28-(N-Acetyl)aminopropylaminooleanol icacid-3-(1'R)-(hydroxyl)methyl-β-D-glucurouopyranoside |
| A101 | 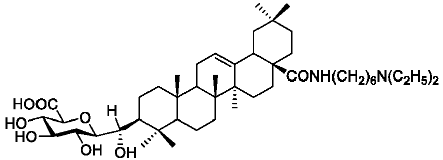 | (3S,5S,8R,9R,10S,14R,17R,18S)-28-(N,N-Diethyl)aminohexylaminooleanoli cacid-3-(1'R)-(hydroxyl)methyl-β-D-glucurouopyranoside |
| A 102 | 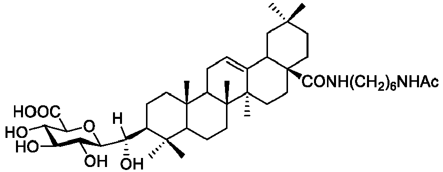 | (3S,5S,8R,9R,10S,14R,17R,18S)-28-(N-Acetyl)aminohexylaminooleanoli cacid-3-(1'R)-(hydroxyl)methyl-β-D-glucurouopyranoside |
| A 103 | 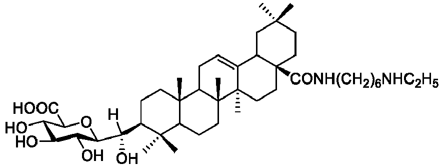 | (3S,5S,8R,9R,10S,14R,17R,18S)-28-(N-Ethyl)aminohexylaminooleanolic acid-3-(1'R)-(hydroxyl)methyl-β-D-glucurouopyranoside |
| A104 | 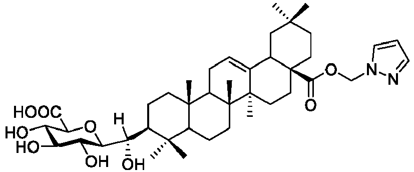 | (3S,5S,8R,9R,10S,14R,17R,18S)-28-O-(Pyrazol-1"yl)methyloleanolicacid-3-(1'R)-(hydroxyl)methyl-β-D-glucurouopyranoside |
| A105 | 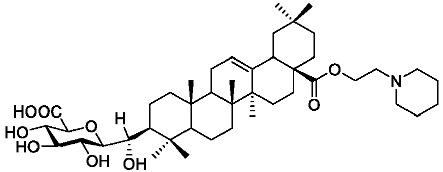 | (3S,5S,8R,9R,10S,14R,17R,18S)-28-O-(Piperidin-1"-yl)methyloleanolicacid-3-(1'R)-(hydroxyl)methyl-β-D-glucurouopyranoside |
| A106 | 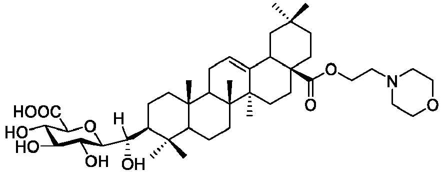 | (3S,5S,8R,9R,10S,14R,17R,18S)-28-O-(Morpholin-4"-yl)methyloleanolicacid-3-(1'R)-(hydroxyl)methyl-β-D-glucurouopyranoside |
| A 107 | 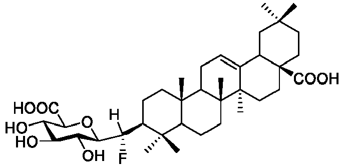 | (3S,5S,8R,9R,10S,14R,17R,18S)-Oleanolicacid-3-(1'R)-fluoromethyl-β-D-glucurouopyranoside |
| A108 | 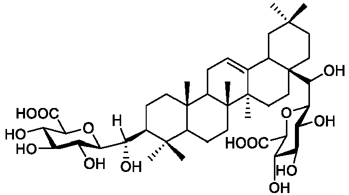 | (3S,5S,8R,9R,10S,14R,17R,18S)-Oleanolicacid-3-(1'R)-(hydroxyl)methyl-28-(1"R)-(hydroxyl)methyl-β-D-diglucurouopyranoside |
| A109 | 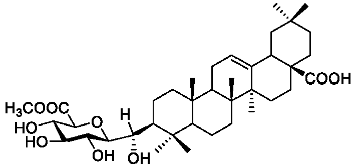 | (3S,5S,8R,9R,10S,14R,17R,18S)-Oleanolicacid-3-(1'R)-(hydroxyl)methyl-6"-O-methyl-β-D-glucurouopyranoside |
| A110 | 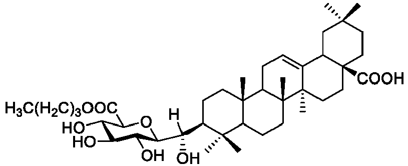 | (3S,5S,8R,9R,10S,14R,17R,18S)-Oleanolicacid-3-(1''R)-(hydroxyl)methyl-6"-O-n-butyl-β-D-glucurouopyranoside |
| A111 | 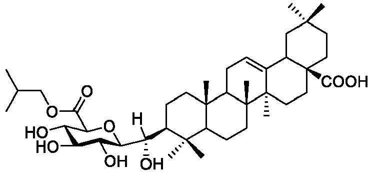 | (3S,5S,8R,9R,10S,14R,17R,18S)-Oleanolicacid-3-(1'R)-(hydroxyl)methyl-6"-O-isobutyl-β-D-glucurouopyranoside |
| A112 | 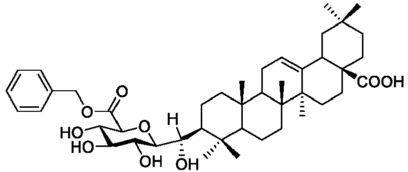 | (3S,5S,8R,9R,10S,14R,17R,18S)-Oleanolicacid-3-(1'R)-(hydroxyl)methyl-6"-O-benzyl-β-D-glucurouopyranoside |
| A113 | 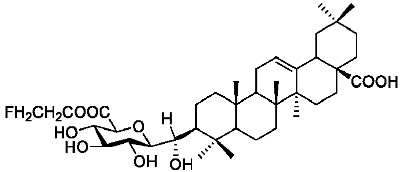 | (3S,5S,8R,9R,10S,14R,17R,18S)-Oleanolicacid-3-(1'R)-(hydroxyl)methyl-6"-O-(1-fluoroacetyl)-β-D-glucurouopyranoside |
| A114 | 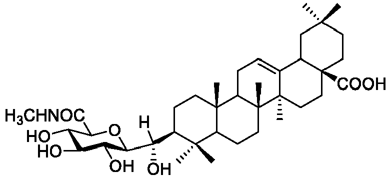 | (3S,5S,8R,9R,10S,14R,17R,18S)-Oleanolicacid-3-(1'R)-(hydroxyl)methyl-6"-methylamino-β-D-glucurouopyranoside |
| A115 | 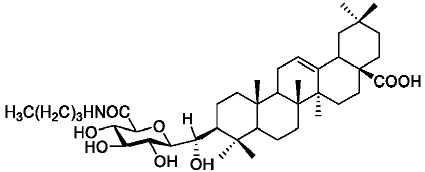 | (3S,5S,8R,9R,10S,14R,17R,18S)-Oleanolicacid-3-(1'R)-(hydroxyl)methyl-6"-n-butylamino-β-D-glucurouopyranoside |
| A116 | 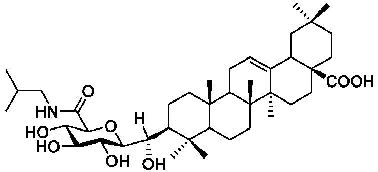 | (3S,5S,8R,9R,10S,14R,17R,18S)-Oleanolicacid-3-(1'R)-(hydroxyl)methyl-6"-isobutylamino-β-D-glucurouopyranoside |
| A117 | 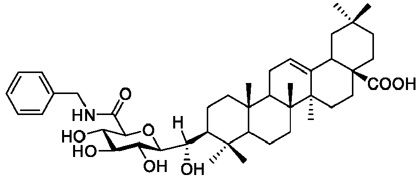 | (3S,5S,8R,9R,10S,14R,17R,18S)-Oleanolicacid-3-(1'R)-(hydroxyl)methyl-6"-benzylamino-β-D-glucurouopyranoside |
| A118 | 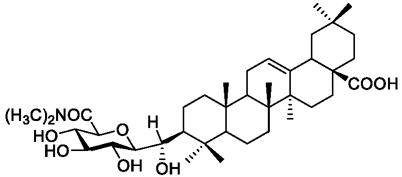 | (3S,5S,8R,9R,10S,14R,17R,18S)-Oleanolicacid-3-(1'R)-(hydroxyl)methyl-6"-dimethylamino-β-D-glucurouopyranoside |
| A119 | 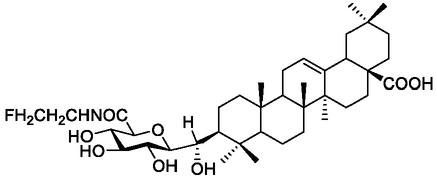 | (3S,5S,8R,9R,10S,14R,17R,18S)-Oleanolicacid-3-(1'R)-(hydroxyl)methyl-6"-(1-fluoroethylamino)-β-D-glucurouopyranoside |
| A 120 | 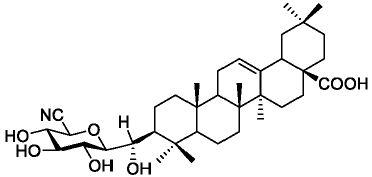 | (3S,5S,8R,9R,10S,14R,17R,18S)-Oleanolicacid-3-(1'R)-(hydroxyl)methyl-5"-cyano-β-D-xylopyranoside |
| A121 | 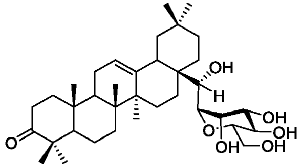 | (5S,8R,9R,10S,14R,17R,18S)-3-Carbonyloleanolicacid-17-(1'S)-(hydroxyl)methyl-β-D-mannopyranoside |
| A 122 | 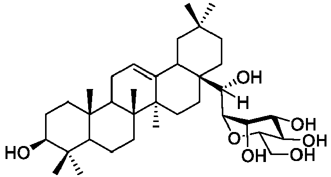 | (3S,5S,8R,9R,10S,14R,17R,18S)-Oleanolicacid-17-(1'S)-(hydroxyl)methyl-β-D-mannopyranoside |
| A123 | 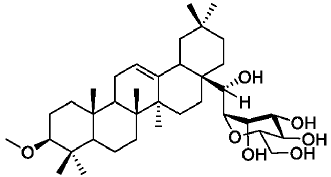 | (3S,5S,8R,9R,10S,14R,17R,18S)-3-Methoxyoleanolicacid-17-(1'S)-(hydroxyl)methyl-β-D-mannopyranoside |
| A124 | 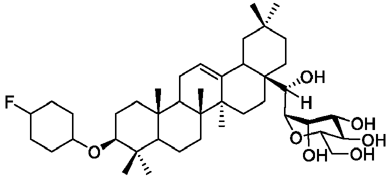 | (3S,5S,8R,9R,10S,14R,17R,18S)-3-(4-Fluorocyclohexoxy)oleanolicacid-17-(1'S)-(hydroxyl)methyl-β-D-mannopyranoside |
| A125 | 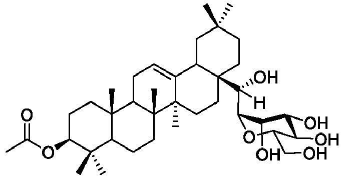 | (3S,5S,8R,9R,10S,14R,17R,18S)-3-Acetoxyoleanolicacid-17-(1'S)-(hydroxyl)methyl-β-D-mannopyranoside |
| A126 | 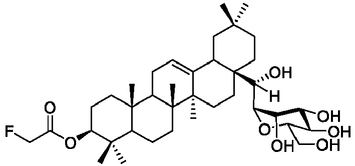 | (3S,5S,8R,9R,10S,14R,17R,18S)-3-Fluorocetoxyoleanolicacid-17-(1'S)-(hydroxyl)methyl-β-D-mannopyranoside |
| A 127 | 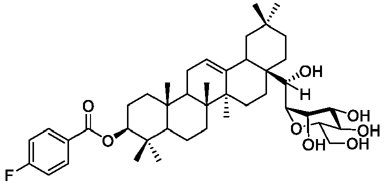 | (3S,5S,8R,9R,10S,14R,17R,18S)-3-(4-fluorophenylacetoxy)oleanolicaci d-17-(1'S)-(hydroxyl)methyl-β-D-mannopyranoside |
| A128 | 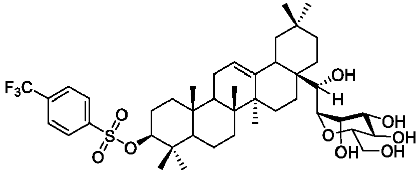 | (3S,5S,8R,9R,10S,14R,17R,18S)-3-(4-Trifluoromethylphenylsulfonylox y)oleanolicacid-17-(1'S)-(hydroxyl)methyl-β-D-mannopyranoside |
| A 129 | 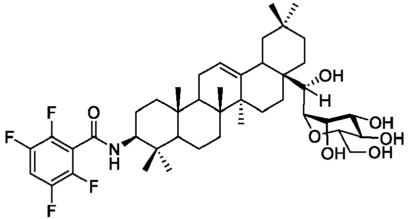 | (3S,5S,8R,9R,10S,14R,17R,18S)-3-(2,3,5,6-Tetrafluorobenzamide)oleanolicac id-17-(1'S)-(hydroxyl)methyl-β-D-mannopyranoside |
| A130 | 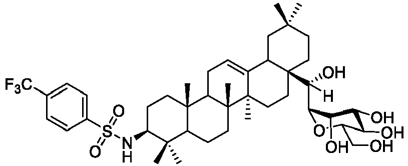 | (3S,5S,8R,9R,10S,14R,17R,18S)-3-(4-Trifluoromethylbenzenesulfonami do)oleanolicacid-17-(1'S)-(hydroxyl)methyl-β-D-mannopyranoside |
| A131 | 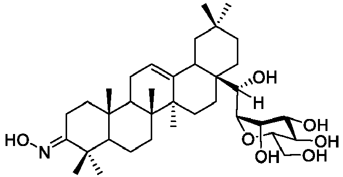 | (5S,8R,9R,10S,14R,17R,18S)-3-Oximidooleanolicacid-17-(1'S)-(hydroxyl)methyl-β-D-mannopyranoside |
| A132 | 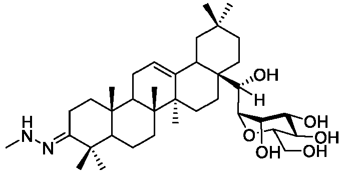 | (5S,8R,9R,10S,14R,17R,18S)-3-Methylhydrazinylideneoleanolica cid-17-(1'S)-(hydroxyl)methyl-β-D-mannopyranoside |
| A133 | 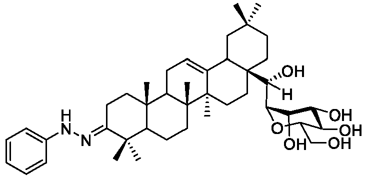 | (5S,8R,9R,10S,14R,17R,18S)-3-Phenylhydrazinylideneoleanolicac id-17-(1'S)-(hydroxyl)methyl-β-D-mannopyranoside |
| A134 | 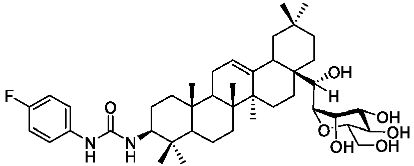 | (3S,5S,8R,9R,10S,14R,17R,18S)-3-(4-Fluorophenylureido)oleanolicacid -17-(1'S)-(hydroxyl)methyl-β-D-mannopyranoside |
| A135 | 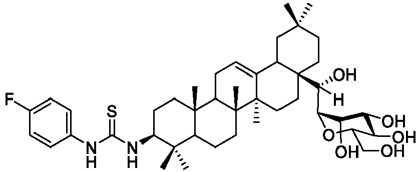 | (3S,5S,8R,9R,10S,14R,17R,18S)-3-(4-Fluorophenylthioureido)oleanolic acid-17-(1'S)-(hydroxyl)methyl-β-D-mannopyranoside |
| A136 | 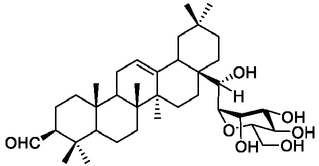 | (3S,5S,8R,9R,10S,14R,17R,18S)-3-Formyloleanolicacid-17-(1'S)-(hydroxyl)methyl-β-D-mannopyranoside |
| A137 | 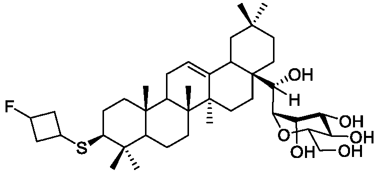 | (3S,5S,8R,9R,10S,14R,17R,18S)-3-(3-Fluorocyclobutylthio)oleanolicaci d-17-(1'S)-(hydroxyl)methyl-β-D-mannopyranoside |
| A138 | 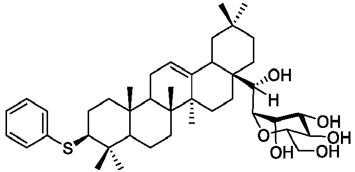 | (3S,5S,8R,9R,10S,14R,17R,18S)-3 -Phenylthiooleanolic acid- 17-(1'S)-(hydroxyl)methyl-β-D-mannopyranoside |
| A139 | 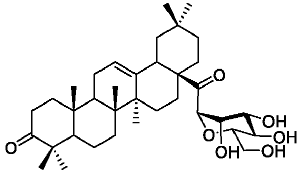 | (5S,8R,9R,10S,14R,17R,18S)-3-Carbonyloleanolicacid-28-β-D-mannopyranoside |
| A140 | 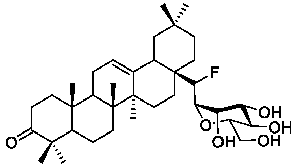 | (5S,8R,9R,10S,14R,17R,18S)-3-Carbonyloleanolicacid-17-(1'S)-fluoromethyl-β-D-mannopyranoside |
| A141 | 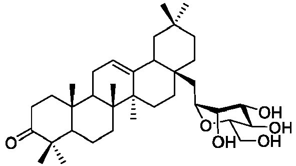 | (5S,8R,9R,10S,14R,17R,18S)-3-Carbonyloleanolicacid-17-methyl-β-D-mannopyranoside |
| A142 | 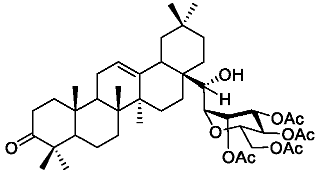 | (5S,8R,9R,10S,14R,17R,18S)-3-Carbonyloleanolicacid-17-(1'S)-(hydroxyl)methyl-2",3",4",6"-O-Tetraacetyl-β-D-mannopyranoside |
| A143 | 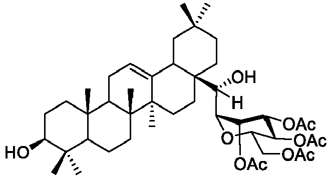 | (3S,5S,8R,9R,10S,14R,17R,18S)-Oleanolicacid-17-(1'S)-(hydroxyl)methyl-2",3",4",6"-O-tetraacetyl-β-D-mannopyranoside |
| A144 | 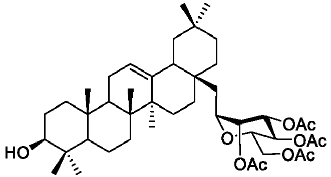 | (5S,8R,9R,10S,14R,17R,18S)-3-Carbonyloleanolicacid-17-methyl-2", 3 ",4", 6"-O-tetraacetyl-β-D-mannopyranoside |
| A145 | 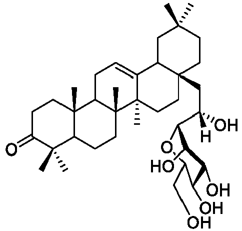 | (5S,8R,9R,10S,14R,17R,18S)-3-Carbonyloleanolicacid-17-(2'S)-(hydroxyl)ethyl-β-D-mannopyranoside |
| A146 | 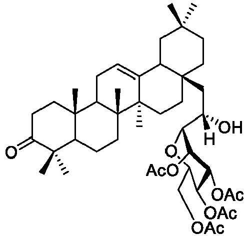 | (5S,8R,9R,10S,14R,17R,18S)-3-Carbonyloleanolicacid-17-(2'S)-(hydroxyl)ethyl-2",3",4",6"-O-Tetraacetyl-β-D-mannopyranoside |
| A147 | 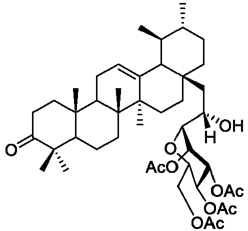 | (5S,8R,9R,10S,14R,17R,18S)-3-Carbonylursolicacid-17-(2'S)-(hydroxyl)ethyl-2",3",4",6"-O-tetraacetyl-β-D-mannopyranoside |
| A148 | 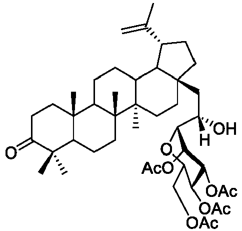 | (5S,8R,9R,10S,14R,17R,18S)-3-Carbonylbetulinicacid-17-(2'S)-(hydroxyl)ethyl-2",3",4",6"-O-tetraacetyl-β-D-mannopyranoside |
| A149 | 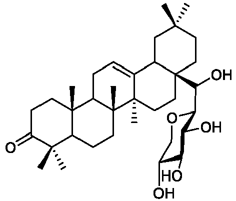 | (5S,8R,9R,10S,14R,17R,18S)-3-Carbonyloleanolicacid-17-(1'R)-(hydroxyl)methyl-β-D-xylopyranoside |
| A150 | 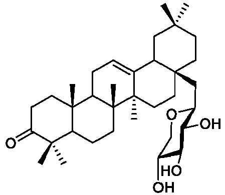 | (5S,8R,9R,10S,14R,17R,18S)-3-Carbonyloleanolicacid-17methyl-β-D-xylopyranoside |
| A151 | 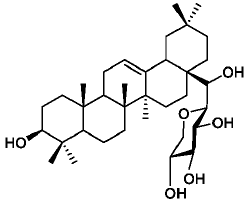 | (3S,5S,8R,9R,10S,14R,17R,18S)-Oleanolicacid-17-(1'R)-(hydroxyl)methyl-β-D-xylopyranoside |
| A152 | 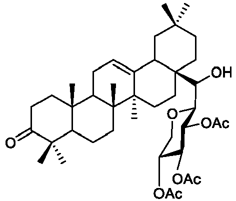 | (5S,8R,9R,10S,14R,17R,18S)-3-Carbonyloleanolicacid-17-(1 'R)-(hydroxyl)methyl-2",3",4"-O-triacetyl-β-D-xylopyranoside |
| A153 | 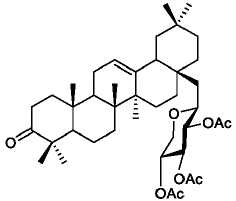 | (5S,8R,9R,10S,14R,17R,18S)-3-Carbonyloleanolicacid-17-methyl-2",3",4"-O-triacetyl-β-D-xylopyranoside |
| A154 | 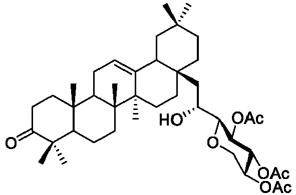 | (5S,8R,9R,10S,14R,17R,18S)-3-Carbonyloleanolicacid-17-(2'R)-(hydroxyl)ethyl-2",3",4"-O-triacetyl-β-D-xylopyranoside |
| A155 | 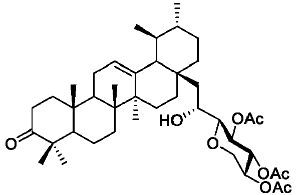 | (5S,8R,9R,10S,14R,17R,18S)-3-Carbonylursolicacid-17-(2'R)-(hydroxyl)ethyl-2",3",4"-O-triacetyl-β-D-xylopyranoside |
| A156 | 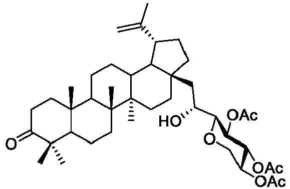 | (5S,8R,9R,10S,14R,17R,18S)-3-Carbonylbetulinicacid-17-(2'R)-(hydroxyl)ethyl-2",3",4"-O-triacetyl-β-D-xylopyranoside |
| A157 | 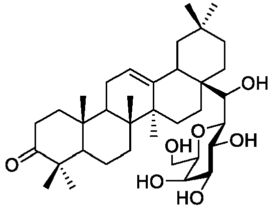 | (5S,8R,9R,10S,14R,17R,18S)-3-Carbonyloleanolicacid-17-(1'R)-(hydroxyl)methyl-β-D-galactopyrano side |
| A158 | 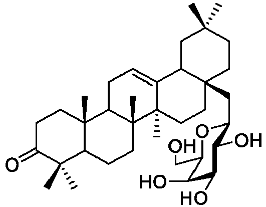 | (5S,8R,9R,10S,14R,17R,18S)-3-Carbonyloleanolicacid-17-methyl-β-D-galactopyranoside |
| A159 | 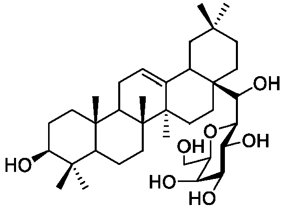 | (3S,5S,8R,9R,10S,14R,17R,18S)-Oleanolicacid-17-(1'R)-(hydroxyl)methyl-β-D-galactopyrano side |
| A 160 | 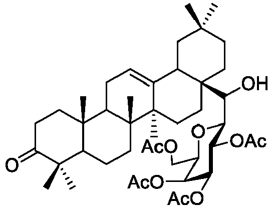 | (5S,8R,9R,10S,14R,17R,18S)-3-Carbonyloleanolicacid-17-(1'R)-(hydroxyl)methyl-2",3",4",6"-O-tetraacetyl-β-D-galactopyrano side |
| A161 | 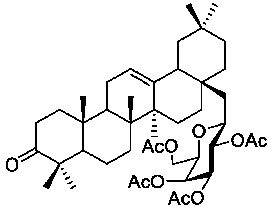 | (5S,8R,9R,10S,14R,17R,18S)-3-Carbonyloleanolicacid-17-methyl-2",3 ",4",6"-O-tetraacetyl-β-D-galactopyranoside |
| A162 | 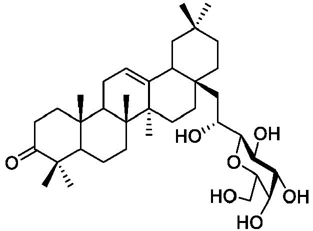 | (5S,8R,9R,10S,14R,17R,18S)-3-Carbonyloleanolicacid-17-(2'R)-(hydroxyl)ethyl-β-D-galactopyranoside |
| A163 | 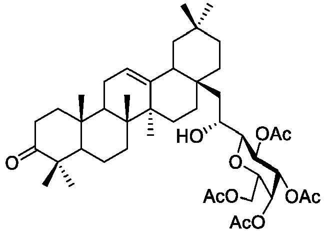 | (5S,8R,9R,10S,14R,17R,18S)-3-Carbonyloleanolicacid-17-(2'R)-(hydroxyl)ethyl-2",3",4",6"-O-tetraacetyl-β-D-galactopyranoside |
| A164 | 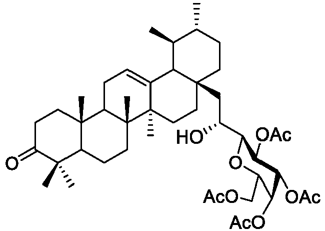 | (5S,8R,9R,10S,14R,17R,18S)-3-Carbonylursolicacid-17-(2'R)-(hydroxyl)ethyl-2",3",4",6"-O-tetraacetyl-β-D-galactopyranoside |
| A165 | 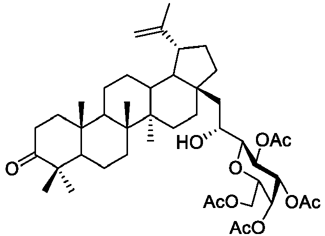 | (5S,8R,9R,10S,14R,17R,18S)-3-Carbonylbetulinicacid-17-(2'R)-(hydroxyl)ethyl-2",3",4",6"-O-tetraacetyl-β-D-galactopyranoside |
| A166 | 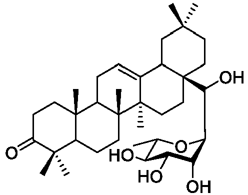 | (5S,8R,9R,10S,14R,17R,18S)-3-Carbonyloleanolicacid-17-(1'R)-(hydroxyl)methyl-α-L-rhamnopyranoside |
| A167 | 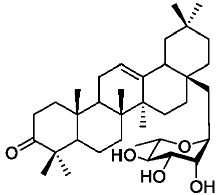 | (5S,8R,9R,10S,14R,17R,18S)-3-Carbonyloleanolicacid-17-methyl-α-L-rhamnopyranoside |
| A168 | 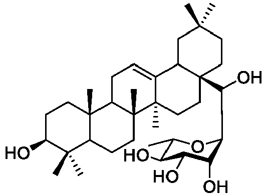 | (3S,5S,8R,9R,10S,14R,17R,18S)-Oleanolicacid-17-(1 'R)-(hydroxyl)methyl-α-L-rhamnopyrano side |
| A169 | 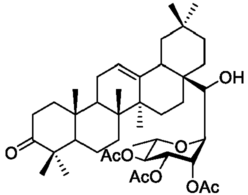 | (5S,8R,9R,10S,14R,17R,18S)-3-Carbonyloleanolicacid-17-(1'R)-(hydroxyl)methyl-2",3",4"-O-triacetyl-α-L-rhamnopyranoside |
| A170 | 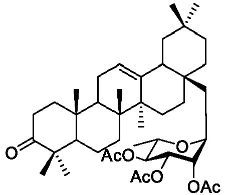 | (5S,8R,9R,10S,14R,17R,18S)-3-Carbonyloleanolicacid-17-methyl-2",3",4"-O-triacetyl-α-L-rhamnopyranoside |
| A171 | 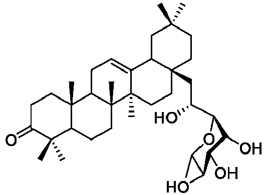 | (5S,8R,9R,10S,14R,17R,18S)-3-Carbonyloleanolicacid-17-(2'R)-(hydroxyl)ethyl-α-L-rhamnopyrano side |
| A172 | 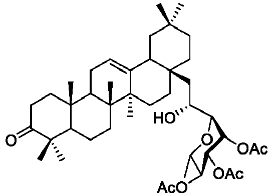 | (5S,8R,9R,10S,14R,17R,18S)-3-Carbonyloleanolicacid-17-(2'R)-(hydroxyl)ethyl-2",3",4"-O-triacetyl-α-L-rhamnopyranoside |
| A173 | 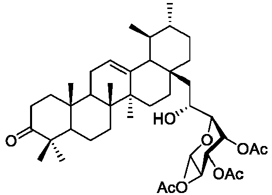 | (5S,8R,9R,10S,14R,17R,18S)-3-Carbonylursolicacid- 17-(2'R)-(hydroxyl)ethyl-2",3",4"-O-triacetyl-α-L-rhamnopyranoside |
| A174 | 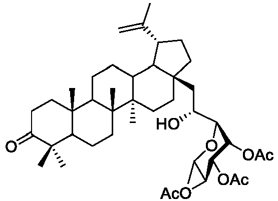 | (5S,8R,9R,10S,14R,17R,18S)-3-Carbonylbetulinicacid-17-(2'R)-(hydroxyl)ethyl-2",3",4"-O-triacetyl-α-L-rhamnopyranoside |
| A175 | 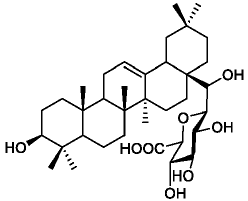 | (3S,5S,8R,9R,10S,14R,17R,18S)-Oleanolicacid-17-(1'R)-(hydroxyl)methyl-β-D-glucurouopyranoside |
| A176 | 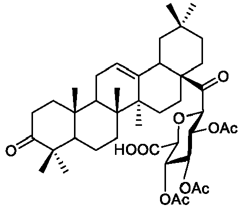 | (5S,8R,9R,10S,14R,17R,18S)-3-Carbonyloleanolicacid-17-carbonyl-2",3",4"-O-triacetyl-β-D-glucurouopyranoside |
| A177 | 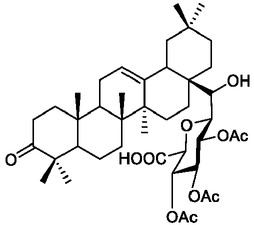 | (5S,8R,9R,10S,14R,17R,18S)-3-Carbonyloleanolicacid-17-(1'R)-(hydroxyl)methyl-2",3",4"-O-triacetyl-β-D-glucurouopyranoside |
| A178 | 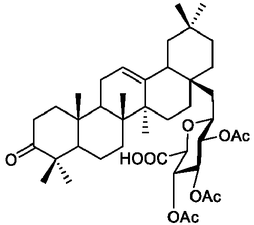 | (5S,8R,9R,10S,14R,17R,18S)-3-Carbonyloleanolicacid-17-methyl-2",3",4"-O-triacetyl-β-D-glucurouopyranoside |
| A179 | 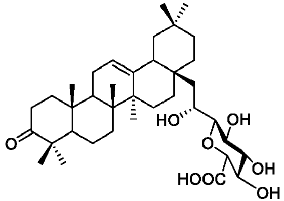 | (5S,8R,9R,10S,14R,17R,18S)-3-Carbonyloleanolicacid-17-(2'R)-(hydroxyl)ethyl-β-D-glucurouopyranoside |
| A180 | 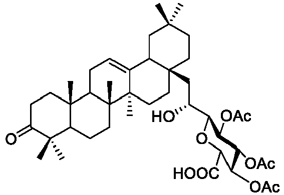 | (5S,8R,9R,10S,14R,17R,18S)-3-Carbonyloleanolicacid-17-(2'R)-(hydroxyl)ethyl-2",3",4"-O-triacetyl-β-D-glucurouopyranoside |
| A181 | 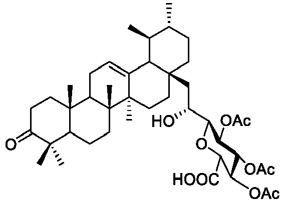 | (5S,8R,9R,10S,14R,17R,18S)-3-Carbonylursolicacid-17-(2'R)-(hydroxyl)ethyl-2",3",4"-O-triacetyl-β-D-glucurouopyranoside |
| A182 | 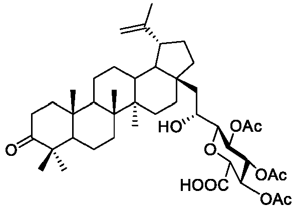 | (5S,8R,9R,10S,14R,17R,18S)-3-Carbonylbetulinicacid-17-(2'R)-(hydroxyl)ethyl-2",3",4"-O-triacetyl-β-D-glucurouopyranoside |
| A183 | 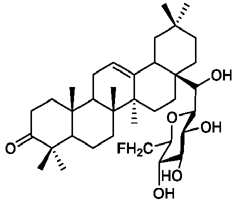 | (5S,8R,9R,10S,14R,17R,18S)-3-Carbonyloleanolicacid-17-(1'R)-(hydroxyl)methyl-6"-deoxy-6"-fluoro-β-D-glucopyranoside |
| A184 | 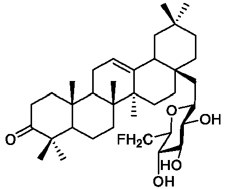 | (5S,8R,9R,10S,14R,17R,18S)-3-Carbonyloleanolicacid-17methyl-6"-deoxy-6"-fluoro-β-D-glucopyranoside |
| A185 | 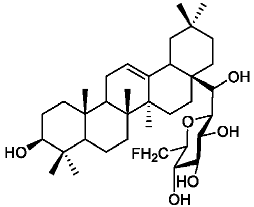 | (3S,5S,8R,9R,10S,14R,17R,18S)-Oleanolicacid-17-(1'R)-(hydroxyl)methyl-6"-deoxy-6"-fluoro-β-D-glucopyranoside |
| A186 | 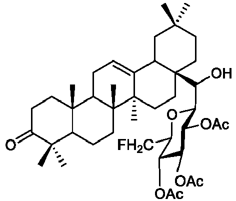 | (5S,8R,9R, 10S, 14R, 17R, 1 8S)-3-Carbonyloleanolicacid-17-(1'R)-(hydroxyl)methyl-2",3",4"-O-triacetyl-6"-deoxy-6"-fluoro-β-D-glucopyrano side |
| A187 | 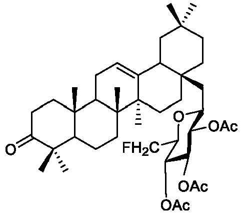 | (5S,8R,9R,10S,14R,17R,18S)-3-Carbonyloleanolicacid-17-methyl-2",3",4"-O-triacetyl-6"-deoxy-6"-fluoro-β-D-glucopyranoside |
| A188 | 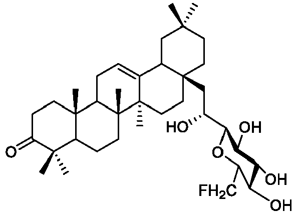 | (5S,8R,9R,10S,14R,17R,18S)-3-Carbonyloleanolicacid-17-(2'R)-(hydroxyl)ethyl-6"-deoxy-6"-fluoro-β-D-glucopyranoside |
| A189 | 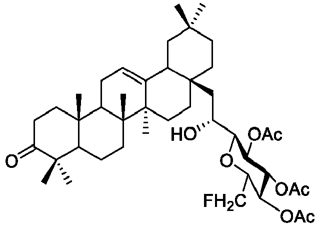 | (5S,8R,9R,10S,14R,17R,18S)-3-Carbonyloleanolicacid-17-(2'R)-(hydroxyl)ethyl-2",3",4"-O-triacetyl-6"-deoxy-6"-fluoro-β-D-glucopyranoside |
| A190 | 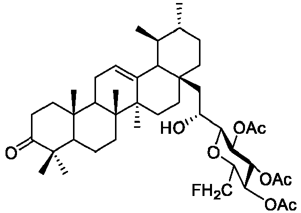 | (5S,8R,9R,10S,14R,17R,18S)-3-Carbonylursolicacid-17-(2'R)-(hydroxyl)ethyl-2",3",4"-O-triacetyl-6"-deoxy-6"-fluoro-β-D-glucopyranoside |
| A191 | 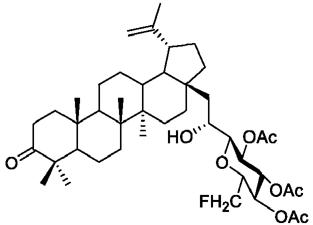 | (5S,8R,9R,10S,14R,17R,18S)-3-Carbonylbetulinicacid-17-(2'R)-(hydroxyl)ethyl-2",3",4"-O-triacetyl-6"-deoxy-6"-fluoro-β-D-glucopyranoside |
| A192 | 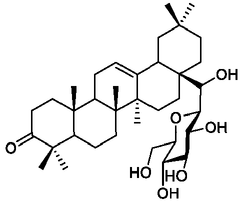 | (5S,8R,9R,10S,14R,17R,18S)-3-Carbonyloleanolicacid-17-(1'R)-(hydroxyl)methyl-β-D-glucopyranoside |
| A193 | 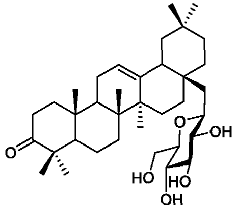 | (5S,8R,9R,10S,14R,17R,18S)-3-Carbonyloleanolicacid-17methyl--β-D-glucopyranoside |
| A194 | 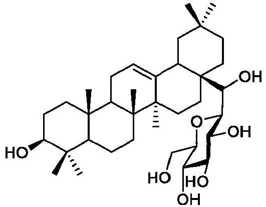 | (3S,5S,8R,9R,10S,14R,17R,18S)-Oleanolicacid-17-(1'R)-(hydroxyl)methyl-β-D-glucopyranoside |
| A195 | 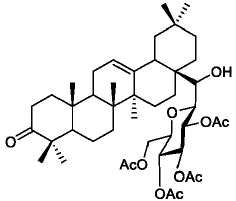 | (5S,8R,9R,10S,14R,17R,18S)-3-Carbonyloleanolicacid-17-(1'R)-(hydroxyl)methyl-2",3",4",6"-O-tetraacetyl-β-D-glucopyranoside |
| A196 | 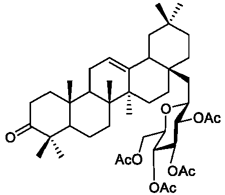 | (5S,8R,9R,10S,14R,17R,18S)-3-Carbonyloleanolicacid-17-methyl-2",3",4",6"-O-tetraacetyl-β-D-glucopyranoside |
| A 197 | 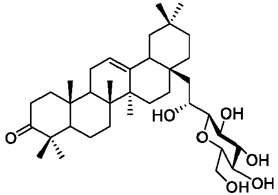 | (5S,8R,9R,10S,14R,17R,18S)-3-Carbonyloleanolicacid-17-(2'R)-(hydroxyl)ethyl-β-D-glucopyranoside |
| A198 | 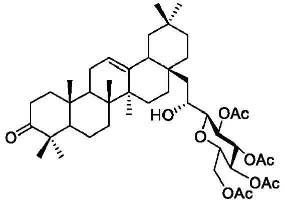 | (5S,8R,9R,10S,14R,17R,18S)-3-Carbonyloleanolicacid-17-(2'R)-(hydroxyl)ethyl-2",3",4",6"-O-tetraacetyl-β-D-glucopyranoside |
| A199 | 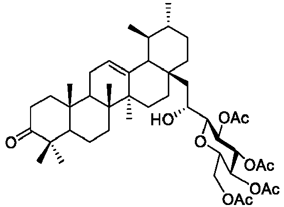 | (5S,8R,9R,10S,14R,17R,18S)-3-Carbonylursolicacid-17-(2'R)-(hydroxyl)ethyl-2",3",4",6"-O-tetraacetyl-β-D-glucopyranoside |
| A200 | 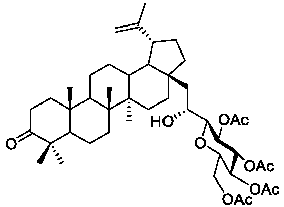 | (5S,8R,9R,10S,14R,17R,18S)-3-Carbonylbetulinicacid-17-(2'R)-(hydroxyl)ethyl-2",3",4",6"-O-tetraacetyl-β-D-glucopyranoside |
| A201 | 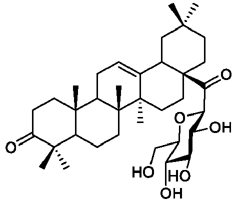 | (5S,8R,9R,10S,14R,17R,18S)-3-Carbonyloleanolicacid-28-β-D-glucopyranoside |
| A202 | 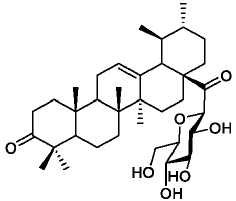 | (5S,8R,9R,10S,14R,17R,18S)-3-Carbonylursolicacid-28-β-D-glucopyranoside |
| A203 | 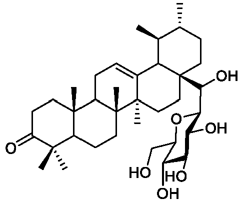 | (5S,8R,9R,10S,14R,17R,18S)-3-Carbonylursolicacid-17-(1'R)-(hydroxyl)methyl-β-D-glucopyranoside |
| A204 | 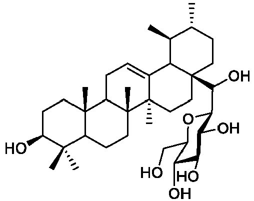 | (3S,5S,8R,9R,10S,14R,17R,18S)-Ursolicacid-17-(1'R)-(hydroxyl)methyl-β-D-glucopyranoside |
| A205 | 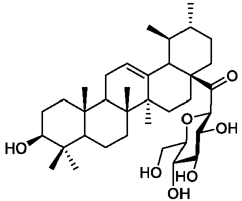 | (3S,5S,8R,9R,10S,14R,17R,18S)-Ursolicacid-28-β-D-glucopyranoside |
| A206 | 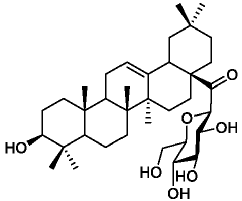 | (3S,5S,8R,9R,10S,14R,17R,18S)-Oleanolicacid-28-β-D-glucopyranoside |
| A207 | 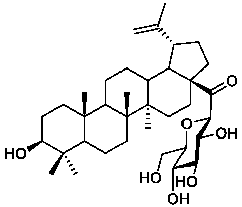 | (3S,5S,8R,9R,10S,13R,14R,17S,1 8R,19R)-Betulinicacid-28-β-D-glucopyranoside |
| A208 | 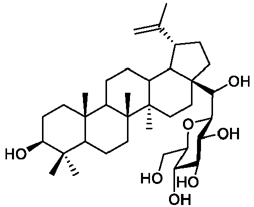 | (3S,5S,8R,9R,10S,13R,14R,17S,1 8R,19R)-Betulinicacid-17-(1'R)-(hydroxyl)methyl-β-D-glucopyranoside |
| A209 | 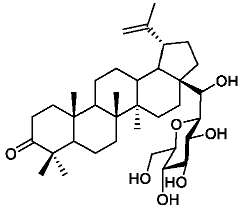 | (5S,8R,9R,10S,13R,14R,17S,18R, 19R)-3-Carbonylbetulinicacid-17-(1'R)-(hydroxyl)methyl-β-D-glucopyranoside |
| A210 | 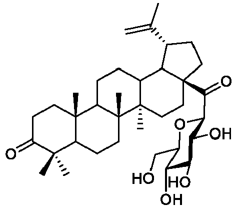 | (5S,8R,9R,10S,13R,14R,17S,18R, 19R)-3-Carbonylbetulinicacid-28-β-D-glucopyranoside |
| A211 | 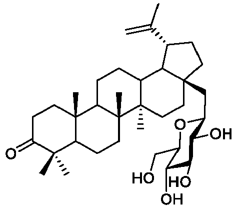 | (5S,8R,9R,10S,13R,14R,17S,18R, 19R)-3-Carbonylbetulinicacid-17-methyl-β-D-glucopyranoside |
| A212 | 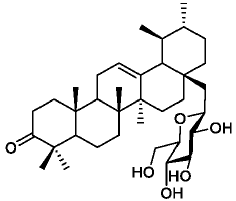 | (5S,8R,9R,10S,14R,17R,18S)-3-Carbonylursolicacid-17-methyl-β-D-glucopyranoside |
| A213 | 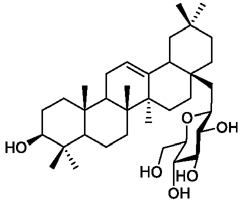 | (3S,5S,8R,9R,10S,14R,17R,18S)-Oleanolicacid-17-methyl-β-D-glucopyranoside |
| A214 | 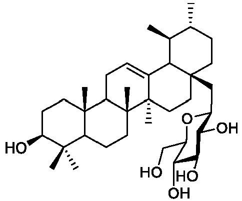 | (3S,5S,8R,9R,10S,14R,17R,18S)-3-Carbonylursolicacid-17-methyl-β-D-glucopyranoside |
| A215 | 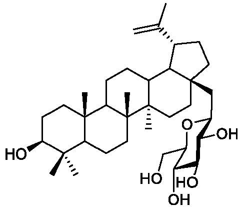 | (3S,5S,8R,9R,10S,13R, 4R,17S,1 8R,19R)-Betulinicacid-17-methyl-β-D-glucopyranoside |
| A216 | 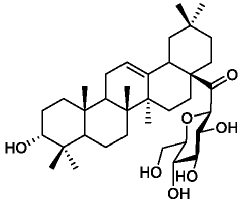 | (3R,5S,8R,9R,10S,14R,17R,18S)-Oleanolicacid-28-β-D-glucopyranoside |
| A217 | 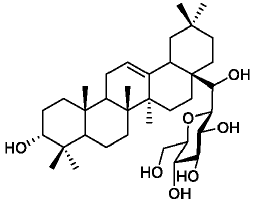 | (3R,5S,8R,9R,10S,14R,17R,18S)-Oleanolicacid-17-(1'R)-(hydroxyl)methyl-β-D-glucopyranoside |
| A218 | 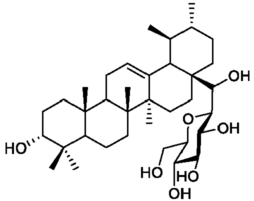 | (3R,5S,8R,9R,10S,14R,17R,18S)-Ursolicacid-17-(1'R)-(hydroxyl)methyl-β-D-glucopyranoside |
| A219 | 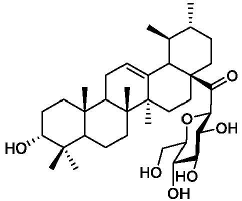 | (3R,5S,8R,9R,10S,14R,17R,18S)-Ursolicacid-28-β-D-glucopyranoside |
| A220 | 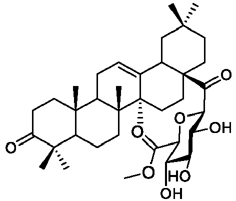 | (5S,8R,9R,10S,14R,17R,18S)-3-Carbonyloleanolicacid-28-6"-O-methyl-β-D-glucurouopyranoside |
| A221 | 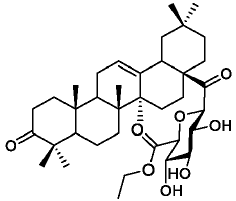 | (5S,8R,9R,10S,14R,17R,18S)-3-Carbonyloleanolicacid-28-6"-O-ethyl-β-D-glucurouopyranoside |
| A222 | 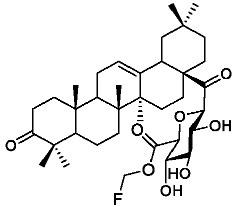 | (5S,8R,9R,10S,14R,17R,18S)-3-Carbonyloleanolicacid-28-6"-O-2‴-fluoroethyl-β-D-glucurouopyranoside |
| A223 | 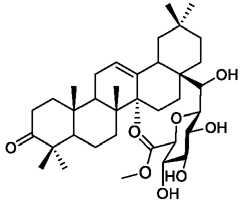 | (5S,8R,9R,10S,14R,17R,18S)-3-Carbonyloleanolicacid-17-(1'R)-(hydroxyl)methyl-6"-O-methyl-β-D-glucurouopyranoside |
| A224 | 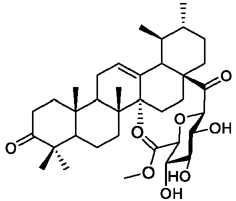 | (5S,8R,9R,10S,14R,17R,18S)-3-Carbonylursolicacid-28-6"-O-methyl-β-D-glucurouopyranoside |
| A225 | 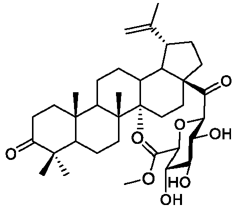 | (3S,5S,8R,9R,10S,13R,14R,17S,1 8R,19R)-Betulinicacid-28-6"-O-methyl-β-D-glucurouopyranoside |
| A226 | 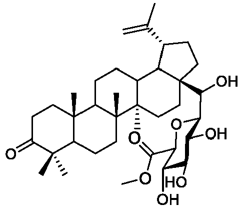 | (5S,8R,9R,10S,14R,17R,18S)-3-Carbonylbetulinicacid-17-(1'R)-(hydroxyl)methyl-6"-O-methyl-β-D-glucurouopyranoside |
| A227 | 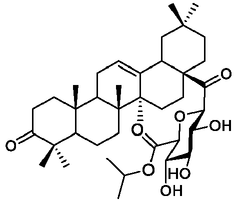 | (5S,8R,9R,10S,14R,17R,18S)-3-Carbonyloleanolicacid-28-6"-O-isopropyl-β-D-glucurouopyranoside |
| A228 | 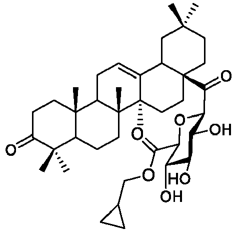 | (5S,8R,9R,10S,14R,17R,18S)-3-Carbonyloleanolicacid-28-6"-O-cyclopropylmethyl-β-D-glucurouopyranoside |
| A229 | 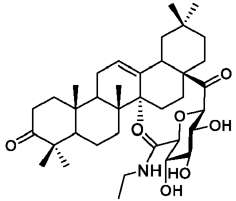 | (5S,8R,9R,10S,14R,17R,18S)-3-Carbonyloleanolicacid-28-6"-N-ethyl-β-D-glucurouopyranoside |
| A230 | 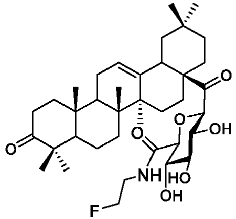 | (5S,8R,9R,10S,14R,17R,18S)-3-Carbonyloleanolicacid-28-6"-N-2‴-fluoroethyl-β-D-glucurouopyranoside |

### Active ingredient

The compounds of the invention can be acrylic acid derivative compounds with the structure shown in the following general formula I, or racemates, R-isomers, S-isomers, pharmaceutically acceptable salts thereof, or the mixtures thereof:

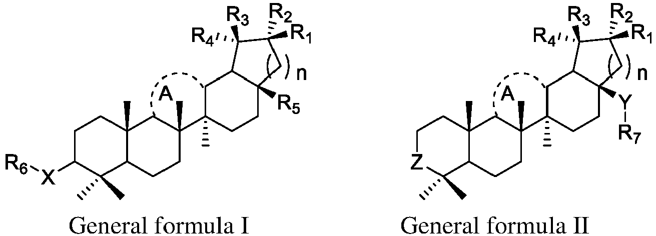

The definition of each group is the same as before.

The compounds of the invention have asymmetric center, chiral axis and chiral plane, and can present in the form of racemate, R-isomer or S-isomer. Those skilled in the art can obtain R-isomer and/or S-isomer from racemate by conventional technical means.

The invention provides medicinal salts of compounds of general formula I and general formula II, in particular, the conventional medicinal salts formed by reacting the compounds of general formula I and general formula II with inorganic acids or organic acids. For example, the conventional medicinal salts can be prepared by reacting compounds of general formula I and general formula II with inorganic acids or organic acids. The inorganic acids include hydrochloric acid, hydrobromic acid, sulfuric acid, nitric acid, sulfamic acid and phosphoric acid, etc., and the organic acids include citric acid, tartaric acid, lactic acid, pyruvic acid, acetic acid, benzenesulfonic acid, p-toluenesulfonic acid, methanesulfonic acid, naphthalene sulfonic acid, ethanesulfonic acid, naphthalene disulfonic acid, maleic acid, malic acid, malonic acid fumaric acid, succinic acid, propionic acid, oxalic acid, trifluoroacetic acid, stearic acid, pamoic acid, hydroxy maleic acid, phenylacetic acid, benzoic acid, salicylic acid, glutamic acid, ascorbic acid, p-aminobenzenesulfonic acid, 2-acetoxybenzoic acid and hydroxyethanesulfonic acid, etc.; or sodium salts, potassium salts, calcium salts, aluminum salts or ammonium salts formed by compounds of general formula I and general formula II and inorganic bases; or methylamine salts, ethylamine salts or ethanolamine salts formed by compounds of general formula I and general formula II and organic bases.

### Preparation method

On the other hand, the invention provides a preparation method of compounds represented by general formula I and general formula II, and the preparation method is carried out according to the following scheme 1 or scheme 2.

The compound of formula I can be prepared by the method shown in scheme 1 below.

The structural formula and R group label used in the following scheme are only used in this part. Compound of formula (III), compound of formula (IV) and compound of formula (V) can be commercially available or synthesized using conventional techniques in the art.

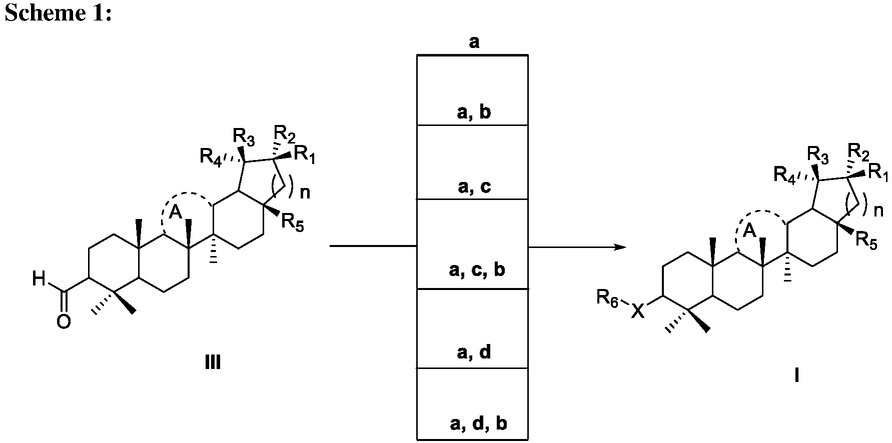

The definition of each group is the same as before.
Step a: adding compound IV and compound III to 0.1mol/L of redistilled tetrahydrofuran solution of samarium diiodide at 0 °C,, and reacting for 1h under the protection of argon.
Step b: dissolving the product of the previous step in the mixed solvent of ethyl acetate: methanol (1:1), adding Pd/C, and reacting for 12h at 50 °C, in the hydrogen environment.
Step c: dissolving the product of the previous step in CS₂, then adding NaH (60% oil mixture), and stirring for 2 hours at room temperature. Then adding CH₃I for reaction overnight, and after column chromatography, dissolving the reaction mixture in ultra dry toluene. Adding AIBN and tributyltin hydride under stirring, and stirring under reflux for 4 hours.
Step d: Dissolving the product of the previous step in dichloromethane, adding Des Martin reagent and stirring for 2h.

Wherein, the structure of compound IV is:

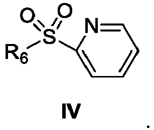

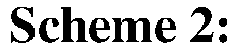

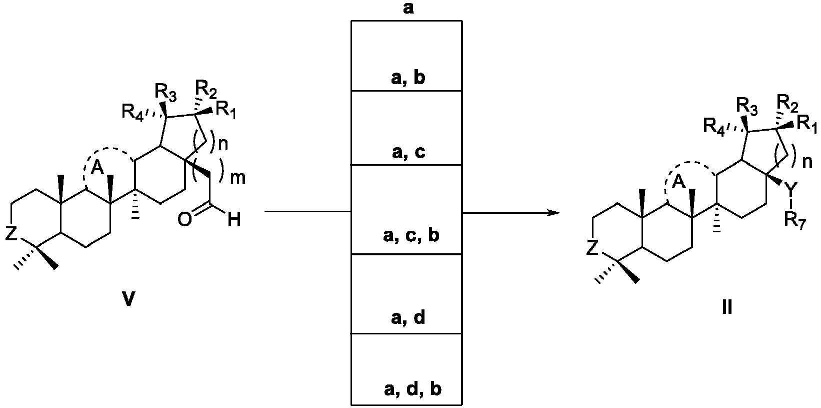

The definition of each group is the same as before.

The definitions of steps a, b, c and d are the same as those in Scheme 1.

### Pharmaceutical composition and administration method

Since the compounds of the present invention have excellent hypoglycemic and influenza virus inhibiting activities, the compound of the present invention and various crystal forms thereof, pharmaceutically acceptable inorganic or organic salts, hydrates or solvates thereof, and pharmaceutical composition containing the compound according to the present invention as main active ingredient can be used to treat, prevent and alleviate diseases associated with high blood glucose and influenza virus.

The pharmaceutical composition of the invention comprises the compound of the present invention or the pharmaceutically acceptable salts thereof in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg compounds of the invention per dose, preferably, 5-200mg compounds of the present invention per dose. Preferably, the "dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral administration, intratumoral administration, rectal administration, parenteral (intravenous, intramuscular or subcutaneous) administration, and topical administration.

Solid dosage forms for oral administration include capsules, tablets, pills, powders and granules. In these solid dosage forms, the active compounds are mixed with at least one conventional inert excipient (or carrier), such as sodium citrate or CaHPO₄, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The dosage forms of the compounds of the invention for topical administration include ointment, powder, patch, spray and inhalation. The active ingredients are mixed under sterile conditions with physiologically acceptable carriers and any preservatives, buffers, or propellants that may be required when necessary.

Compounds of the present invention can be administrated alone, or in combination with any other pharmaceutically acceptable compounds.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 5-500mg. Of course, the particular dose should also depend on various factors, such as the route of administration, patient healthy status, which are well within the skills of an experienced physician.

### The main advantages of the present invention include:

The invention provides a pentacyclic triterpenoid carbon glycoside derivative compound shown in general formula I or pharmaceutically acceptable salts, racemates, R-isomers or S-isomers thereof or the mixtures thereof.

The invention also provides a preparation method of the above compounds.

The invention also provides the use of the above pentacyclic triterpenoid carbon glycoside derivative compound or pharmaceutically acceptable salts, racemates, R-isomers or S-isomers thereof or the mixtures thereof in the preparation of medicine for treating or preventing metabolic diseases such as diabetes and hyperlipidemia.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The experimental materials and reagents used in the following examples can be commercially available unless otherwise specified.

The invention will be further illustrated in the following examples. These examples are only to illustrate the invention, but not to limit the invention in any way.

### Example 1 (3S,5S,8R,9R,10S,14R,17R,18S)-28-O-Benzyloleanolicacid-3-(1'R)-(hydroxy)methyl-2",3",4",6"-O-tetrabenzyl-O-D-glucopyranoside (A1)

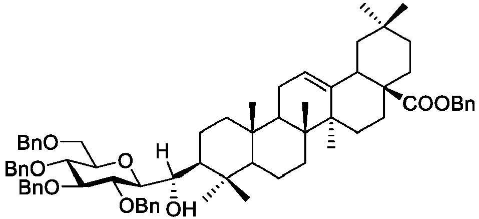

Compounds (3S,5S,8R,9R,10S,14R,17R,18S)-28-O-benzyl-3-aldehydeoleanolic acid (100.0 mg, 0.18 mmol) and 2,3,4,6-O-tetrabenzyl-1-(pyridin-1-yl) sulfonyl-β-D-thioglucopyranoside (207.7 mg, 0.36 mmol) were dissolved in 15 mL of redistilled tetrahydrofuran. Under argon protection, 0.1 mol/L of tetrahydrofuran solution of amarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain the target product A1 (152 mg, 78%). ¹H NMR (600 MHz, CDCl₃) δ 7.40 - 7.08 (m, 26H), 5.29 (t, *J* = 3.4 Hz, 1H), 5.05 (dt, *J =* 16.2, 11.9 Hz, 3H), 4.95 (d, *J =* 11.0 Hz, 1H), 4.83 (dd, *J* = 19.7, 11.0 Hz, 2H), 4.74 (d, *J* = 11.2 Hz, 1H), 4.65 - 4.47 (m, 3H), 4.01 (d, *J* = 5.8 Hz, 1H), 3.78 (t, *J* = 8.9 Hz, 1H), 3.68 (s, 1H), 3.66 - 3.62 (m, 1H), 3.58 (t, *J* = 9.1 Hz, 1H), 3.39 (dt, *J* = 9.7, 3.0 Hz, 1H), 3.31 (dd, *J* = 9.2, 6.7 Hz, 1H), 2.89 (dd, *J* = 13.6, 4.0 Hz, 1H), 1.98 (td, *J* = 13.5, 3.9 Hz, 1H), 1.87 - 1.76 (m, 2H), 1.26 (s, 3H), 1.12 (s, 3H), 0.94 (s, 3H), 0.91 (d, *J =* 1.5 Hz, 4H), 0.89 (s, 3H), 0.85 (s, 3H), 0.61 (s, 3H). LRMS (ESI):1083.66 [M+H]⁺.

### Example 2 (3S,5S,8R,9R,10S,14R,17R,18S)-Oleanolicacid-3-(1'R)-(hydroxy)methyl-β-D-glucopyranoside (A2)

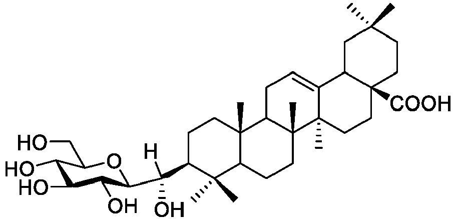

Compounds (3S, 5S, 8R, 9R, 10S, 14R, 17R, 18S)-28-O-benzyl-3-aldehyde oleanolic acid (100.0 mg, 0.18 mmol) and 2,3,4,6-O-tetrabenzyl-1-(pyridin-1-yl) sulfonyl-β-D-thioglucopyranoside (207.7 mg, 0.36 mmol) were dissolved in 15 mL of redistilled tetrahydrofuran. Under argon protection, 0.1 mol/L of tetrahydrofuran solution of samarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain 152 mg of A1. Then it was dissolved in the mixed solution of ethyl acetate: methanol (15 mL: 15 mL), 30 mg of Pd/C was added in hydrogen environment and the mixture was reacted at 50 °C for 12 hours. Then the target product A2 (70 mg, 79%) was obtained by column chromatography. ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 3 (3R,5S,8R,9R,10S,14R,17R,18S)-28-O-Benzyloleanolicacid-3-(1'R)-(hydroxy)methyl-2",3",4",6"-O-tetrabenzyl-β-D-glucopyranoside (A3)

(3S,5S,8R,9R,10S,14R,17R,18S)-28-O-Benzyl-3-aldehydeoleanolic acid was replaced by (3R,5S,8R,9R,10S,14R,17R,18S)-28-O-benzyl-3-aldehydeoleanolic acid. Other required raw materials, reagents and preparation methods were the same as those in Example 1 to obtain A3 (yield 85%). ¹H NMR (600 MHz, CDCl₃) δ 7.40 - 7.08 (m, 26H), 5.29 (t, *J* = 3.4 Hz, 1H), 5.05 (dt, *J* = 16.2, 11.9 Hz, 3H), 4.95 (d, *J* = 11.0 Hz, 1H), 4.83 (dd, *J* = 19.7, 11.0 Hz, 2H), 4.74 (d, *J* = 11.2 Hz, 1H), 4.65 - 4.47 (m, 3H), 4.01 (d, *J* = 5.8 Hz, 1H), 3.78 (t, *J* = 8.9 Hz, 1H), 3.68 (s, 1H), 3.66 - 3.62 (m, 1H), 3.58 (t, *J* = 9.1 Hz, 1H), 3.39 (dt, *J =* 9.7, 3.0 Hz, 1H), 3.31 (dd, *J* = 9.2, 6.7 Hz, 1H), 2.89 (dd, *J* = 13.6, 4.0 Hz, 1H), 1.98 (td, *J* = 13.5, 3.9 Hz, 1H), 1.87 - 1.76 (m, 2H), 1.26 (s, 3H), 1.12 (s, 3H), 0.94 (s, 3H), 0.91 (d, *J =* 1.5 Hz, 4H), 0.89 (s, 3H), 0.85 (s, 3H), 0.61 (s, 3H). LRMS (ESI): 1083.66 [M+H]⁺.

### Example 4 (3R,5S,8R,9R,10S,14R,17R,18S)-Oleanolicacid-3-(1'R)-(hydroxy)methyl-β-D-glucopyranoside (A4)

(3S,5S,8R,9R,10S,14R,17R,18S)-28-O-Benzyl-3-aldehyde oleanolic acid was replaced by (3R,5S,8R,9R,10S,14R,17R,18S)-3-aldehyde oleanolic acid. Other required raw materials, reagents and preparation methods are the same as those in Example 2 to obtain A4 (yield 74%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J =* 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 5. (3S,5S,8R,9R,10S,14R,17R,18S)-28-O-Benzyloleanolicacid-3-methyl-2",3",4",6"-O-tetrabenzyl-β-D-glucopyranoside (A5)

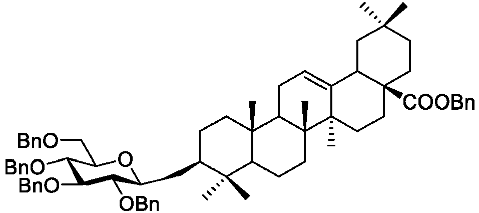

Compounds (3S,5S,8R,9R,10S,14R,17R,18S)-28-O-benzyl-3-aldehyde oleanolic acid (66.7 mg, 0.12 mmol) and 2,3,4,6-O-tetrabenzyl-1-(pyridin-1-yl)sulfonyl-β-D-thioglucopyranoside (138.5 mg, 0.24 mmol) were dissolved in 15 mL of redistilled tetrahydrofuran. Under argon protection, 0.1 mol/L of tetrahydrofuran solution of samarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain 152 mg of A1. Then it was dissolved in 10 mL of CS₂, NaH (15 mg, 0.37 mmol, 60% oil mixture) was added, and the mixture was stirred for 2 hours at room temperature. Then CH₃I (31 µL, 0.50 mmol) was added to react overnight. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain intermediate A. Intermediate A and AIBN (23 mg, 0.14 mmol) were dissolved in 20mL of ultradry toluene, tributyltin hydride (0.12 mL, 0.45 mmol) was added under stirring, and the mixture was stirred under reflux for 4 hours. The reaction was completed as shown by TLC test. The final reaction solution was concentrated and subjected to column chromatography to obtain the target product A5 (64 mg, 65%).

¹H NMR (600 MHz, CDCl₃) δ 7.40 - 7.08 (m, 26H), 5.29 (t, *J* = 3.4 Hz, 1H), 5.05 (dt, *J =* 16.2, 11.9 Hz, 3H), 4.95 (d, *J =* 11.0 Hz, 1H), 4.83 (dd, *J =* 19.7, 11.0 Hz, 2H), 4.74 (d, *J =* 11.2 Hz, 1H), 4.65 - 4.47 (m, 3H), 4.01 (d, *J* = 5.8 Hz, 1H), 3.78 (t, *J* = 8.9 Hz, 1H), 3.68 (s, 1H), 3.66 - 3.62 (m, 1H), 3.58 (t, *J* = 9.1 Hz, 1H), 3.39 (dt, *J =* 9.7, 3.0 Hz, 1H), 3.31 (dd, *J =* 9.2, 6.7 Hz, 1H), 2.89 (dd, *J* = 13.6, 4.0 Hz, 1H), 1.98 (td, *J* = 13.5, 3.9 Hz, 1H), 1.87 - 1.76 (m, 2H), 1.26 (s, 3H), 1.12 (s, 3H), 0.94 (s, 3H), 0.91 (d, *J* = 1.5 Hz, 4H), 0.89 (s, 3H), 0.85 (s, 3H), 0.61 (s, 3H). LRMS (ESI): 1067.67 [M+H]⁺.

### Example 6 (3S,5S,8R,9R,10S,14R,17R,18S)-Oleanolicacid-methyl-O-D-glucopyranoside (A6)

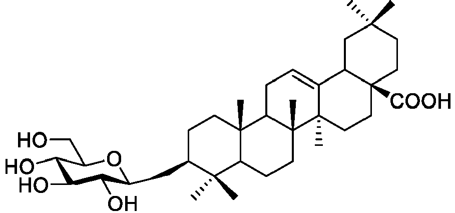

Compounds (3S,5S,8R,9R,10S,14R,17R,18S)-28-O-benzyl-3-aldehydeoleanolic acid (66.7 mg, 0.12 mmol) and 2,3,4,6-O-tetrabenzyl-1-(pyridin-1-yl)sulfonyl-β**-**D-thioglucopyranoside (138.5 mg, 0.24 mmol) were dissolved in 15 mL of redistilled tetrahydrofuran. Under argon protection, 0.1 mol/L of tetrahydrofuran solution of amarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain A1. Then it was dissolved in 10 mL of CS₂, NaH (15 mg, 0.37 mmol, 60% oil mixture) was added, and the mixture was stirred for 2 hours at room temperature. Then CH₃I (31 µL, 0.50 mmol) was added to react overnight. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain intermediate A. Intermediate A and AIBN (23 mg, 0.14 mmol) were dissolved in 20mL of ultradry toluene, tributyltin hydride (0.12 mL, 0.45 mmol) was added under stirring, and the mixture was stirred under reflux for 4 hours. The reaction was completed as shown by TLC test. The final reaction solution was concentrated and subjected to column chromatography to obtain 64 mg of A5. Then it was dissolved in the mixed solution of ethyl acetate: methanol (15 mL: 15 mL), 13 mg of Pd/C was added in hydrogen environment and the mixture was reacted at 50 °C for 12 hours. Then the target product A6 (27 mg, 75%) was obtained by column chromatography.

¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J =* 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J =* 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 7 (3R,5S,8R,9R,10S,14R,17R,18S)-28-O-Benzyloleanolicacid-3-methyl-2",3",4",6"-O-tetrabenzyl-β-D-glucopyranoside (A7)

(3S,5S,8R,9R,10S,14R,17R,18S)-28-O-Benzyl-3-aldehyde oleanolic acid was replaced by (3R,5S,8R,9R,10S,14R,17R,18S)-28-O-benzyl-3-aldehydeoleanolic acid. Other required raw materials, reagents and preparation methods are the same as those in Example 5 to obtain A7 (yield 67%). ¹H NMR (600 MHz, CDCl₃) δ 7.40 - 7.08 (m, 26H), 5.29 (t, *J* = 3.4 Hz, 1H), 5.05 (dt, *J* = 16.2, 11.9 Hz, 3H), 4.95 (d, *J* = 11.0 Hz, 1H), 4.83 (dd, *J* = 19.7, 11.0 Hz, 2H), 4.74 (d, *J* = 11.2 Hz, 1H), 4.65 - 4.47 (m, 3H), 4.01 (d, *J* = 5.8 Hz, 1H), 3.78 (t, *J* = 8.9 Hz, 1H), 3.68 (s, 1H), 3.66 - 3.62 (m, 1H), 3.58 (t, *J* = 9.1 Hz, 1H), 3.39 (dt, *J =* 9.7, 3.0 Hz, 1H), 3.31 (dd, *J* = 9.2, 6.7 Hz, 1H), 2.89 (dd, *J* = 13.6, 4.0 Hz, 1H), 1.98 (td, *J* = 13.5, 3.9 Hz, 1H), 1.87 - 1.76 (m, 2H), 1.26 (s, 3H), 1.12 (s, 3H), 0.94 (s, 3H), 0.91 (d, *J =* 1.5 Hz, 4H), 0.89 (s, 3H), 0.85 (s, 3H), 0.61 (s, 3H). LRMS (ESI): 1067.67 [M+H]⁺.

### Example 8 (3R,5S,8R,9R,10S,14R,17R,18S)-Oleanolicacid-3-methyl-β-D-glucopyranoside (A8)

Replace (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehydeoleanolic acid. Other required raw materials, reagents and preparation methods are the same as those in Example 6 to obtain A8 (yield 71%).¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J =* 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 9 (3S,5S,8R,9R,10S,14R,17R,18S)-28-O-Benzyloleanolicacid-3-carbonyl-2",3",4",6"-O-tetrabenzyl-β-D-glucopyranoside (A9)

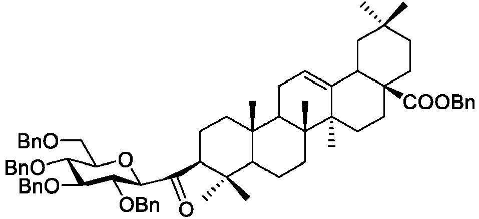

Compounds (3S,5S,8R,9R,10S,14R,17R,18S)-28-O-benzyl-3-aldehydeoleanolic acid (66.7 mg, 0.12 mmol) and 2,3,4,6-O-tetrabenzyl-1-(pyridin-1-yl)sulfonyl-β-D-thioglucopyranoside (138.5 mg, 0.24 mmol) were dissolved in 15 mL of redistilled tetrahydrofuran. Under argon protection, 0.1 mol/L of tetrahydrofuran solution of samarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain 152 mg of A1. Then it was dissolved in 10 mL of dichloromethane, Des Martin reagent (76 mg, 0.18 mmol) was added, and the mixture was stirred for 2 hours at room temperature. The reaction was completed as shown by TLC test. The reaction solution was quenched with saturated sodium thiosulfate and extracted with dichloromethane. The organic layers were combined, washed with saturated sodium chloride solution, dried over anhydrous sodium sulfate and concentrated. The crude product was subjected to column chromatography to obtain the target product A9 (90 mg, 90%). ¹H NMR (600 MHz, CDCl₃) δ 7.40 - 7.08 (m, 26H), 5.29 (t, *J* = 3.4 Hz, 1H), 5.05 (dt, *J* = 16.2, 11.9 Hz, 3H), 4.95 (d, *J =* 11.0 Hz, 1H), 4.83 (dd, *J =* 19.7, 11.0 Hz, 2H), 4.74 (d, *J =* 11.2 Hz, 1H), 4.65 - 4.47 (m, 3H), 4.01 (d, *J =* 5.8 Hz, 1H), 3.78 (t, *J =* 8.9 Hz, 1H), 3.68 (s, 1H), 3.66 - 3.62 (m, 1H), 3.58 (t, *J =* 9.1 Hz, 1H), 3.39 (dt, *J* = 9.7, 3.0 Hz, 1H), 3.31 (dd, *J* = 9.2, 6.7 Hz, 1H), 2.89 (dd, *J* = 13.6, 4.0 Hz, 1H), 1.98 (td, *J* = 13.5, 3.9 Hz, 1H), 1.87 - 1.76 (m, 2H), 1.26 (s, 3H), 1.12 (s, 3H), 0.94 (s, 3H), 0.91 (d, *J* = 1.5 Hz, 4H), 0.89 (s, 3H), 0.85 (s, 3H), 0.61 (s, 3H). LRMS (ESI): 1081.65 [M+H]⁺.

### Example 10. (3S,5S,8R,9R,10S,14R,17R,18S)-Oleanolicacid3-carbonyl-β-D-glucopyranoside (A10)

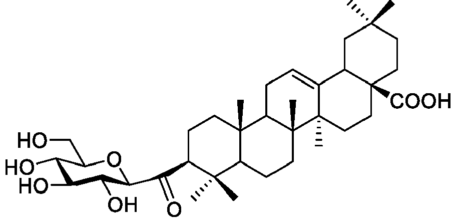

Compounds (3S,5S,8R,9R,10S,14R,17R,18S)-28-O-benzyl-3-aldehydeoleanolic acid (66.7 mg, 0.12 mmol) and 2,3,4,6-O-tetrabenzyl-1-(pyridin-1-yl)sulfonyl-β-D-thioglucopyranoside (138.5 mg, 0.24 mmol) were dissolved in 15 mL of redistilled tetrahydrofuran. Under argon protection, 0.1 mol/L of tetrahydrofuran solution of samarium diiodide (15 mL, 1.5 mmol) was added at 0 °C, and the mixture was stirred for 1 hour. The reaction was completed as shown by TLC test. The reaction solution was quenched with saturated ammonium chloride and extracted with dichloromethane. The organic layers were combined, washed with saturated sodium chloride solution, dried over anhydrous sodium sulfate and concentrated. The crude product was subjected to column chromatography to obtain A1. Then it was dissolved in 10 mL of dichloromethane, Des Martin reagent (76 mg, 0.18 mmol) was added, and the mixture was stirred for 2 hours at room temperature. The reaction was completed as shown by TLC test. The reaction solution was quenched with saturated sodium thiosulfate and extracted with dichloromethane. The organic layers were combined, washed with saturated sodium chloride solution, dried over anhydrous sodium sulfate and concentrated. The crude product was subjected to column chromatography to obtain 90mg of A9. Then it was dissolved in the mixed solution of ethyl acetate: methanol (15 mL: 15 mL), 13 mg of Pd/C was added in hydrogen environment and the mixture was reacted at 50 °C for 12 hours. Then the target product A10 (38 mg, 73%) was obtained by column chromatography.

¹H NMR (500 MHz, MeOD) δ 5.28 (s, 1H), 4.16 (s, 1H), 3.79 (d, *J* = 2.0 Hz, 2H), 3.46 (dd, J = 20.4, 9.3 Hz, 2H), 3.38 (s, 1H), 3.35 - 3.31 (m, 1H), 3.23 (d, *J =* 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J =* 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 11 (3R,5S,8R,9R,10S,14R,17R,18S)-28-O-Benzyloleanolicacid-3-carbonyl-2",3",4",6"-O-tetrabenzyl-β-D-glucopyranoside (A11)

(3S,5S,8R,9R,10S,14R,17R,18S)-28-O-Benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 9 to obtain A11 (yield 89%). ¹H NMR (600 MHz, CDCl₃) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 1081.65 [M+H]⁺.

### Example 12 (3R,5S,8R,9R,10S,14R,17R,18S)-Oleanolic acid-3-carbonyl-β-D-glucopyranoside (A12)

(3S,5S,8R,9R,10S,14R,17R,18S)-28-O-Benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A12 (yield 91%). ¹H NMR (500 MHz, MeOD) δ 5.28 (s, 1H), 4.16 (s, 1H), 3.79 (d, J= 2.0 Hz, 2H), 3.46 (dd, J = 20.4, 9.3 Hz, 2H), 3.38 (s, 1H), 3.35 - 3.31 (m, 1H), 3.23 (d, J = 9.5 Hz, 1H), 3.11 (d, J = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 13 (3S,5S,8R,9R,10S,14R,17R,18S)-Oleanolic acid-3-(1'R)-(hydroxy)methyl-β-D-galactopyranoside (A13)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl) sulfonyl-β-D-thiogalactopyranoside. Other required raw materials, reagents and preparation methods are the same as those in Example 2 to obtain A13 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 14 (3R,5S,8R,9R,10S,14R,17R,18S)-Oleanolic acid-3-(1'R)-(hydroxy)methyl-β-D-galactopyranoside (A14)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A14 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 15 (3S,5S,8R,9R,10S,14R,17R,18S)-Oleanolic acid-3-methyl-β-D-galactopyranoside (A15)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside. Other required raw materials, reagents and preparation methods are the same as those in Example 6 to obtain A15 (yield 63%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 16 (3R,5S,8R,9R,10S,14R,17R,18S)-Oleanolic acid-3-methyl-β-D-galactopyranoside (A16)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A16 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 17 (3S,5S,8R,9R,10S,14R,17R,18S)-Oleanolic acid-3-carbonyl-β-D-galactopyranoside (A17)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside. Other required raw materials, reagents and preparation methods are the same as those in Example 10 to obtain A17 (yield 81%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 18 (3R,5S,8R,9R,10S,14R,17R,18S)-Oleanolic acid-3-carbonyl-β-D-galactopyranoside (A18)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example10 to obtain A18 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 19 (3S,5S,8R,9R,10S,14R,17R,18S)-Oleanolic acid-3-(1'S)-(hydroxy)methyl-α-D-mannopyranoside (A19)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside. Other required raw materials, reagents and preparation methods are the same as those in Example 2 to obtain A19 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 20 (3R,5S,8R,9R,10S,14R,17R,18S)-Oleanolic acid-3-(1'S)-(hydroxy)methyl-α-D-mannopyranoside (A20)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A20 (yield 62%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.43 [M-H]⁻.

### Example 21 (3S,5S,8R,9R,10S,14R,17R,18S)-Oleanolic acid-3-methyl-α-D-mannopyranoside (A21)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside. Other required raw materials, reagents and preparation methods are the same as those in Example 6 to obtain A21 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 22 (3R,5S,8R,9R,10S,14R,17R,18S)-Oleanolic acid-3-methyl-α-D-mannopyranoside (A22)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A22 (yield 64%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 23 (3S,5S,8R,9R,10S,14R,17R,18S)-Oleanolic acid-3-carbonyl-α-D-mannopyranoside (A23)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside. Other required raw materials, reagents and preparation methods are the same as those in Example 10 to obtain A23 (yield 91%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 24 (3R,5S,8R,9R,10S,14R,17R,18S)-Oleanolic acid-3-carbonyl-α-D-mannopyranoside (A24)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiomannopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A24 (yield 86%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 25 (3S,5S,8R,9R,10S,14R,17R,18S)-Oleanolic acid-3-(1'R)-(hydroxy)methyl-β-D-glucurouopyranoside (A25)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside. Other required raw materials, reagents and preparation methods are the same as those in Example 2 to obtain A25 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 26 (3R,5S,8R,9R,10S,14R,17R,18S)-Oleanolic acid-3-(1'R)-(hydroxy)methyl-β-D-glucurouopyranoside (A26)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A26 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 27 (3S,5S,8R,9R,10S,14R,17R,18S)-Oleanolic acid-3-methyl-β-D-glucurouopyranoside (A27)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside. Other required raw materials, reagents and preparation methods are the same as those in Example 6 to obtain A27 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 28 (3R,5S,8R,9R,10S,14R,17R,18S)-Oleanolic acid-3-methyl-β-D-glucurouopyranoside (A28)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A28 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):629.41 [M-H]⁻.

### Example 29 (3S,5S,8R,9R,10S,14R,17R,18S)-Oleanolic acid-3-carbonyl-β-D-glucurouopyranoside (A29)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside. Other required raw materials, reagents and preparation methods are the same as those in Example 10 to obtain A29 (yield 89%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):643.39 [M-H]⁻.

### Example 30 (3R,5S,8R,9R,10S,14R,17R,18S)-Oleanolic acid-3-carbonyl-β-D-glucurouopyranoside (A30)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A30 (yield 88%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):643.39 [M-H]⁻.

### Example 31 (3S,5S,8R,9R,10S,14R,17R,18S)-Oleanolic acid-3-(1'R)-(hydroxy)methyl-6"-deoxy-β-D-glucopyranoside (A31)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside. Other required raw materials, reagents and preparation methods are the same as those in Example 2 to obtain A31 (yield 67%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 32 (3R,5S,8R,9R,10S,14R,17R,18S)-Oleanolic acid-3-(1'R)-(hydroxy)methyl-6"-deoxy-β-D-glucopyranoside (A32)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A32 (yield 78%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 33 (3S,5S,8R,9R,10S,14R,17R,18S)-Oleanolic acid-3-methyl-6" -deoxy-β-D-glucopyranoside (A33)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside. Other required raw materials, reagents and preparation methods are the same as those in Example 6 to obtain A33 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 34 (3R,5S,8R,9R,10S,14R,17R,18S)-Oleanolic acid-3-methyl-6"-deoxy-β-D-glucopyranoside (A34)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A34 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 35 (3S,5S,8R,9R,10S,14R,17R,18S)-Oleanolic acid-3-carbonyl-6"-deoxy-β-D-glucopyranoside (A35)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside. Other required raw materials, reagents and preparation methods are the same as those in Example 10 to obtain A35 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 36 (3R,5S,8R,9R,10S,14R,17R,18S)-Oleanolic acid-3-carbonyl-6"-deoxy-β-D-glucopyranoside (A36)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A36 (yield 85%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 37 (3S,5S,8R,9R,10S,14R,17R,18S)-Oleanolic acid-3-(1'R)-(hydroxy)methyl-β-D-xylopyranoside (A37)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside. Other required raw materials, reagents and preparation methods are the same as those in Example 2 to obtain A37 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):601.42 [M-H]⁻.

### Example 38 (3R,5S,8R,9R,10S,14R,17R,18S)-Oleanolic acid-3-(1'R)-(hydroxy)methyl-β-D-xylopyranoside (A38)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A38 (yield 78%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):601.42 [M-H]⁻.

### Example 39 (3S,5S,8R,9R,10S,14R,17R,18S)-Oleanolic acid-3-methyl-β-D-xylopyranoside (A39)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside. Other required raw materials, reagents and preparation methods are the same as those in Example 6 to obtain A39 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):585.42 [M-H]⁻.

### Example 40 (3R,5S,8R,9R,10S,14R,17R,18S)-Oleanolic acid-3-methyl-β-D-xylopyranoside (A40)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A40 (yield 66%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):585.42 [M-H]⁻.

### Example 41 (3S,5S,8R,9R,10S,14R,17R,18S)-Oleanolic acid-3-carbonyl-β-D-xylopyranoside (A41)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside. Other required raw materials, reagents and preparation methods are the same as those in Example 10 to obtain A41 (yield 88%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.40 [M-H]⁻.

### Example 42 (3R,5S,8R,9R,10S,14R,17R,18S)-Oleanolic acid-3-carbonyl-β-D-xylopyranoside (A42)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A42 (yield 81%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.40 [M-H]⁻.

### Example 43 (3S,5S,8R,9R,10S,14R,17R,18S)-Oleanolic acid-3-(1'S)-(hydroxy)methyl-α-L-rhamnopyranoside (A43)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiorhamnopyranoside. Other required raw materials, reagents and preparation methods are the same as those in Example 2 to obtain A43 (yield 75%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 44 (3R,5S,8R,9R,10S,14R,17R,18S)-Oleanolic acid-3-(1'S)-(hydroxy)methyl-α-L-rhamnopyranoside (A44)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiorhamnopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A44 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 45 (3S,5S,8R,9R,10S,14R,17R,18S)-Oleanolic acid-3-(1'S)-methyl-α-L-rhamnopyranoside (A45)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiorhamnopyranoside. Other required raw materials, reagents and preparation methods are the same as those in Example 6 to obtain A45 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 46 (3R,5S,8R,9R,10S,14R,17R,18S)-Oleanolic acid-3-(1'S)-methyl-α-L-rhamnopyranoside (A46)

2,3,4,6-O-Tetrabenzyl-1-(pyridin-1-yl)sulfonyl-(3-D-thioglucopyranoside was replaced by 2,3,4,6-O-tetrabenzyl-1-(pyridin-1-yl)sulfonyl-α-D-thiorhamnopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A46 (yield 66%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 47 (3S,5S,8R,9R,10S,14R,17R,18S)-Oleanolic acid-3-(1'S)-carbonyl-α-L-rhamnopyranoside (A47)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiorhamnopyranoside. Other required raw materials, reagents and preparation methods are the same as those in Example 10 to obtain A47 (yield 84%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 48 (3R,5S,8R,9R,10S,14R,17R,18S)-Oleanolic acid-3-(1'S)-carbonyl-α-L-rhamnopyranoside (A48)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-α-D-thiorhamnopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A48 (yield 87%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 49 (3S,5S,8R,9R,10S,14R,17R,18S)-Oleanolic acid-3-(1'S)-(hydroxy)methyl-α-L-fucopyranoside (A49)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiofucopyranoside. Other required raw materials, reagents and preparation methods are the same as those in Example 2 to obtain A49 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 50 (3R,5S,8R,9R,10S,14R,17R,18S)-Oleanolic acid-3-(1'S)-(hydroxy)methyl-α-L-fucopyranoside (A50)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiofucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A50 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.43 [M-H]⁻.

### Example 51 (3S,5S,8R,9R,10S,14R,17R,18S)-Oleanolic acid-3-(1'S)-methyl-α-L-fucopyranoside (A51)

2,3,4,6-O-Tetrabenzyl-1-(pyridin-1-yl)sulfonyl-(3-D-thioglucopyranoside was replaced by 2,3,4,6-O-tetrabenzyl-1-(pyridin-1-yl)sulfonyl-β-D-thiofucopyranoside. Other required raw materials, reagents and preparation methods are the same as those in Example 6 to obtain A51 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 52 (3R,5S,8R,9R,10S,14R,17R,18S)-Oleanolic acid-3-(1'S)-methyl-α-L-fucopyranoside (A52)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiofucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A52 (yield 61%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.44 [M-H]⁻.

### Example 53 (3S,5S,8R,9R,10S,14R,17R,18S)-Oleanolic acid-3-(1'S)-carbonyl-α-L-fucopyranoside (A53)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiofucopyranoside. Other required raw materials, reagents and preparation methods are the same as those in Example 10 to obtain A53 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 54 (3R,5S,8R,9R,10S,14R,17R,18S)-Oleanolic acid-3-(1'S)-carbonyl-α-L-fucopyranoside (A54)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiofucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A54 (yield 82%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 55 (3S,5S,8R,9R,10S,14R,17R,18S)-Oleanolic acid-3-(1'R)-(hydroxy)methyl-6"-deoxy-6"-fluoro-β-D-glucopyranoside (A55)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside. Other required raw materials, reagents and preparation methods are the same as those in Example 2 to obtain A55 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):633.42 [M-H]⁻.

### Example 56 (3R,5S,8R,9R,10S,14R,17R,18S)-Oleanolic acid-3-(1'R)-(hydroxy)methyl-6"-deoxy-6"-fluoro-β-D-glucopyranoside (A56)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A56 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):633.42 [M-H]⁻.

### Example 57 (3S,5S,8R,9R,10S,14R,17R,18S)-Oleanolic acid-3-methyl-6"-deoxy-6"-fluoro-β-D-glucopyranoside (A57)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside. Other required raw materials, reagents and preparation methods are the same as those in Example 6 to obtain A57 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43 [M-H]⁻.

### Example 58 3R,5S,8R,9R,10S,14R,17R,18S)-Oleanolic acid-3-methyl-6"-deoxy-6"-fluoro-β-D-glucopyranoside (A58)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A58 (yield 66%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43 [M-H]⁻.

### Example 59 (3S,5S,8R,9R,10S,14R,17R,18S)-Oleanolic acid-3-carbonyl-6"-deoxy-6"-fluoro-β-D-glucopyranoside (A59)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside. Other required raw materials, reagents and preparation methods are the same as those in Example 10 to obtain A59 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.41 [M-H]⁻.

### Example 60 (3R,5S,8R,9R,10S,14R,17R,18S)-Oleanolic acid-3-carbonyl-6"-deoxy-6"-fluoro-β-D-glucopyranoside (A60)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A60 (yield 89%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.41 [M-H]⁻.

### Example 61 (3S,5S,8R,9R,10S,14R,17R,18S)-Oleanolic acid-3-(1'R)-(hydroxy)methyl-β-D-xylopyranosyl(1→3)-β-D-glucurouopyranoside (A61)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside. Other required raw materials, reagents and preparation methods are the same as those in Example 2 to obtain A61 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):777.45[M-H]⁻.

### Example 62 (3R,5S,8R,9R,10S,14R,17R,18S)-Oleanolic acid-3-(1'R)-(hydroxy)methyl-β-D-xylopyranosyl(1→3)-β-D-glucurouopyranoside (A62)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 2 to obtain A62 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):777.45 [M-H]⁻.

### Example 63 (3S,5S,8R,9R,10S,14R,17R,18S)-Oleanolic acid-3-methyl-β-D-xylopyranosyl(1→3)-β-D-glucurouopyranoside (A63)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside. Other required raw materials, reagents and preparation methods are the same as those in Example 6 to obtain A63 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):761.46 [M-H].

### Example 64 (3R,5S,8R,9R,10S,14R,17R,18S)-Oleanolic acid-3-methyl-β-D-xylopyranosyl(1→3)-β-D-glucurouopyranoside (A64)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 6 to obtain A64 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):761.46 [M-H]⁻.

### Example 65 (3S,5S,8R,9R,10S,14R,17R,18S)-Oleanolic acid-3-carbonyl-β-D-xylopyranosyl(1→3)-β-D-glucurouopyranoside (A65)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside. Other required raw materials, reagents and preparation methods are the same as those in Example 10 to obtain A65 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):775.43 [M-H]⁻.

### Example 66 (3R,5S,8R,9R,10S,14R,17R,18S)-Oleanolic acid-3-carbonyl-β-D-xylopyranosyl(1→3)-β-D-glucurouopyranoside (A66)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-xylopyranosyl(1→3)-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S)-28-O-benzyl-3-aldehyde oleanolic acid. Other required raw materials, reagents and preparation methods are the same as those in Example 10 to obtain A66 (yield 89%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):775.43 [M-H]⁻.

### Example 67 (3S,5S,8R,9R,10S,14R,17R,18S,19S,20R)-Ursolic acid-3-(1'R)-(hydroxy)methyl-β-D-glucurouopyranoside (A67)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S,19S,20R)-28-O-benzyl-3-aldehyde ursolic acid. Other required raw materials, reagents and preparation methods are the same as those in Example 2 to obtain A67 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 68 (3R,5S,8R,9R,10S,14R,17R,18S,19S,20R)-Ursolic acid-3-(1'R)-(hydroxy)methyl-β-D-glucurouopyranoside (A68)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S,19S,20R)-28-O-benzyl-3-aldehyde ursolic acid. Other required raw materials, reagents and preparation methods are the same as those in Example 2 to obtain A68 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 69 (3S,5S,8R,9R,10S,14R,17R,18S,19S,20R)-Ursolic acid-3-methyl-β-D-glucurouopyranoside (A69)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S,19S,20R)-28-O-benzyl-3-aldehyde ursolic acid. Other required raw materials, reagents and preparation methods are the same as those in Example 6 to obtain A69 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):613.42 [M-H]⁻.

### Example 70 (3R,5S,8R,9R,10S,14R,17R,18S,19S,20R)-Ursolic acid-3-methyl-β-D-glucurouopyranoside (A70)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S,19S,20R)-28-O-benzyl-3-aldehyde ursolic acid. Other required raw materials, reagents and preparation methods are the same as those in Example 6 to obtain A70 (yield 63%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 71 (3S,5S,8R,9R,10S,14R,17R,18S,19S,20R)-Ursolic acid-3-carbonyl-β-D-glucurouopyranoside (A71)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S,19S,20R)-28-O-benzyl-3-aldehyde ursolic acid. Other required raw materials, reagents and preparation methods are the same as those in Example 10 to obtain A71 (yield 85%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):627.40 [M-H]⁻.

### Example 72 (3R,5S,8R,9R,10S,14R,17R,18S,19S,20R)-Ursolic acid-3-carbonyl-β-D-glucurouopyranoside (A72)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,14R,17R,18S,19S,20R)-28-O-benzyl-3-aldehyde ursolic acid. Other required raw materials, reagents and preparation methods are the same as those in Example 10 to obtain A72 (yield 89%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):627.40 [M-H]⁻.

### Example 73 (3S,5S,8R,9R,10S,13R,14R,17S,18R,19R)-Betulinic acid-3-(1'R)-(hydroxy)methyl-β-D-glucurouopyranoside (A73)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,13R,14R,17S,18R,19R)-28-O-benzyl-3-aldehyde betulinic acid. Other required raw materials, reagents and preparation methods are the same as those in Example 2 to obtain A73 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 74 (3S,5S,8R,9R,10S,13R,14R,17S,18R,19R)-Betulinic acid-3-(1'R)-(hydroxy)methyl-β-D-glucurouopyranoside (A74)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,13R,14R,17S,18R,19R)-28-O-benzyl-3-aldehyde betulinic acid. Other required raw materials, reagents and preparation methods are the same as those in Example 2 to obtain A74 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M-H]⁻.

### Example 75 (3S,5S,8R,9R,10S,13R,14R,17S,18R,19R)-Betulinic acid-3-methyl-β-D-glucurouopyranoside (A75)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,13R,14R,17S,18R,19R)-28-O-benzyl-3-aldehyde betulinic acid. Other required raw materials, reagents and preparation methods are the same as those in Example 6 to obtain A75 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.42 [M-H]⁻.

### Example 76 (3S,5S,8R,9R,10S,13R,14R,17S,18R,19R)-Betulinic acid-3-methyl-β-D-glucurouopyranoside (A76)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,13R,14R,17S,18R,19R)-28-O-benzyl-3-aldehyde betulinic acid. Other required raw materials, reagents and preparation methods are the same as those in Example 6 to obtain A76 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):613.42 [M-H]⁻.

### Example 77 (3S,5S,8R,9R,10S,13R,14R,17S,18R,19R)-Betulinic acid-3-carbonyl-β-D-glucurouopyranoside (A77)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,13R,14R,17S,18R,19R)-28-O-benzyl-3-aldehyde betulinic acid. Other required raw materials, reagents and preparation methods are the same as those in Example 10 to obtain A77 (yield 88%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):627.40 [M-H]⁻.

### Example 78 (3S,5S,8R,9R,10S,13R,14R,17S,18R,19R)-Betulinic acid-3-carbonyl-β-D-glucurouopyranoside (A78)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3R,5S,8R,9R,10S,13R,14R,17S,18R,19R)-28-O-benzyl-3-aldehyde betulinic acid. Other required raw materials, reagents and preparation methods are the same as those in Example 10 to obtain A78 (yield 89%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):627.40 [M-H]⁻.

### Example 79 (5S,8R,9R,10S,14R,17R,18S)-3-Carbonyl oleanolic acid-28-β-D-glucurouopyranoside (A79)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 2 to obtain A79 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.38 [M-H]⁻.

### Example 80 (5S,8R,9R,10S,14R,17R,18S)-3-Carbonyl oleanolic acid-17-(1'R)-(hydroxy)methyl-β-D-glucurouopyranoside (A80)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A80 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.40 [M-H]⁻.

### Example 81 (5S,8R,9R,10S,14R,17R,18S)-3-Carbonyl oleanolic acid-17-methyl-β-D-glucurouopyranoside (A81)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A81 (yield 90%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.40 [M-H]⁻.

### Example 82 (5S,8R,9R,10S,14R,17R,18S19S,20R)-3-Carbonyl ursolic acid-28-β-D-glucurouopyranoside (A82)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S19S,20R)-3-carbonyl-17-aldehyde ursolic acid. Other required raw materials, reagents and preparation methods are the same as those in Example 2 to obtain A82 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.38 [M-H]⁻.

### Example 83 (5S,8R,9R,10S,14R,17R,18S19S,20R)-3-Carbonyl ursolic acid-17-(1'R)-(hydroxy)methyl-β-D-glucurouopyranoside (A83)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S19S,20R)-3-carbonyl-17-aldehyde ursolic acid. Other required raw materials, reagents and preparation methods are the same as those in Example 6 to obtain A83 (yield 69%). ¹HNMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.40 [M-H]⁻.

### Example 84 (5S,8R,9R,10S,14R,17R,18S,19S,20R)-3-Carbonyl ursolic acid-17-methyl-β-D-glucurouopyranoside (A84)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S19S,20R)-3-carbonyl-17-aldehyde ursolic acid. Other required raw materials, reagents and preparation methods are the same as those in Example 10 to obtain A84 (yield 86%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.40 [M-H]⁻.

### Example 85 (5S,8R,9R,10S,13R,14R,17S,18R,19R)-3-Carbonyl betulinic acid-28-β-D-glucurouopyranoside (A85)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,13R,14R,17S,18R,19R)-3-carbonyl-17-aldehyde betulinic acid. Other required raw materials, reagents and preparation methods are the same as those in Example 2 to obtain A85 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):613.38 [M-H]⁻.

### Example 86 (5S,8R,9R,10S,13R,14R,17S,18R,19R)-3-Carbonyl betulinic acid-17-(1'R)-(hydroxy)methyl-β-D-glucurouopyranoside (A86)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,13R,14R,17S,18R,19R)-3-carbonyl-17-aldehyde betulinic acid. Other required raw materials, reagents and preparation methods are the same as those in Example 6 to obtain A86 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):615.40 [M-H]⁻.

### Example 87 (5S,8R,9R,10S,13R,14R,17S,18R,19R)-3-Carbonyl betulinic acid-17-methyl-β-D-glucurouopyranoside (A87)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,13R,14R,17S,18R,19R)-3-carbonyl-17-aldehyde betulinic acid. Other required raw materials, reagents and preparation methods are the same as those in Example 10 to obtain A87 (yield 85%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):599.40 [M-H]⁻.

### Example 88 (3S,5S,8R,9R,10S,14R,17R,18S)-28-O-Ethyl oleanolic acid-3-(1'R)-(hydroxy)methyl-β-D-glucurouopyranoside (A88)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-ethyl-3-aldehyde oleanolic acid. Other required raw materials, reagents and preparation methods are the same as those in Example 2 to obtain A88 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):673.44 [M-H]⁻.

### Example 89 (3S,5S,8R,9R,10S,14R,17R,18S)-28-O-Allyl oleanolic acid-3-(1'R)-(hydroxy)methyl-β-D-glucurouopyranoside (A89)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-allyl-3-aldehyde oleanolic acid. Other required raw materials, reagents and preparation methods are the same as those in Example 2 to obtain A89 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):685.44 [M-H]⁻.

### Example 90 (3S,5S,8R,9R,10S,14R,17R,18S)-28-O-(O-Ethyl)carboxymethyl oleanolic acid-3-(1'R)-(hydroxy)methyl-β-D-glucurouopyranoside (A90)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(O-ethyl)carboxymethyl-3-aldehyde oleanolic acid. Other required raw materials, reagents and preparation methods are the same as those in Example 2 to obtain A90 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):731.44 [M-H]⁻.

### Example 91 (3S,5S,8R,9R,10S,14R,17R,18S)-28-O-Acetoxy oleanolic acid-3-(1'R)-(hydroxy)methyl-β-D-glucurouopyranoside (A91)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-acetoxy-3-aldehyde oleanolic acid. Other required raw materials, reagents and preparation methods are the same as those in Example 2 to obtain A91 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):703.41 [M-H]⁻.

### Example 92 (3S,5S,8R,9R,10S,14R,17R,18S)-28-O-Hydroxyethyl oleanolic acid-3-(1'R)-(hydroxy)methyl-β-D-glucurouopyranoside (A92)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-hydroxyethyl-3-aldehyde oleanolic acid. Other required raw materials, reagents and preparation methods are the same as those in Example 2 to obtain A92 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):689.43 [M-H]⁻.

### Example 93 (3S,5S,8R,9R,10S,14R,17R,18S)-28-O-(N-Methyl) aminoethyl oleanolic acid-3-(1'R)-(hydroxy)methyl-β-D-glucurouopyranoside (A93)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N-methyl) aminoethyl-3-aldehyde oleanolic acid. Other required raw materials, reagents and preparation methods are the same as those in Example 2 to obtain A93 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):702.47 [M-H]⁻.

### Example 94 (3S,5S,8R,9R,10S,14R,17R,18S)-28-O-(N,N-Diethyl)aminoethyl oleanolic acid-3-(1'R)-(hydroxy)methyl-β-D-glucurouopyranoside (A94)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N,N-diethyl)aminoethyl-3-aldehyde oleanolic acid. Other required raw materials, reagents and preparation methods are the same as those in Example 2 to obtain A94 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):744.51 [M-H]⁻.

### Example 95 (3S,5S,8R,9R,10S,14R,17R,18S)-28-O-Hydroxylbut-2"'-ynyl oleanolic acid-3-(1'R)-(hydroxy)methyl-β-D-glucurouopyranoside (A95)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-hydroxylbut-2"'-ynyl-3-aldehyde oleanolic acid. Other required raw materials, reagents and preparation methods are the same as those in Example 2 to obtain A95 (yield 62%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):713.43 [M-H]⁻.

### Example 96 (3S,5S,8R,9R,10S,14R,17R,18S)-28-O-Cyclohexyl oleanolic acid-3-(1'R)-(hydroxy)methyl-β-D-glucurouopyranoside (A96)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-cyclohexyl-3-aldehyde oleanolic acid. Other required raw materials, reagents and preparation methods are the same as those in Example 2 to obtain A96 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):727.49 [M-H]⁻.

### Example 97 (3S,5S,8R,9R,10S,14R,17R,18S)-28-(O-Methyl)carboxymethylamino oleanolic acid-3-(1'R)-(hydroxy)methyl-β-D-glucurouopyranoside (A97)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(O-methyl)carboxymethylamino-3-aldehyde oleanolic acid. Other required raw materials, reagents and preparation methods are the same as those in Example 2 to obtain A97 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):716.45 [M-H]⁻.

### Example 98 (3S,5S,8R,9R,10S,14R,17R,18S)-28-Carboxymethylamino oleanolic acid-3-(1'R)-(hydroxy)methyl-β-D-glucurouopyranoside (A98)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-carboxymethylamino-3-aldehyde oleanolic acid. Other required raw materials, reagents and preparation methods are the same as those in Example 2 to obtain A98 (yield 62%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):702.43 [M-H]⁻.

### Example 99 (3S,5S,8R,9R,10S,14R,17R,18S)-28-(N,N-Diethyl)aminopropylamino oleanolic acid-3-(1'R)-(hydroxy)methyl-β-D-glucurouopyranoside (A99)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N,N-diethyl)aminopropylamino-3-aldehyde oleanolic acid. Other required raw materials, reagents and preparation methods are the same as those in Example 2 to obtain A99 (yield 66%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):757.54 [M-H]⁻.

### Example 100 (3S,5S,8R,9R,10S,14R,17R,18S)-28-(N-Acetyl) aminopropylamino oleanolic acid-3-(1'R)-(hydroxy)methyl-β-D-glucurouopyranoside (A100)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N-acetyl) aminopropylamino-3-aldehyde oleanolic acid. Other required raw materials, reagents and preparation methods are the same as those in Example 2 to obtain A100 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):743.49 [M-H]⁻.

### Example 101 (3S,5S,8R,9R,10S,14R,17R,18S)-28-(N,N-Diethyl)aminohexylamino oleanolic acid-3-(1'R)-(hydroxy)methyl-(3-D-glucurouopyranoside (A101)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N,N-diethyl)aminohexylamino-3-aldehyde oleanolic acid. Other required raw materials, reagents and preparation methods are the same as those in Example 2 to obtain A101 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J =* 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):799.59 [M-H]⁻.

### Example 102 (3S,5S,8R,9R,10S,14R,17R,18S)-28-(N-Acetyl) aminohexylamino oleanolic acid-3-(1'R)-(hydroxy)methyl-β-D-glucurouopyranoside (A102)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N-acetyl)aminohexylamino-3-aldehyde oleanolic acid. Other required raw materials, reagents and preparation methods are the same as those in Example 2 to obtain A102 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):785.54 [M-H]⁻.

### Example 103 (3S,5S,8R,9R,10S,14R,17R,18S)-28-(N-Ethyl) aminohexylamino oleanolic acid-3-(1'R)-(hydroxy)methyl-β-D-glucurouopyranoside (A103)

2,3,4,6-O-tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(N-ethyl) aminohexylamino-3-aldehyde oleanolic acid. Other required raw materials, reagents and preparation methods are the same as those in Example 2 to obtain A103 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):771.56 [M-H]⁻.

### Example 104 (3S,5S,8R,9R,10S,14R,17R,18S)-28-O-(Pyrazol-1"-yl)methyl oleanolic acid-3-(1'R)-(hydroxy)methyl-β-D-glucurouopyranoside (A104)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(pyrazol-1-yl)methyl-3-aldehyde oleanolic acid. Other required raw materials, reagents and preparation methods are the same as those in Example 2 to obtain A104 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):725.45 [M-H]⁻.

### Example 105 (3S,5S,8R,9R,10S,14R,17R,18S)-28-O-(Piperidin-1"-yl)methyl oleanolic acid-3-(1'R)-(hydroxy)methyl-β-D-glucurouopyranoside (A105)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(piperidin-1-yl)methyl-3-aldehyde oleanolic acid. Other required raw materials, reagents and preparation methods are the same as those in Example 2 to obtain A105 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):756.51 [M-H]⁻.

### Example 106 (3S,5S,8R,9R,10S,14R,17R,18S)-28-O-(Morpholin-4"-yl)methyl oleanolic acid-3-(1'R)-(hydroxy)methyl-β-D-glucurouopyranoside (A106)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-28-O-(morpholin-4-yl)methyl-3-aldehyde oleanolic acid. Other required raw materials, reagents and preparation methods are the same as those in Example 2 to obtain A106 (yield 67%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):758.49 [M-H]⁻.

### Example 107 (3S,5S,8R,9R,10S,14R,17R,18S)-Oleanolic acid-3-(1'R)-fluoromethyl-β-D-glucurouopyranoside (A107)

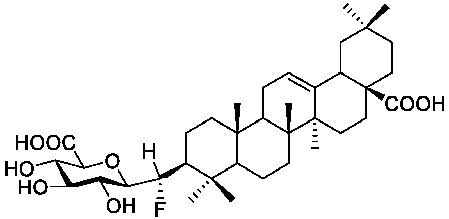

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and Des Martin reagent was replaced by DAST. Other required raw materials, reagents and preparation methods are the same as those in Example 10 to obtain A107 (yield 65%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J =* 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):647.40 [M-H]⁻.

### Example 108 (3S,5S,8R,9R,10S,14R,17R,18S)-Oleanolic acid-3-(1'R)-(hydroxy)methyl-28-(1"R)-(hydroxy)methyl-β-D-diglucurouopyranoside (A108)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (3S,5S,8R,9R,10S,14R,17R,18S)-3,17-dialdehyde oleanolic acid. Other required raw materials, reagents and preparation methods are the same as those in Example 2 to obtain A108 (yield 62%). ¹H NMR (500 MHz, MeOD) δ 5.28 (s, 1H), 4.16 (s, 1H), 3.79 (d, *J* = 2.0 Hz, 4H), 3.46 (dd, *J =* 20.4, 9.3 Hz, 4H), 3.38 (s, 2H), 3.35 - 3.31 (m, 2H), 3.23 (d, *J* = 9.5 Hz, 2H), 3.11 (d, *J* = 9.5 Hz, 2H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):807.46 [M-H]⁻.

### Example 109 (3S,5S,8R,9R,10S,14R,17R,18S)-Oleanolic acid-3-(1'R)-(hydroxy)methyl-6"-O-methyl-β-D-glucurouopyranoside (A109)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-O-methyl-(3-D-thioglucurouopyranoside. Other required raw materials, reagents and preparation methods are the same as those in Example 2 to obtain A109 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):659.42 [M-H]⁻.

### Example 110 (3S,5S,8R,9R,10S,14R,17R,18S)-Oleanolic acid-3-(1'R)-(hydroxy)methyl-6"-O-n-butyl-β-D-glucurouopyranoside (A110)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-0-n-butyl-β-D-thioglucurouopyranoside. Other required raw materials, reagents and preparation methods are the same as those in Example 2 to obtain A110 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):701.47 [M-H]⁻.

### Example 111 (3S,5S,8R,9R,10S,14R,17R,18S)-Oleanolic acid-3-(1'R)-(hydroxy)methyl-6"-O-isobutyl-(3-D-glucurouopyranoside (A111)

2,3,4,6-O-Tetrabenzyl-1-(pyridin-1-yl)sulfonyl-β-D-thioglucopyranoside was replaced by 2,3,4, 6-O-tetrabenzyl-1-(pyridin-1-yl) sulfonyl-6-O-isobutyl-β-D-thioglucurouopyrano side. Other required raw materials, reagents and preparation methods are the same as those in Example 2 to obtain A111 (yield 61%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):701.47 [M-H]⁻.

### Example 112 (3S,5S,8R,9R,10S,14R,17R,18S)-Oleanolic acid-3-(1'R)-(hydroxy)methyl-6"-O-benzyl-β-D-glucurouopyranoside (A112)

2,3,4,6-O-Tetrabenzyl-1-(pyridin-1-yl)sulfonyl-β-D-thioglucopyranoside was replaced by 2,3,4,6-0-tetrabenzyl-1-(pyridin-1-yl)sulfonyl-6-0-benzyl-β-D-thioglucurouopyranoside. Other required raw materials, reagents and preparation methods are the same as those in Example 2 to obtain A112 (yield 68%). ¹H NMR (500 MHz, MeOD) δ 7.32 (m, 5H), 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):735.46 [M-H]⁻.

### Example 113 (3S,5S,8R,9R,10S,14R,17R,18S)-Oleanolic acid-3-(1'R)-(hydroxy)methyl-6"-O-(1-fluoroacetyl)-β-D-glucurouopyranoside (A113)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6"-O-(1-fluoroacetyl)-β-D-thioglucurouopyranoside. Other required raw materials, reagents and preparation methods are the same as those in Example 2 to obtain A113 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):658.44 [M-H]⁻.

### Example 114 (3S,5S,8R,9R,10S,14R,17R,18S)-Oleanolic acid-3-(1'R)-(hydroxy)methyl-6"-methylamino-β-D-glucurouopyranoside (A114)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-methylamino-β-D-thioglucurouopyranoside. Other required raw materials, reagents and preparation methods are the same as those in Example 2 to obtain A114 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):658.44 [M-H]⁻.

### Example 115 (3S,5S,8R,9R,10S,14R,17R,18S)-Oleanolic acid-3-(1'R)-(hydroxy)methyl-6"-butylamino-β-D-glucurouopyranoside (A115)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-butylamino-β-D-thioglucurouopyranoside. Other required raw materials, reagents and preparation methods are the same as those in Example 2 to obtain A115(yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):600.49 [M-H]⁻.

### Example 116 (3S,5S,8R,9R,10S,14R,17R,18S)-Oleanolic acid-3-(1'R)-(hydroxy)methyl-6"-isobutylamino-β-D-glucurouopyranoside (A116)

2,3,4,6-O-Tetrabenzyl-1-(pyridin-1-yl)sulfonyl-β-D-thioglucopyranoside was replaced by 2,3,4,6-0-tetrabenzyl-1-(pyridin-1-yl)sulfonyl-6-isobutylamino-β-D-thioglucurouopyranoside. Other required raw materials, reagents and preparation methods are the same as those in Example 2 to obtain A116 (yield 80%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):700.49 [M-H]⁻.

### Example 117 (3S,5S,8R,9R,10S,14R,17R,18S)-Oleanolic acid-3-(1'R)-(hydroxy)methyl-6"-benzylamino-β-D-glucurouopyranoside (A117)

2,3,4,6-O-Tetrabenzyl-1-(pyridin-1-yl)sulfonyl-β-D-thioglucopyranoside was replaced by 2,3,4,6-0-tetrabenzyl-1-(pyridin-1-yl)sulfonyl-6-benzylamino-β-D-thioglucurouopyranoside. Other required raw materials, reagents and preparation methods are the same as those in Example 2 to obtain A117 (yield 82%). ¹H NMR (500 MHz, MeOD) δ 7.33(m, 5H), 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):734.47 [M-H]⁻.

### Example 118 (3S,5S,8R,9R,10S,14R,17R,18S)-Oleanolic acid-3-(1'R)-(hydroxy)methyl-6"-dimethylamino-β-D-glucurouopyranoside (A118)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-6-dimethylamino-β-D-thioglucurouopyranoside. Other required raw materials, reagents and preparation methods are the same as those in Example 2 to obtain A118 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J =* 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):672.46 [M-H]⁻.

### Example 119 (3S,5S,8R,9R,10S,14R,17R,18S)-Oleanolic acid-3-(1'R)-(hydroxy)methyl-6"-(1-fluoroethylamino)-(3-D-glucurouopyranoside (A119)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl) sulfonyl-6-(1-fluoroethylamino)-β-D-thioglucurouopyranoside. Other required raw materials, reagents and preparation methods are the same as those in Example 2 to obtain A119 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):672.46 [M-H]⁻.

### Example 120 (3S,5S,8R,9R,10S,14R,17R,18S)-Oleanolic acid-3-(1'R)-(hydroxy)methyl-5"-cyano-β-D-xylopyranoside (A120)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-5-cyano-β-D-thioxylopyranoside. Other required raw materials, reagents and preparation methods are the same as those in Example 2 to obtain A120 (yield 78%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):626.41 [M-H]⁻.

### Example 121 (5S,8R,9R,10S,14R,17R,18S)-3-Carbonyl oleanolic acid-17-(1'S)-(hydroxy)methyl-β-D-mannopyranoside (A121)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A121 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):603.42 [M+H]⁺.

### Example 122 (3S,5S,8R,9R,10S,14R,17R,18S)-Oleanolic acid-17-(1'S)-(hydroxy)methyl-β-D-mannopyranoside (A122)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A122 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):605.43 [M+H]⁺.

### Example 123 (3S,5S,8R,9R,10S,14R,17R,18S)-3-Methoxy oleanolic acid-17-(1'S)-(hydroxy)methyl-β-D-mannopyranoside (A123)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-methoxy-17-aldehyde oleanolic acid. Other required raw materials, reagents and preparation methods are the same as those in Example 6 to obtain A123 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):619.45 [M+H]⁺.

### Example 124 (3S,5S,8R,9R,10S,14R,17R,18S)-3-(4-Fluorocyclohexoxy) oleanolic acid-17-(1'S)-(hydroxy)methyl-β-D-mannopyranoside (A124)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-fluorocyclohexoxy)-17-aldehyde oleanolic acid. Other required raw materials, reagents and preparation methods are the same as those in Example 6 to obtain A124 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):705.50 [M+H]⁺.

### Example 125 (3S,5S,8R,9R,10S,14R,17R,18S)-3-Acetoxy oleanolic acid-17-(1'S)-(hydroxy)methyl-β-D-mannopyranoside (A125)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-acetoxy-17-aldehyde oleanolic acid. Other required raw materials, reagents and preparation methods are the same as those in Example 6 to obtain A125 (yield77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):647.44 [M+H]⁺.

### Example 126 (3S,5S,8R,9R,10S,14R,17R,18S)-3-Fluoroacetoxy oleanolic acid-17-(1'S)-(hydroxy)methyl-β-D-mannopyranoside (A126)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-fluoroacetoxy-17-aldehyde oleanolic acid. Other required raw materials, reagents and preparation methods are the same as those in Example 6 to obtain A126 (yield 72%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J =* 11.4 Hz, 1H). LRMS (ESI):665.44 [M+H]⁺.

### Example 127 (3S,5S,8R,9R,10S,14R,17R,18S)-3-(4-Fluorophenylacetoxy) oleanolic acid-17-(1'S)-(hydroxy)methyl-β-D-mannopyranoside (A127)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-fluorophenylacetoxy)-17-aldehyde oleanolic acid. Other required raw materials, reagents and preparation methods are the same as those in Example 6 to obtain A127 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):727.45 [M+H]⁺.

### Example 128 (3S,5S,8R,9R,10S,14R,17R,18S)-3-(4-Trifluoromethylphenylsulfonyloxy) oleanolic acid-17-(1'S)-(hydroxy)methyl-β-D-mannopyranoside (A128)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-trifluoromethylphenylsulfonyloxy)-17-aldehyde oleanolic acid. Other required raw materials, reagents and preparation methods are the same as those in Example 6 to obtain A128 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):813.41 [M+H]⁺.

### Example 129 (3S,5S,8R,9R,10S,14R,17R,18S)-3-(2,3,5,6-Tetrafluorobenzamide) oleanolic acid-17-(1'S)-(hydroxy)methyl-β-D-mannopyranoside (A129)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(2,3,5,6-tetrafluorobenzamide)-17-aldehyde oleanolic acid. Other required raw materials, reagents and preparation methods are the same as those in Example 6 to obtain A129 (yield 76%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):780.44 [M+H]⁺.

### Example 130 (3S,5S,8R,9R,10S,14R,17R,18S)-3-(4-Trifluoromethylbenzenesulfonamido)oleanolic acid-17-(1'S)-(hydroxy)methyl-β-D-mannopyranoside (A130)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-trifluoromethylbenzenesulfonamido)-17-aldehyde oleanolic acid. Other required raw materials, reagents and preparation methods are the same as those in Example 6 to obtain A130 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):812.43 [M+H]⁺.

### Example 131 (5S,8R,9R,10S,14R,17R,18S)-3-Oximido oleanolic acid-17-(1'S)-(hydroxy)methyl-β-D-mannopyranoside (A131)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-oximido-17-aldehyde oleanolic acid. Other required raw materials, reagents and preparation methods are the same as those in Example 6 to obtain A131 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):618.43 [M+H]⁺.

### Example 132 (5S,8R,9R,10S,14R,17R,18S)-3-Methylhydrazinylidene oleanolic acid-17-(1'S)-(hydroxy)methyl-β-D-mannopyranoside (A132)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-methylhydrazinylidene-17-aldehyde oleanolic acid. Other required raw materials, reagents and preparation methods are the same as those in Example 6 to obtain A132 (yield 79%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.46 [M+H]⁺.

### Example 133 (5S,8R,9R,10S,14R,17R,18S)-3-Phenylhydrazinylidene oleanolic acid-17-(1'S)-(hydroxy)methyl-β-D-mannopyranoside (A133)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-phenylhydrazinylidene-17-aldehyde oleanolic acid. Other required raw materials, reagents and preparation methods are the same as those in Example 6 to obtain A133 (yield 76%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):693.48 [M+H]⁺.

### Example 134 (3S,5S,8R,9R,10S,14R,17R,18S)-3-(4-Fluorophenylureido) oleanolic acid-17-(1'S)-(hydroxy)methyl-β-D-mannopyranoside (A134)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-fluorophenylureido)-17-aldehyde oleanolic acid. Other required raw materials, reagents and preparation methods are the same as those in Example 6 to obtain A134 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):741.48 [M+H]⁺.

### Example 135 (3S,5S,8R,9R,10S,14R,17R,18S)-3-(4-Fluorophenylthioureido) oleanolic acid-17-(1'S)-(hydroxy)methyl-β-D-mannopyranoside (A135)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(4-fluorophenylthioureido)-17-aldehyde oleanolic acid. Other required raw materials, reagents and preparation methods are the same as those in Example 6 to obtain A135 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):757.45 [M+H]⁺.

### Example 136 (3S,5S,8R,9R,10S,14R,17R,18S)-3-Formyl oleanolic acid-17-(1'S)-(hydroxy)methyl-β-D-mannopyranoside (A136)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-formyl-17-aldehyde oleanolic acid. Other required raw materials, reagents and preparation methods are the same as those in Example 6 to obtain A136 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43 [M+H]⁺.

### Example 137 (3S,5S,8R,9R,10S,14R,17R,18S)-3-(3-Fluorocyclobutylthio)oleanolic acid-17-(1'S)-(hydroxy)methyl-β-D-mannopyranoside (A137)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-(3-fluorocyclobutylthio)-17-aldehyde oleanolic acid. Other required raw materials, reagents and preparation methods are the same as those in Example 6 to obtain A137 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):693.45 [M+H]⁺.

### Example 138 (3S,5S,8R,9R,10S,14R,17R,18S)-3-Phenylthio oleanolic acid-17-(1'S)-(hydroxy)methyl-β-D-mannopyranoside (A138)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-phenylthio-17-aldehyde oleanolic acid. Other required raw materials, reagents and preparation methods are the same as those in Example 6 to obtain A138 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):697.44 [M+H]⁺.

### Example 139 (5S,8R,9R,10S,14R,17R,18S)-3-Carbonyl oleanolic acid-28-β-D-mannopyranoside (A139)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 2 to obtain A139 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):601.40 [M+H]⁺.

### Example 140 (5S,8R,9R,10S,14R,17R,18S)-3-Carbonyl oleanolic acid-17-(1'S)-fluoromethyl-β-D-mannopyranoside (A140)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-fluoromethyl-17-aldehyde oleanolic acid, and Des Martin reagent was replaced by DAST. Other required raw materials, reagents and preparation methods are the same as those in Example 10 to obtain A140 (yield 67%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):645.41 [M+H]⁺.

### Example 141 (5S,8R,9R,10S,14R,17R,18S)-3-Carbonyl oleanolic acid-17-methyl-β-D-mannopyranoside (A141)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A141 (yield 80%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):587.42 [M+H]⁺.

### Example 142 (5S,8R,9R,10S,14R,17R,18S)-3-Carbonyl oleanolic acid-17-(1'S)-(hydroxy)methyl-2",3 ",4",6"-O-tetraacetyl-β-D-mannopyranoside (A142)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A142 (yield 67%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):771.46 [M+H]⁺.

### Example 143 (3S,5S,8R,9R,10S,14R,17R,18S)-Oleanolic acid-17-(1'S)-(hydroxy)methyl-2",3 ",4",6"-O-tetraacetyl-β-D-mannopyranoside (A143)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A143 (yield 66%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 773.48 [M+H]⁺.

### Example 144 (5S,8R,9R,10S,14R,17R,18S)-3-Carbonyl oleanolic acid-17-methyl-2",3",4",6"-O-tetraacetyl-β-D-mannopyranoside (A144)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A144 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):755.47 [M+H]⁺.

### Example 145 (5S,8R,9R,10S,14R,17R,18S)-3-Carbonyl oleanolic acid-17-(2'S)-(hydroxy)ethyl-β-D-mannopyranoside (A145)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A145 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43 [M+H]⁺.

### Example 146 (5S,8R,9R,10S,14R,17R,18S)-3-Carbonyloleanolic acid-17-(2'S)-(hydroxy)ethyl-2",3 ",4",6"-O-tetraacetyl-β-D-mannopyranoside (A146)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5'S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A146 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):685.48 [M+H]⁺.

### Example 147 (5S,8R,9R,10S,14R,17R,18S)-3-Carbonyl ursolic acid-17-(2'S)-(hydroxy)ethyl-2",3 ",4",6"-O-tetraacetyl-β-D-mannopyranoside (A147)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A147 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):685.48 [M+H]⁺.

### Example 148 (5S,8R,9R,10S,14R,17R,18S)-3-Carbonyl betulinic acid-17-(2'S)-(hydroxy)ethyl-2",3 ",4",6"-O-tetraacetyl-β-D-mannopyranoside (A148)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiomannopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A148 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):685.48 [M+H]⁺.

### Example 149 (5S,8R,9R,10S,14R,17R,18S)-3-Carbonyl oleanolic acid-17-(1'R)-(hydroxy)methyl-β-D-xylopyranoside (A149)

2,3,4,6-O-Tetrabenzyl-1-(pyridin-1-yl)sulfonyl-β-D-thioglucopyranoside was replaced by 2,3,4-O-tribenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A149 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):573.41 [M+H]⁺.

### Example 150 (5S,8R,9R,10S,14R,17R,18S)-3-Carbonyl oleanolic acid-17methyl-β-D-xylopyranoside (A150)

2,3,4,6-O-Tetrabenzyl-1-(pyridin-1-yl)sulfonyl-β-D-thioglucopyranoside was replaced by 2,3,4-O-tribenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A150 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):557.41 [M+H]⁺.

### Example 151 (3S,5S,8R,9R,10S,14R,17R,18S)-Oleanolic acid-17-(1'R)-(hydroxy)methyl-β-D-xylopyranoside (A151)

2,3,4,6-O-Tetrabenzyl-1-(pyridin-1-yl)sulfonyl-β-D-thioglucopyranoside was replaced by 2,3,4-O-tribenzyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A151 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):575.42 [M+H]⁺.

### Example 152 (5S,8R,9R,10S,14R,17R,18S)-3-Carbonyl oleanolic acid-17-(1'R)-(hydroxy)methyl-2",3",4"-O-triacetyl-β-D-xylopyranoside (A152)

2,3,4,6-O-Tetrabenzyl-1-(pyridin-1-yl)sulfonyl-β-D-thioglucopyranoside was replaced by 2,3,4-O-triacetyl-1-(pyridin-1-yl)sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A152 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):698.44 [M+H]⁺.

### Example 153 (5S,8R,9R,10S,14R,17R,18S)-3-Carbonyl oleanolic acid-17-methyl-2",3",4"-O-triacetyl-β-D-xylopyranoside (A153)

2,3,4,6-O-Tetrabenzyl-1-(pyridin-1-yl)sulfonyl-β-D-thioglucopyranoside was replaced by 2,3,4-O-triacetyl-1-(pyridin-1-yl) sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A153 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):683.44 [M+H]⁺.

### Example 154 (5S,8R,9R,10S,14R,17R,18S)-3-Carbonyl oleanolic acid-17-(2'R)-(hydroxy)ethyl-2",3",4"-O-triacetyl-β-D-xylopyranoside (A154)

2,3,4,6-O-Tetrabenzyl-1-(pyridin-1-yl)sulfonyl-β-D-thioglucopyranoside was replaced by 2,3,4-O-triacetyl-1-(pyridin-1-yl) sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A154 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):713.46 [M+H]⁺.

### Example 155 (5S,8R,9R,10S,14R,17R,18S)-3-Carbonyl ursolic acid-17-(2'R)-(hydroxy)ethyl-2",3",4"-O-triacetyl-β-D-xylopyranoside (A155)

2,3,4,6-O-Tetrabenzyl-1-(pyridin-1-yl)sulfonyl-β**-**D-thioglucopyranoside was replaced by 2,3,4-O-triacetyl-1-(pyridin-1-yl) sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A155 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):713.46 [M+H]⁺.

### Example 156 (5S,8R,9R,10S,14R,17R,18S)-3-Carbonyl betulinic acid-17-(2'R)-(hydroxy)ethyl-2",3",4"-O-triacetyl-β-D-xylopyranoside (A156)

2,3,4,6-O-Tetrabenzyl-1-(pyridin-1-yl)sulfonyl-β**-**D-thioglucopyranoside was replaced by 2,3,4-O-triacetyl-1-(pyridin-1-yl) sulfonyl-β-D-thioxylopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A156 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) :713.46 [M+H]⁺.

### Example 157 (5S,8R,9R,10S,14R,17R,18S)-3-Carbonyl oleanolic acid-17-(1'R)-(hydroxy)methyl-β-D-galactopyranoside (A157)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A157 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):603.42 [M+H]⁺.

### Example 158 (5S,8R,9R,10S,14R,17R,18S)-3-Carbonyl oleanolic acid-17-methyl-β-D-galactopyranoside (A158)

2,3,4,6-O-Tetrabenzyl-1-(pyridin-1-yl)sulfonyl-β**-**D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A158 (yield 76%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):587.42 [M+H]⁺.

### Example 159 (3S,5S,8R,9R,10S,14R,17R,18S)-Oleanolic acid-17-(1'R)-(hydroxy)methyl-β-D-galactopyranoside (A159)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A159 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):605.43 [M+H]⁺.

### Example 160 (5S,8R,9R,10S,14R,17R,18S)-3-Carbonyl oleanolic acid-17-(1'R)-(hydroxy)methyl-2",3 ",4",6"-O-tetraacetyl-β-D-galactopyranoside (A160)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A160 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):771.46 [M+H]⁺.

### Example 161 (5S,8R,9R,10S,14R,17R,18S)-3-Carbonyl oleanolic acid-17-methyl-2",3",4",6"-O-tetraacetyl-P-D-galactopyranoside (A 161)

2,3,4,6-O-Tetrabenzyl-1-(pyridin-1-yl)sulfonyl-β-D-thioglucopyranoside was replaced by 2,3,4,6-O-tetraacetyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A161 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):755.47 [M+H]⁺.

### Example 162 (5S,8R,9R,10S,14R,17R,18S)-3-Carbonyl oleanolic acid-17-(2'R)-(hydroxy)ethyl-β-D-galactopyranoside (A162)

2,3,4,6-O-Tetrabenzyl-1-(pyridin-1-yl)sulfonyl-β-D-thioglucopyranoside was replaced by 2,3,4,6-O-tetrabenzyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A162 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43 [M+H]⁺.

### Example 163 (5S,8R,9R,10S,14R,17R,18S)-3-Carbonyl oleanolic acid-17-(2'R)-(hydroxy)ethyl-2",3 ",4",6"-O-tetraacetyl-β-D-galactopyranoside (A163)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A163 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):785.48 [M+H]⁺.

### Example 164 (5S,8R,9R,10S,14R,17R,18S)-3-Carbonyl ursolic acid-17-(2'R)-(hydroxy)ethyl-2",3 ",4",6"-O-tetraacetyl-β-D-galactopyranoside (A164)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A164 (yield 68%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 785.48 [M+H]⁺.

### Example 165 (5S,8R,9R,10S,14R,17R,18S)-3-Carbonyl betulinic acid-17-(2'R)-(hydroxy)ethyl-2",3 ",4",6"-O-tetraacetyl-β-D-galactopyranoside (A165)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiogalactopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A165 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) : 785.48 [M+H]⁺.

### Example 166 (5S,8R,9R,10S,14R,17R,18S)-3-Carbonyl oleanolic acid-17-(1'R)-(hydroxy)methyl-α-L-rhamnopyranoside (A166)

2,3,4,6-O-Tetrabenzyl-1-(pyridin-1-yl)sulfonyl-β-D-thioglucopyranoside was replaced by 2,3,4-O-tribenzyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A166 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):587.42 [M+H]⁺.

### Example 167 (5S,8R,9R,10S,14R,17R,18S)-3-Carbonyl oleanolic acid-17-methyl-α-L-rhamnopyranoside (A167)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A167 (yield 74%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):571.43 [M+H]⁺.

### Example 168 (3S,5S,8R,9R,10S,14R,17R,18S)-Oleanolic acid-17-(1'R)-(hydroxy)methyl-α-L-rhamnopyranoside (A168)

2,3,4,6-O-Tetrabenzyl-1-(pyridin-1-yl)sulfonyl-β-D-thioglucopyranoside was replaced by 2,3,4-O-tribenzyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A168 (yield 75%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):589.44 [M+H]⁺.

### Example 169 (5S,8R,9R,10S,14R,17R,18S)-3-Carbonyl oleanolic acid-17-(1'R)-(hydroxy)methyl-2",3",4"-O-triacetyl-α-L-rhamnopyranoside (A169)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A169 (yield 73%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):713.46 [M+H]⁺.

### Example 170 (5S,8R,9R,10S,14R,17R,18S)-3-Carbonyl oleanolic acid-17-methyl-2",3",4"-O-triacetyl-α-L-rhamnopyranoside (A170)

2,3,4,6-O-Tetrabenzyl-1-(pyridin-1-yl)sulfonyl-β**-**D-thioglucopyranoside was replaced by 2,3,4-O-triacetyl-1-(pyridin-1-yl) sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A170 (yield 74%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):697.46 [M+H]⁺.

### Example 171 (5S,8R,9R,10S,14R,17R,18S)-3-Carbonyl oleanolic acid-17-(2'R)-(hydroxy)ethyl-α-L-rhamnopyranoside (A171)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A171 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):601.44 [M+H]⁺.

### Example 172 (5S,8R,9R,10S,14R,17R,18S)-3-Carbonyl oleanolic acid-17-(2'R)-(hydroxy)ethyl-2",3",4"-O-triacetyl-α-L-rhamnopyranoside (A172)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A172 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):727.47 [M+H]⁺.

### Example 173 (5S,8R,9R,10S,14R,17R,18S)-3-Carbonyl ursolic acid-17-(2'R)-(hydroxy)ethyl-2",3",4"-O-triacetyl-α-L-rhamnopyranoside (A173)

2,3,4,6-O-Tetrabenzyl-1-(pyridin-1-yl)sulfonyl-β**-**D-thioglucopyranoside was replaced by 2,3,4-O-triacetyl-1-(pyridin-1-yl) sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A173 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 727.47 [M+H]⁺.

### Example 174 (5S,8R,9R,10S,14R,17R,18S)-3-Carbonyl betulinic acid-17-(2'R)-(hydroxy)ethyl-2",3",4"-O-triacetyl-α-L-rhamnopyranoside (A174)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A174 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) : 727.47 [M+H]⁺.

### Example 175 (3S,5S,8R,9R,10S,14R,17R,18S)-Oleanolic acid-17-(1'R)-(hydroxy)methyl-β-D-glucurouopyranoside (A175)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A175 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):619.41 [M+H]⁺.

### Example 176 (5S,8R,9R,10S,14R,17R,18S)-3-Carbonyl oleanolic acid-17-carbonyl-2",3",4"-O-triacetyl-β-D-glucurouopyranoside (A176)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A176 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):741.41 [M+H]⁺.

### Example 177 (5S,8R,9R,10S,14R,17R,18S)-3-Carbonyl oleanolic acid-17-(1'R)-(hydroxy)methyl-2",3",4"-O-triacetyl-β-D-glucurouopyranoside (A177)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A177 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):743.43 [M+H]⁺.

### Example 178 (5S,8R,9R,10S,14R,17R,18S)-3-Carbonyl oleanolic acid-17-methyl-2",3",4"-O-triacetyl-β-D-glucurouopyranoside (A178)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A178 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):727.43 [M+H]⁺.

### Example 179 (5S,8R,9R,10S,14R,17R,18S)-3-Carbonyl oleanolic acid-17-(2'R)-(hydroxy)ethyl-β-D-glucurouopyranoside (A179)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A179 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):631.41 [M+H]⁺.

### Example 180 (5S,8R,9R,10S,14R,17R,18S)-3-Carbonyl oleanolic acid-17-(2'R)-(hydroxy)ethyl-2",3",4"-O-triacetyl-β-D-glucurouopyranoside (A180)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A180 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):757.44 [M+H]⁺.

### Example 181 (5S,8R,9R,10S,14R,17R,18S)-3-Carbonyl ursolic acid-17-(2'R)-(hydroxy)ethyl-2",3",4"-O-triacetyl-β-D-glucurouopyranoside (A181)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A181 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 757.44 [M+H]⁺.

### Example 182 (5S,8R,9R,10S,14R,17R,18S)-3-Carbonyl betulinic acid-17-(2'R)-(hydroxy)ethyl-2",3",4"-O-triacetyl-β-D-glucurouopyranoside (A182)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-β-D-thioglucurouopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A182 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) : 757.44 [M+H]⁺.

### Example 183 (5S,8R,9R,10S,14R,17R,18S)-3-Carbonyl oleanolic acid-17-(1'R)-(hydroxy)methyl-6"-deoxy-6"-fluoro-β-D-glucopyranoside (A183)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A183 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):605.41 [M+H]⁺.

### Example 184 (5S,8R,9R,10S,14R,17R,18S)-3-Carbonyl oleanolic acid-17methyl-6"-deoxy-6" -fluoro-β-D-glucopyranoside (A184)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A184 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):589.42 [M+H]⁺.

### Example 185 (3S,5S,8R,9R,10S,14R,17R,18S)-Oleanolic acid-17-(1'R)-(hydroxy)methyl-6"-deoxy-6"-fluoro-β-D-glucopyranoside (A185)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A185 (yield 75%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):607.43 [M+H]⁺.

### Example 186 (5S,8R,9R,10S,14R,17R,18S)-3-Carbonyl oleanolic acid-17-(1'R)-(hydroxy)methyl-2",3",4"-O-triacetyl-6"-deoxy-6"-fluoro-β-D-glucopyranoside (A186)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A186 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):731.45 [M+H]⁺.

### Example 187 (5S,8R,9R,10S,14R,17R,18S)-3-Carbonyl oleanolic acid-17-methyl-2",3",4"-O-triacetyl-6"-deoxy-6"-fluoro-β-D-glucopyranoside (A187)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A187 (yield 75%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):715.45 [M+H]⁺.

### Example 188 (5S,8R,9R,10S,14R,17R,18S)-3-Carbonyl oleanolic acid-17-(2'R)-(hydroxy)ethyl-6"-deoxy-6"-fluoro-β-D-glucopyranoside (A188)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A188 (yield 76%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J =* 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):619.43 [M+H]⁺.

### Example 189 (5S,8R,9R,10S,14R,17R,18S)-3-Carbonyl oleanolic acid-17-(2'R)-(hydroxy)ethyl-2",3",4"-O-triacetyl-6"-deoxy-6"-fluoro-β-D-glucopyranoside (A189)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A189 (yield 69%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):745.46 [M+H]⁺.

### Example 190 (5S,8R,9R,10S,14R,17R,18S)-3-Carbonyl ursolic acid-17-(2'R)-(hydroxy)ethyl-2",3",4"-O-triacetyl-6"-deoxy-6"-fluoro-β-D-glucopyranoside (A190)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A190 (yield 67%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 745.46 [M+H]⁺.

### Example 191 (5S,8R,9R,10S,14R,17R,18S)-3-Carbonyl betulinic acid-17-(2'R)-(hydroxy)ethyl-2",3",4"-O-triacetyl-6"-deoxy-6"-fluoro-β-D-glucopyranoside (A191)

2,3,4,6-O-Tetrabenzyl-1-(pyridin-1-yl)sulfonyl-β**-**D-thioglucopyranoside was replaced by 2,3,4-O-triacetyl-1-(pyridin-1-yl)sulfonyl-6-deoxy-6-fluoro-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A191 (yield 71%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) : 745.46 [M+H]⁺.

### Example 192 (5S,8R,9R,10S,14R,17R,18S)-3-Carbonyl oleanolic acid-17-(1'R)-(hydroxy)methyl-β-D-glucopyranoside (A192)

2,3,4,6-O-Tetrabenzyl-1-(pyridin-1-yl)sulfonyl-β**-**D-thioglucopyranoside was replaced by 2,3,4,6-O-tetrabenzyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A192 (yield 70%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):603.42 [M+H]⁺.

### Example 193 (5S,8R,9R,10S,14R,17R,18S)-3-Carbonyl oleanolic acid-17methyl--β-D-glucopyranoside (A193)

2,3,4,6-O-Tetrabenzyl-1-(pyridin-1-yl)sulfonyl-β**-**D-thioglucopyranoside was replaced by 2,3,4,6-O-tetrabenzyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A193 (yield 84%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):587.42 [M+H]⁺.

### Example 194 (3S,5S,8R,9R,10S,14R,17R,18S)-Oleanolic acid-17-(1'R)-(hydroxy)methyl-β-D-glucopyranoside (A194)

2,3,4,6-O-Tetrabenzyl-1-(pyridin-1-yl)sulfonyl-β**-**D-thioglucopyranoside was replaced by 2,3,4,6-O-tetrabenzyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-hydroxy-17-aldehyde oleanolic acid. Other required raw materials, reagents and preparation methods are the same as those in Example 6 to obtain A194 (yield 79%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, J = 7.1 Hz, 2H), 1.86 - 1.05 (m, 21H), 1.06 - 0.93 (m, 1H), 0.90 - 0.82 (m, 6H), 0.81 (d, *J* = 11.4 Hz, 1H). LRMS (ESI):605.43[M+H]⁺.

### Example 195 (5S,8R,9R,10S,14R,17R,18S)-3-Carbonyl oleanolic acid-17-(1'R)-(hydroxy)methyl-2",3 ",4",6"-O-tetraacetyl-β-D-glucopyranoside (A195)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 6 to obtain A195 (yield 79%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):771.46 [M+H]⁺.

### Example 196 (5S,8R,9R,10S,14R,17R,18S)-3-Carbonyl oleanolic acid-17-methyl-2",3",4",6"-O-tetraacetyl-β-D-glucopyranoside (A196)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-aldehyde oleanolic acid. Other required raw materials, reagents and preparation methods are the same as those in Example 10 to obtain A196 (yield 78%). ¹H NMR (500 MHz, MeOD) δ 5.28 (s, 1H), 4.16 (s, 1H), 3.79 (d, *J =* 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):755.47 [M+H]⁺.

### Example 197 (5S,8R,9R,10S,14R,17R,18S)-3-Carbonyl oleanolic acid-17-(2'R)-(hydroxy)ethyl-β-D-glucopyranoside (A197)

2,3,4,6-O-Tetrabenzyl-1-(pyridin-1-yl)sulfonyl-β**-**D-thioglucopyranoside was replaced by 2,3,4,6-O-tetrabenzyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A197 (yield 75%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):617.43[M+H]⁺.

### Example 198 (5S,8R,9R,10S,14R,17R,18S)-3-Carbonyl oleanolic acid-17-(2'R)-(hydroxy)ethyl-2",3",4",6"-O-tetraacetyl-P-D-glucopyranoside (A 198)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thioglucopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl oleanolic acid. Other required raw materials, reagents and preparation methods are the same as those in Example 6 to obtain A198 (yield 72%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI):785.48 [M+H]⁺.

### Example 199 (5S,8R,9R,10S,14R,17R,18S)-3-Carbonyl ursolic acid-17-(2'R)-(hydroxy)ethyl-2",3",4",6"-O-tetraacetyl-β-D-glucopyranoside (A199)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl ursolic acid. Other required raw materials, reagents and preparation methods are the same as those in Example 6 to obtain A199 (yield 79%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI): 785.48 [M+H]⁺.

### Example 200 (5S,8R,9R,10S,14R,17R,18S)-3-Carbonyl betulinic acid-17-(2'R)-(hydroxy)ethyl-2",3",4",6"-O-tetraacetyl-β-D-glucopyranoside (A200)

2,3,4,6-O-Tetrabenzyl-1-(pyridin-1-yl)sulfonyl-β**-**D-thioglucopyranoside was replaced by 2,3,4,6-O-tetraacetyl-1-(pyridin-1-yl)sulfonyl-β-D-thiorhamnopyranoside, and (3S,5S,8R,9R,10S,14R,17R,18S)-28-O-benzyl-3-aldehyde oleanolic acid was replaced by (5S,8R,9R,10S,14R,17R,18S)-3-carbonyl-17-formylmethyl betulinic acid. Other required raw materials, reagents and preparation methods are the same as those in Example 6 to obtain A200 (yield 77%). ¹H NMR (500 MHz, MeOD) δ 5.28 (s, 1H), 4.16 (s, 1H), 3.79 (d, *J* = 2.0 Hz, 2H), 3.46 (dd, *J* = 20.4, 9.3 Hz, 2H), 3.38 (s, 1H), 3.35 - 3.31 (m, 1H), 3.23 (d, *J* = 9.5 Hz, 1H), 3.11 (d, *J* = 9.5 Hz, 1H), 2.88 (dd, *J* = 13.5, 3.0 Hz, 1H), 2.03 (dd, *J* = 13.5, 10.3 Hz, 1H), 1.93 (d, *J* = 7.1 Hz, 2H), 1.86 - 1.05 (m, 21H), 1.06 - 0.93 (m, 12H), 0.90 - 0.82 (m, 6H), 0.81 (d, *J* = 11.4 Hz, 1H). LRMS (ESI) : 785.48 [M+H]⁺.

### Example 201 (5S,8R,9R,10S,14R,17R,18S)-3-Carbonyl oleanolic acid-28-β-D-glucopyranoside (A201)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A201(yield 72%). ¹H NMR (500 MHz, CDCl₃) δ 5.48 (d, *J* = 5.9 Hz, 1H), 5.33 (tdd, *J* = 5.1, 1.8, 1.1 Hz, 1H), 5.07 (d, *J* = 5.7 Hz, 1H), 4.57 (d, *J* = 6.3 Hz, 1H), 4.44 (dd, *J* = 8.8, 2.6 Hz, 1H), 4.19 (t, *J* = 4.5 Hz, 1H), 3.71 - 3.58 (m, 3H), 3.58 (dt, *J* = 7.7, 3.5 Hz, 1H), 3.58 - 3.49 (m, 1H), 3.31 (dddd, *J* = 8.5, 7.8, 5.8, 1.8 Hz, 1H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.87 (m, 1H), 1.91 - 1.84 (m, 1H), 1.86 - 1.81 (m, 1H), 1.84 - 1.78 (m, 2H), 1.72 - 1.59 (m, 6H), 1.62 - 1.48 (m, 3H), 1.51 - 1.41 (m, 1H), 1.44 - 1.36 (m, 2H), 1.40 - 1.33 (m, 1H), 1.36 - 1.29 (m, 1H), 1.15 (s, 2H), 1.08 (t, J = 1.6 Hz, 5H), 0.99 - 0.92 (m, 6H), 0.93 (d, J = 6.6 Hz, 5H). LRMS (ESI): 601.4 [M+H]⁺.

### Example 202 (5S,8R,9R,10S,14R,17R,18S)-3-Carbonyl ursolic acid-28-β-D-glucopyranoside (A202)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl ursolic acid. Other required raw materials, reagents and preparation methods are the same as those in Example 10 to obtain A202(yield 72%). ¹H NMR (500 MHz, CDCl₃) δ 5.53 - 5.46 (m, 2H), 5.07 (d, J = 5.7 Hz, 1H), 4.57 (d, J = 6.3 Hz, 1H), 4.51 (dd, J = 8.8, 2.6 Hz, 1H), 4.19 (t, J = 4.5 Hz, 1H), 3.71 - 3.58 (m, 3H), 3.58 (dt, J = 7.7, 3.5 Hz, 1H), 3.58 - 3.49 (m, 1H), 3.31 (dddd, J = 8.4, 7.8, 5.8, 1.8 Hz, 1H), 2.48 (ddd, J = 12.5, 6.5, 4.0 Hz, 1H), 2.40 (ddd, J = 12.5, 6.6, 4.0 Hz, 1H), 2.28 - 2.22 (m, 1H), 2.06 - 1.91 (m, 2H), 1.89 - 1.78 (m, 4H), 1.77 (ddd, J = 12.5, 7.7, 5.2 Hz, 1H), 1.73 - 1.57 (m, 5H), 1.60 - 1.54 (m, 1H), 1.57 - 1.51 (m, 1H), 1.48 - 1.41 (m, 1H), 1.44 - 1.38 (m, 2H), 1.41 - 1.35 (m, 1H), 1.35 (dtd, J = 6.5, 3.2, 1.6 Hz, 1H), 1.34 - 1.25 (m, 1H), 1.20 (s, 2H), 1.08 (t, J = 1.6 Hz, 5H), 0.99 - 0.90 (m, 9H), 0.91 (d, J = 2.0 Hz, 2H). LRMS (ESI): 601.4 [M+H]⁺.

### Example 203 (5S,8R,9R,10S,14R,17R,18S)-3-Carbonyl ursolic acid-17-(1'R)-(hydroxy)methyl-β-D-glucopyranoside (A203)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl ursolic acid. Other required raw materials, reagents and preparation methods are the same as those in Example 2 to obtain A203(yield 64%). ¹H NMR (500 MHz,CDCl₃) δ 5.47 (tdd, J = 4.9, 1.7, 1.0 Hz, 1H), 4.95 (d, J = 5.3 Hz, 1H), 4.81 (dd, J = 11.0, 5.7 Hz, 2H), 4.61 (d, J = 6.2 Hz, 1H), 4.20 (t, J = 4.5 Hz, 1H), 4.00 - 3.88 (m, 2H), 3.72 (dddd, J = 8.8, 7.9, 6.1, 2.8 Hz, 1H), 3.70 - 3.65 (m, 1H), 3.68 - 3.55 (m, 4H), 2.48 (ddd, J = 12.5, 6.5, 4.0 Hz, 1H), 2.40 (ddd, J = 12.5, 6.6, 4.0 Hz, 1H), 2.06 - 1.86 (m, 4H), 1.84 (ddd, J = 6.6, 2.9, 1.5 Hz, 1H), 1.84 - 1.78 (m, 1H), 1.71 - 1.64 (m, 1H), 1.65 (dd, J = 3.9, 1.6 Hz, 1H), 1.66 - 1.54 (m, 4H), 1.57 - 1.51 (m, 1H), 1.54 - 1.44 (m, 1H), 1.47 - 1.39 (m, 2H), 1.42 - 1.35 (m, 3H), 1.37 - 1.32 (m, 1H), 1.35 - 1.25 (m, 1H), 1.11 - 1.05 (m, 8H), 0.99 - 0.88 (m, 11H). LRMS (ESI): 603.4 [M+H]⁺.

### Example 204 (3S,5S,8R,9R,10S,14R,17R,18S)-Ursolic acid-17-(1'R)-(hydroxy)methyl-β-D-glucopyranoside (A204)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl ursolic acid. Other required raw materials, reagents and preparation methods are the same as those in Example 2 to obtain A204(yield 50%). ¹H NMR (500 MHz, CDCl₃) δ 5.47 (tdd, J = 4.9, 1.7, 1.0 Hz, 1H), 4.95 (d, J = 5.3 Hz, 1H), 4.81 (dd, J = 11.0, 5.7 Hz, 2H), 4.61 (d, J = 6.2 Hz, 1H), 4.20 (t, J = 4.5 Hz, 1H), 4.00 - 3.88 (m, 2H), 3.76 - 3.55 (m, 6H), 3.31 - 3.23 (m, 1H), 3.00 (d, J = 10.8 Hz, 1H), 2.01 - 1.86 (m, 4H), 1.74 - 1.25 (m, 19H), 1.09 (s, 2H), 0.97 (d, J = 1.5 Hz, 3H), 0.95 - 0.90 (m, 9H), 0.93 - 0.87 (m, 6H). LRMS (ESI): 605.4 [M+H]⁺.

### Example 205 (3S,5S,8R,9R,10S,14R,17R,18S)-Ursolic acid-28-β-D-glucopyranoside (A205)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl ursolic acid. Other required raw materials, reagents and preparation methods are the same as those in Example 10 to obtain A205(yield 50%). ¹H NMR (500 MHz, CDCl₃) δ 5.53 - 5.46 (m, 2H), 5.07 (d, J = 5.7 Hz, 1H), 4.57 (d, J = 6.3 Hz, 1H), 4.51 (dd, J = 8.8, 2.6 Hz, 1H), 4.19 (t, J = 4.5 Hz, 1H), 3.71 - 3.49 (m, 5H), 3.36 - 3.23 (m, 2H), 3.00 (d, J = 10.8 Hz, 1H), 2.28 - 2.22 (m, 1H), 2.01 - 1.93 (m, 1H), 1.89 - 1.24 (m, 20H), 1.20 (s, 2H), 0.99 - 0.87 (m, 16H). LRMS (ESI): 603.4 [M+H]⁺.

### Example 206 (3S,5S,8R,9R,10S,14R,17R,18S)-Oleanolic acid-28-β-D-glucopyranoside (A206)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl oleanolic acid. Other required raw materials, reagents and preparation methods are the same as those in Example 10 to obtain A206(yield 50%). ¹H NMR (500 MHz, CDCl₃) δ 5.48 (d, J = 5.9 Hz, 1H), 5.33 (tdd, J = 5.1, 1.8, 1.1 Hz, 1H), 5.07 (d, J = 5.7 Hz, 1H), 4.57 (d, J = 6.3 Hz, 1H), 4.44 (dd, J = 8.8, 2.6 Hz, 1H), 4.19 (t, J = 4.5 Hz, 1H), 3.71 - 3.58 (m, 3H), 3.58 (dt, J = 7.7, 3.5 Hz, 1H), 3.58 - 3.49 (m, 1H), 3.36 - 3.23 (m, 2H), 3.00 (d, J = 10.8 Hz, 1H), 2.17 (tq, J = 5.1, 1.0 Hz, 1H), 2.05 (dddd, J = 12.5, 6.2, 5.1, 1.0 Hz, 1H), 1.96 - 1.78 (m, 3H), 1.74 - 1.39 (m, 15H), 1.42 - 1.27 (m, 4H), 1.15 (s, 2H), 0.99 - 0.87 (m, 17H). LRMS (ESI): 603.4 [M+H]⁺.

### Example 207 (3S,5S,8R,9R,10S,13R,14R,17S,18R,19R)-Betulinic acid-28-β-D-glucopyranoside (A207)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl betulinic acid. Other required raw materials, reagents and preparation methods are the same as those in Example 10 to obtain A207(yield 55%). ¹H NMR (500 MHz, CDCl₃) δ 5.48 (d, J = 5.9 Hz, 1H), 5.07 (d, J = 5.7 Hz, 1H), 4.83 (h, J = 1.6 Hz, 1H), 4.67 (h, J = 1.5 Hz, 1H), 4.57 (d, J = 6.3 Hz, 1H), 4.47 (dd, J = 8.8, 2.5 Hz, 1H), 4.19 (t, J = 4.5 Hz, 1H), 3.72 - 3.49 (m, 5H), 3.36 - 3.23 (m, 2H), 3.04 - 2.96 (m, 2H), 1.82 - 1.48 (m, 22H), 1.51 - 1.47 (m, 1H), 1.50 - 1.43 (m, 1H), 1.45 - 1.35 (m, 1H), 1.31 (ddq, J = 6.8, 4.0, 1.5 Hz, 1H), 1.18 (ddp, J = 7.4, 4.4, 1.5 Hz, 1H), 0.99 - 0.91 (m, 11H), 0.82 (t, J = 1.5 Hz, 3H). LRMS (ESI): 603.4 [M+H]⁺.

### Example 208 (3S,5S,8R,9R,10S,13R,14R,17S,18R,19R)-Betulinic acid-17-(1'R)-(hydroxy)methyl-β-D-glucopyranoside (A208)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl betulinic acid. Other required raw materials, reagents and preparation methods are the same as those in Example 2 to obtain A208(yield 56%). ¹H NMR (500 MHz, CDCl₃) δ 4.95 (d, *J* = 5.1 Hz, 1H), 4.84 - 4.79 (m, 2H), 4.69 (d, *J* = 5.3 Hz, 1H), 4.65 (h, *J* = 1.6 Hz, 1H), 4.61 (d, *J* = 6.2 Hz, 1H), 4.20 (t, *J* = 4.5 Hz, 1H), 3.93 (dddd, *J* = 8.6, 7.6, 5.9, 1.8 Hz, 1H), 3.85 (ddd, *J* = 7.7, 5.1, 2.8 Hz, 1H), 3.72 (dddd, *J* = 8.8, 7.5, 6.0, 2.9 Hz, 1H), 3.71 - 3.64 (m, 1H), 3.68 - 3.61 (m, 2H), 3.64 - 3.58 (m, 1H), 3.57 (ddd, *J* = 12.3, 4.6, 3.3 Hz, 1H), 3.31 - 3.23 (m, 1H), 2.18 - 2.09 (m, 1H), 1.76 - 1.27 (m, 25H), 1.18 (ddp, *J* = 7.4, 4.4, 1.5 Hz, 1H), 0.99 - 0.90 (m, 11H), 0.82 (t, *J* = 1.5 Hz, 3H). LRMS (ESI): 605.4 [M+H]⁺.

### Example 209 (5S,8R,9R,10S,13R,14R,17S,18R,19R)-3-Carbonyl betulinic acid-17-(1'R)-(hydroxy)methyl-β-D-glucopyranoside (A209)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl betulinic acid. Other required raw materials, reagents and preparation methods are the same as those in Example 2 to obtain A209(yield 60%). ¹H NMR (500 MHz, CDCl₃) δ 4.84 - 4.79 (m, 1H), 4.71 - 4.59 (m, 1H), 3.76 - 3.53 (m, 2H), 2.53 - 2.41 (m, 1H), 1.86 - 1.76 (m, 1H), 1.73 (q, *J* = 1.4 Hz, 1H), 1.63 - 1.28 (m, 7H), 1.13 - 1.05 (m, 2H), 0.97 (d, *J* = 1.5 Hz, 1H), 0.92 (d, *J* = 1.5 Hz, 1H), 0.85 (t, *J* = 1.4 Hz, 1H). LRMS (ESI): 603.4 [M+H]⁺.

### Example 210 (5S,8R,9R,10S,13R,14R,17S,18R,19R)-3-Carbonyl betulinic acid-28-β-D-glucopyranoside (A210)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl betulinic acid. Other required raw materials, reagents and preparation methods are the same as those in Example 10 to obtain A210(yield 51%). ¹H NMR (500 MHz, CDCl₃) δ 5.48 (d, *J* = 5.9 Hz, 1H), 5.07 (d, *J* = 5.7 Hz, 1H), 4.83 (h, *J* = 1.6 Hz, 1H), 4.67 (h, *J* = 1.5 Hz, 1H), 4.57 (d, *J* = 6.3 Hz, 1H), 4.47 (dd, *J* = 8.8, 2.5 Hz, 1H), 4.19 (t, *J* = 4.5 Hz, 1H), 3.72 - 3.49 (m, 5H), 3.31 (dddd, *J* = 8.4, 7.7, 5.8, 1.8 Hz, 1H), 3.00 (dddt, *J* = 10.3, 5.0, 3.3, 1.6 Hz, 1H), 2.53 - 2.41 (m, 2H), 1.86 - 1.59 (m, 11H), 1.62 - 1.57 (m, 1H), 1.59 - 1.53 (m, 2H), 1.51 (ddddd, *J* = 12.4, 7.7, 6.5, 2.6, 1.4 Hz, 7H), 1.50 - 1.40 (m, 3H), 1.44 - 1.34 (m, 2H), 1.13 - 1.05 (m, 7H), 0.96 (dd, *J* = 12.7, 1.5 Hz, 6H), 0.85 (t, *J* = 1.4 Hz, 3H). LRMS (ESI): 601.4 [M+H]⁺.

### Example 211 (5S,8R,9R,10S,13R,14R,17S,18R,19R)-3-Carbonyl betulinic acid-17-methyl-β-D-glucopyranoside (A211)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl betulinic acid. Other required raw materials, reagents and preparation methods are the same as those in Example 6 to obtain A211(yield 61%). ¹H NMR (500 MHz, CDCl₃) δ 4.85 (d, *J* = 5.9 Hz, 1H), 4.83 - 4.78 (m, 2H), 4.65 (h, *J* = 1.5 Hz, 1H), 4.61 (d, *J* = 6.4 Hz, 1H), 4.23 - 4.16 (m, 1H), 3.88 (ddtd, *J* = 32.6, 9.0, 7.5, 2.1 Hz, 2H), 3.73 (dddd, *J* = 8.8, 7.7, 6.2, 2.2 Hz, 1H), 3.70 - 3.63 (m, 2H), 3.60 - 3.51 (m, 2H), 2.53 - 2.41 (m, 2H), 2.23 (dtq, *J* = 7.0, 5.3, 1.7 Hz, 1H), 1.96 (dd, *J* = 13.8, 7.2 Hz, 1H), 1.83 (ddd, *J* = 5.7, 2.8, 1.4 Hz, 1H), 1.83 - 1.76 (m, 1H), 1.73 (q, *J* = 1.5 Hz, 3H), 1.70 - 1.60 (m, 1H), 1.63 - 1.57 (m, 1H), 1.59 - 1.54 (m, 1H), 1.57 - 1.44 (m, 10H), 1.47 - 1.42 (m, 1H), 1.44 - 1.37 (m, 3H), 1.40 - 1.33 (m, 1H), 1.21 (dd, *J* = 6.9, 5.6 Hz, 1H), 1.13 - 1.05 (m, 7H), 0.97 (d, *J* = 1.5 Hz, 3H), 0.92 (d, *J* = 1.3 Hz, 3H), 0.85 (t, *J* = 1.5 Hz, 3H). LRMS (ESI): 587.4[M+H]⁺.

### Example 212 (5S,8R,9R,10S,14R,17R,18S)-3-Carbonyl ursolic acid-17-methyl-β-D-glucopyranoside (A212)

Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl ursolic acid. Other required raw materials, reagents and preparation methods are the same as those in Example 6 to obtain A212(yield 57%). ¹H NMR (500 MHz, CDCl₃) δ 5.45 (tdd, *J* = 4.9, 1.8, 1.1 Hz, 1H), 4.85 (d, *J* = 5.9 Hz, 1H), 4.80 (d, *J* = 5.7 Hz, 1H), 4.61 (d, *J* = 6.4 Hz, 1H), 4.23 - 4.17 (m, 1H), 3.96 - 3.88 (m, 1H), 3.84 (dtd, *J* = 9.3, 7.0, 2.3 Hz, 1H), 3.73 (dddd, *J* = 8.9, 7.9, 6.3, 2.3 Hz, 1H), 3.70 - 3.62 (m, 2H), 3.62 - 3.51 (m, 2H), 2.48 (ddd, *J* = 12.5, 6.5, 4.0 Hz, 1H), 2.40 (ddd, *J* = 12.5, 6.6, 4.0 Hz, 1H), 2.07 - 2.01 (m, 1H), 2.04 - 1.98 (m, 1H), 1.96 (dddd, *J* = 12.5, 6.3, 4.9, 1.0 Hz, 1H), 1.84 (ddd, *J* = 6.6, 2.9, 1.5 Hz, 1H), 1.84 - 1.78 (m, 1H), 1.80 - 1.70 (m, 3H), 1.70 - 1.20 (m, 15H), 1.11 - 1.05 (m, 9H), 0.97 (d, *J* = 1.5 Hz, 3H), 0.95 - 0.86 (m, 9H). LRMS (ESI): 587.4[M+H]⁺.

### Example 213 (3S,5S,8R,9R,10S,14R,17R,18S)-Oleanolic acid-17-methyl-β-D-glucopyranoside (A213)

Compound (3S,5S,8R,9R,10S,14R,17R,18S)-28-O-benzyl-3-aldehyde oleanolic acid was replaced by (3S, and 5S,8R,9R,10S,14R,17R,18S)-17-formyl oleanolic acid. Other required raw materials, reagents and preparation methods are the same as those in Example 6 to obtain A213(yield 56%). ¹H NMR (500 MHz, CDCl₃) δ 5.26 (tdd, *J* = 5.3, 1.8, 1.0 Hz, 1H), 4.85 (d, *J* = 5.9 Hz, 1H), 4.80 (d, *J* = 5.7 Hz, 1H), 4.61 (d, *J* = 6.4 Hz, 1H), 4.23 - 4.17 (m, 1H), 3.96 - 3.87 (m, 1H), 3.84 - 3.70 (m, 2H), 3.70 - 3.62 (m, 2H), 3.62 - 3.51 (m, 2H), 3.31 - 3.23 (m, 1H), 3.00 (d, *J* = 10.8 Hz, 1H), 2.68 (tq, *J* = 4.8, 1.0 Hz, 1H), 2.03 (dd, *J* = 14.0, 6.7 Hz, 1H), 1.85 (dddd, *J* = 12.7, 6.4, 5.2, 1.0 Hz, 1H), 1.76 (dddd, *J =* 12.5, 6.4, 5.1, 1.0 Hz, 1H), 1.74 - 1.20 (m, 22H), 1.09 (s, 2H), 0.99 - 0.87 (m, 17H). LRMS (ESI): 589.4[M+H]⁺.

### Example 214 (3S,5S,8R,9R,10S,14R,17R,18S)-3-Carbonyl ursolic acid-17-methyl-β-D-glucopyranoside (A214)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl ursolic acid. Other required raw materials, reagents and preparation methods are the same as those in Example 6 to obtain A214(yield 56%). ¹H NMR (500 MHz, CDCl₃) δ 5.45 (tdd, *J* = 5.0, 1.8, 1.1 Hz, 1H), 4.85 (d, *J* = 5.9 Hz, 1H), 4.80 (d, *J* = 5.7 Hz, 1H), 4.61 (d, *J* = 6.4 Hz, 1H), 4.23 - 4.17 (m, 1H), 3.96 - 3.88 (m, 1H), 3.84 (dtd, *J* = 9.3, 7.0, 2.3 Hz, 1H), 3.73 (dddd, *J* = 8.9, 7.9, 6.3, 2.3 Hz, 1H), 3.70 - 3.62 (m, 2H), 3.62 - 3.51 (m, 2H), 3.31 - 3.23 (m, 1H), 3.00 (d, *J* = 10.8 Hz, 1H), 2.03 (dd, *J* = 13.9, 7.0 Hz, 1H), 2.00 - 1.91 (m, 2H), 1.80 - 1.20 (m, 23H), 1.09 (s, 2H), 0.97 (d, *J* = 1.5 Hz, 3H), 0.95 - 0.86 (m, 14H). LRMS (ESI): 589.4[M+H]⁺.

### Example 215 (3S,5S,8R,9R,10S,13R,14R,17S,18R,19R)-Betulinic acid-17-methyl-β-D-glucopyranoside (A215)

Compound (3S,5S,8R,9R,10S,14R,17R,18S)-28-O-benzyl-3-aldehyde oleanolic acid was replaced by (3S,5S,8R,9R,10S,14R,17R,18S)-17-formyl betulinic acid. Other required raw materials, reagents and preparation methods are the same as those in Example 6 to obtain A215(yield 48%). ¹H NMR (500 MHz, CDCl₃) δ 4.85 (d, *J* = 5.9 Hz, 1H), 4.83 - 4.78 (m, 2H), 4.65 (h, *J* = 1.5 Hz, 1H), 4.61 (d, *J* = 6.4 Hz, 1H), 4.23 - 4.16 (m, 1H), 3.88 (ddtd, *J* = 32.6, 9.0, 7.5, 2.1 Hz, 2H), 3.73 (dddd, *J* = 8.8, 7.7, 6.2, 2.2 Hz, 1H), 3.70 - 3.63 (m, 2H), 3.60 - 3.51 (m, 2H), 3.31 - 3.23 (m, 1H), 3.00 (d, *J =* 10.8 Hz, 1H), 2.23 (dtq, *J* = 7.0, 5.3, 1.7 Hz, 1H), 1.96 (dd, *J* = 13.8, 7.2 Hz, 1H), 1.76 - 1.33 (m, 24H), 1.31 (ddq, *J* = 6.8, 4.1, 1.5 Hz, 1H), 1.24 - 1.15 (m, 2H), 0.99 - 0.91 (m, 11H), 0.82 (t, *J* = 1.5 Hz, 3H). LRMS (ESI): 589.4[M+H]⁺.

### Example 216 (3R,5S,8R,9R,10S,14R,17R,18S)-Oleanolic acid-28-β-D-glucopyranoside (A216)

Compound (3S,5S,8R,9R,10S,14R,17R,18S)-28-O-benzyl-3-aldehyde oleanolic acid was replaced by (3R,5S,8R,9R,10S,14R,17R,18S)-17-formyl oleanolic acid. Other required raw materials, reagents and preparation methods are the same as those in Example 10 to obtain A216(yield 55%). ¹H NMR (500 MHz, CDCl₃) δ 5.48 (d, *J* = 5.9 Hz, 1H), 5.33 (tdd, *J* = 5.1, 1.8, 1.1 Hz, 1H), 5.07 (d, *J* = 5.7 Hz, 1H), 4.57 (d, *J* = 6.2 Hz, 1H), 4.44 (dd, *J* = 8.8, 2.6 Hz, 1H), 4.19 (t, *J* = 4.5 Hz, 1H), 3.67 (ddd, *J* = 10.9, 3.9, 2.8 Hz, 1H), 3.67 - 3.61 (m, 1H), 3.64 - 3.49 (m, 3H), 3.36 - 3.25 (m, 2H), 3.00 (d, *J* = 10.8 Hz, 1H), 2.17 (tq, *J* = 5.1, 1.0 Hz, 1H), 2.05 (dddd, *J* = 12.5, 6.2, 5.1, 1.0 Hz, 1H), 1.96 - 1.79 (m, 3H), 1.79 - 1.40 (m, 15H), 1.43 - 1.27 (m, 4H), 1.15 (s, 2H), 0.99 - 0.88 (m, 17H). LRMS (ESI): 603.4[M+H]⁺.

### Example 217 (3R,5S,8R,9R,10S,14R,17R,18S)-Oleanolic acid-17-(1'R)-(hydroxy)methyl-β-D-glucopyranoside (A217)

Compound (3S,5S,8R,9R,10S,14R,17R,18S)-28-O-benzyl-3-aldehyde oleanolic acid was replaced by (3R,5S,8R,9R,10S,14R,17R,18S)-17-formyl oleanolic acid. Other required raw materials, reagents and preparation methods are the same as those in Example 2 to obtain A217(yield 55%). ¹H NMR (500 MHz, CDCl₃) δ 5.24 (tdd, *J* = 5.1, 1.8, 1.0 Hz, 1H), 4.95 (d, *J* = 5.2 Hz, 1H), 4.82 (d, *J* = 5.9 Hz, 1H), 4.71 (d, *J* = 5.7 Hz, 1H), 4.61 (d, *J* = 6.2 Hz, 1H), 4.20 (t, *J* = 4.5 Hz, 1H), 3.98 - 3.88 (m, 2H), 3.76 - 3.55 (m, 6H), 3.29 (ddd, *J* = 10.8, 7.4, 4.7 Hz, 1H), 3.00 (d, *J =* 10.8 Hz, 1H), 2.23 (tq, *J* = 4.6, 1.1 Hz, 1H), 1.95 - 1.81 (m, 2H), 1.80 - 1.74 (m, 1H), 1.77 - 1.72 (m, 1H), 1.74 - 1.67 (m, 2H), 1.70 - 1.61 (m, 1H), 1.63 - 1.55 (m, 1H), 1.58 - 1.52 (m, 2H), 1.55 - 1.49 (m, 2H), 1.50 (dd, *J* = 5.3, 1.6 Hz, 2H), 1.49 - 1.44 (m, 3H), 1.47 - 1.40 (m, 1H), 1.44 - 1.26 (m, 4H), 1.10 (s, 2H), 0.99 - 0.88 (m, 17H). LRMS (ESI): 605.4[M+H]⁺

### Example 218 (3R,5S,8R,9R,10S,14R,17R,18S)-Ursolic acid-17-(1'R)-(hydroxy)methyl-β-D-glucopyranoside (A218)

Compound (3S,5S,8R,9R,10S,14R,17R,18S)-28-O-benzyl-3-aldehyde oleanolic acid was replaced by (3R,5S,8R,9R,10S,14R,17R,18S)-17-formyl oleanolic acid. Other required raw materials, reagents and preparation methods are the same as those in Example 2 to obtain A218(yield 56%). ¹H NMR (500 MHz, CDCl₃) δ 5.47 (tdd, *J* = 4.9, 1.7, 1.0 Hz, 1H), 4.95 (d, *J* = 5.3 Hz, 1H), 4.81 (dd, *J =* 11.0, 5.7 Hz, 2H), 4.61 (d, *J* = 6.2 Hz, 1H), 4.20 (t, *J* = 4.5 Hz, 1H), 3.96 (ddd, *J* = 10.2, 5.1, 2.3 Hz, 1H), 3.94 - 3.87 (m, 1H), 3.76 - 3.50 (m, 7H), 3.29 (ddd, *J* = 10.8, 7.4, 4.7 Hz, 1H), 3.00 (d, *J* = 10.8 Hz, 1H), 2.06 - 1.24 (m, 25H), 1.09 (s, 2H), 0.97 (d, *J* = 1.5 Hz, 3H), 0.95 (d, *J* = 1.5 Hz, 3H), 0.93 (d, *J* = 1.5 Hz, 3H), 0.93 - 0.89 (m, 10H). LRMS (ESI): 605.4[M+H]⁺

### Example 219 (3R,5S,8R,9R,10S,14R,17R,18S)-Ursolic acid-28-β-D-glucopyranoside (A219)

Compound (3S,5S,8R,9R,10S,14R,17R,18S)-28-O-benzyl-3-aldehyde oleanolic acid was replaced by (3R,5S,8R,9R,10S,14R,17R,18S)-17-formyl ursolic acid. Other required raw materials, reagents and preparation methods are the same as those in Example 10 to obtain A219(yield 56%). ¹H NMR (500 MHz, CDCl₃) δ 5.53 - 5.46 (m, 2H), 5.07 (d, *J* = 5.7 Hz, 1H), 4.57 (d, *J =* 6.3 Hz, 1H), 4.51 (dd, *J =* 8.8, 2.6 Hz, 1H), 4.19 (t, *J* = 4.5 Hz, 1H), 3.71 - 3.58 (m, 3H), 3.58 (dt, *J* = 7.7, 3.5 Hz, 1H), 3.58 - 3.49 (m, 1H), 3.36 - 3.25 (m, 2H), 3.00 (d, *J =* 10.8 Hz, 1H), 2.28 - 2.22 (m, 1H), 2.01 - 1.93 (m, 1H), 1.89 - 1.24 (m, 21H), 1.20 (s, 2H), 0.99 - 0.93 (m, 4H), 0.96 - 0.90 (m, 8H), 0.91 (s, 2H), 0.91 (d, *J* = 5.7 Hz, 1H), 0.90 (d, *J* = 1.5 Hz, 2H). LRMS (ESI): 603.4[M+H]⁺

### Example 220 (5S,8R,9R,10S,14R,17R,18S)-3-Carbonyl oleanolic acid-28-6"-O-methyl-β-D-glucurouopyranoside

2,3,4,6-O-tetrabenzyl-1-(pyridin-1-yl)sulfonyl-β-D-thioglucopyranoside was replaced by 2,3,4-O-tribenzyl-1-(pyridin-1-yl)sulfonyl-6-methoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A220(yield 58%). ¹H NMR (500 MHz, CDCl₃) δ 5.42 (d, *J* = 5.8 Hz, 1H), 5.33 (tdd, *J* = 5.1, 1.8, 1.1 Hz, 1H), 4.89 (d, *J* = 6.4 Hz, 1H), 4.77 (d, *J* = 6.3 Hz, 1H), 4.50 (dd, *J* = 8.7, 2.7 Hz, 1H), 4.39 (d, *J* = 7.9 Hz, 1H), 3.89 - 3.80 (m, 1H), 3.73 (s, 2H), 3.71 - 3.57 (m, 2H), 2.48 (ddd, *J* = 12.4, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.7, 6.4, 5.1, 1.0 Hz, 1H), 1.95 - 1.87 (m, 1H), 1.91 - 1.83 (m, 1H), 1.83 (dtt, *J* = 7.1, 2.7, 1.5 Hz, 2H), 1.83 - 1.78 (m, 1H), 1.72 - 1.65 (m, 2H), 1.65 (d, *J* = 1.6 Hz, 1H), 1.66 - 1.62 (m, 1H), 1.65 - 1.61 (m, 1H), 1.64 - 1.52 (m, 3H), 1.55 - 1.48 (m, 1H), 1.51 - 1.29 (m, 5H), 1.15 (s, 2H), 1.08 (t, *J* = 1.6 Hz, 5H), 0.97 (d, *J* = 1.5 Hz, 3H), 0.95 - 0.90 (m, 8H). LRMS (ESI): 629.4[M+H]⁺.

### Example 221 (5S,8R,9R,10S,14R,17R,18S)-3-Carbonyl oleanolic acid-28-6"-O-ethyl-β-D-glucurouopyranoside (A221)

2,3,4,6-O-tetrabenzyl-1-(pyridin-1-yl)sulfonyl-β-D-thioglucopyranoside was replaced by 2,3,4-O-tribenzyl-1-(pyridin-1-yl)sulfonyl-6-ethoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A221(yield 53%). ¹H NMR (500 MHz, CDCl₃) δ 5.42 (d, *J* = 5.8 Hz, 1H), 5.33 (tdd, *J* = 5.1, 1.8, 1.1 Hz, 1H), 4.95 (d, *J* = 6.4 Hz, 1H), 4.77 (d, *J* = 6.3 Hz, 1H), 4.51 (dd, *J* = 8.7, 2.7 Hz, 1H), 4.45 (d, *J* = 8.2 Hz, 1H), 4.21 (qd, *J* = 6.3, 2.5 Hz, 2H), 3.85 (tdd, *J* = 8.3, 6.4, 1.9 Hz, 1H), 3.71 - 3.57 (m, 2H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.87 (m, 1H), 1.91 - 1.83 (m, 1H), 1.86 - 1.80 (m, 2H), 1.83 - 1.78 (m, 1H), 1.72 - 1.29 (m, 14H), 1.22 - 1.13 (m, 5H), 1.08 (t, *J* = 1.6 Hz, 5H), 0.99 - 0.92 (m, 6H), 0.93 (d, *J* = 6.6 Hz, 5H). LRMS (ESI): 643.4[M+H]⁺.

### Example 222 (5S,8R,9R,10S,14R,17R,18S)-3-Carbonyl oleanolic acid-28-6"-O-2"'-fluoroethyl-β-D-glucurouopyranoside (A222)

2,3,4,6-O-tetrabenzyl-1-(pyridin-1-yl)sulfonyl-β-D-thioglucopyranoside was replaced by 2,3,4-O-tribenzyl-1-(pyridin-1-yl)sulfonyl-6-2-fluoroethyl-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A222(yield 53%). ¹H NMR (500 MHz, CDCl₃) δ 6.11 (s, 1H), 6.02 (s, 1H), 5.42 (d, *J* = 5.8 Hz, 1H), 5.33 (tdd, *J* = 5.1, 1.8, 1.1 Hz, 1H), 4.95 (d, *J* = 6.3 Hz, 1H), 4.77 (d, *J* = 6.2 Hz, 1H), 4.51 (dd, *J* = 8.7, 2.7 Hz, 1H), 3.93 (d, *J* = 8.2 Hz, 1H), 3.85 (tdd, *J* = 8.3, 6.3, 1.9 Hz, 1H), 3.71 - 3.57 (m, 2H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.87 (m, 1H), 1.91 - 1.84 (m, 1H), 1.86 - 1.81 (m, 1H), 1.84 - 1.78 (m, 2H), 1.72 - 1.66 (m, 1H), 1.69 - 1.63 (m, 2H), 1.66 - 1.62 (m, 1H), 1.65 - 1.58 (m, 2H), 1.62 - 1.51 (m, 2H), 1.54 - 1.47 (m, 1H), 1.50 - 1.41 (m, 1H), 1.44 - 1.36 (m, 2H), 1.36 (ddd, *J* = 6.4, 3.4, 1.6 Hz, 1H), 1.36 - 1.29 (m, 1H), 1.15 (s, 2H), 1.08 (t, *J* = 1.6 Hz, 5H), 0.97 (d, *J* = 1.5 Hz, 3H), 0.95 - 0.90 (m, 8H). LRMS (ESI): 647.4[M+H]⁺.

### Example 223 (5S,8R,9R,10S,14R,17R,18S)-3-Carbonyl oleanolic acid-17-(1'R)-(hydroxy)methyl-6"-O-methyl-β-D-glucurouopyranoside (A223)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-methoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 2 to obtain A223(yield 58%). ¹H NMR (500 MHz, CDCl₃) δ 5.24 (tdd, *J* = 5.1, 1.8, 1.0 Hz, 1H), 4.99 (d, *J* = 5.3 Hz, 1H), 4.87 (d, *J* = 6.4 Hz, 1H), 4.75 (d, *J* = 5.7 Hz, 1H), 4.62 (d, *J* = 6.1 Hz, 1H), 4.09 (d, *J* = 7.5 Hz, 1H), 3.93 (ddd, *J* = 7.3, 5.7, 2.5 Hz, 1H), 3.84 (dddd, *J* = 8.3, 7.5, 6.4, 1.8 Hz, 1H), 3.77 - 3.69 (m, 1H), 3.73 (s, 3H), 3.68 - 3.60 (m, 1H), 3.56 (tdd, *J* = 8.3, 6.1, 2.6 Hz, 1H), 2.48 (ddd, *J* = 12.4, 6.5, 4.0 Hz, 1H), 2.40 (ddd, *J* = 12.5, 6.6, 4.0 Hz, 1H), 2.23 (tq, *J* = 4.6, 1.1 Hz, 1H), 1.99 (dddd, *J* = 12.6, 6.4, 5.2, 1.0 Hz, 1H), 1.95 - 1.86 (m, 1H), 1.89 - 1.78 (m, 3H), 1.78 - 1.59 (m, 4H), 1.59 - 1.26 (m, 11H), 1.11 - 1.05 (m, 8H), 0.99 - 0.92 (m, 8H), 0.90 (s, 2H). LRMS (ESI): 631.4[M+H]⁺.

### Example 224 (5S,8R,9R,10S,14R,17R,18S)-3-Carbonyl ursolic acid-28-6"-O-methyl-β-D-glucurouopyranoside (A224)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-methoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl ursolic acid. Other required raw materials, reagents and preparation methods are the same as those in Example 10 to obtain A224(yield 62%). ¹H NMR (500 MHz, CDCl₃) δ 5.50 (tdd, *J* = 4.9, 1.8, 0.9 Hz, 1H), 5.42 (d, *J* = 5.8 Hz, 1H), 4.89 (d, *J* = 6.4 Hz, 1H), 4.77 (d, *J* = 6.3 Hz, 1H), 4.55 (dd, *J* = 8.7, 2.6 Hz, 1H), 4.39 (d, *J* = 7.9 Hz, 1H), 3.89 - 3.80 (m, 1H), 3.73 (s, 2H), 3.71 - 3.57 (m, 2H), 2.48 (ddd, *J* = 12.5, 6.5, 4.0 Hz, 1H), 2.40 (ddd, *J* = 12.5, 6.6, 4.0 Hz, 1H), 2.28 - 2.22 (m, 1H), 2.06 - 1.91 (m, 2H), 1.89 - 1.78 (m, 4H), 1.77 (ddd, *J* = 12.5, 7.7, 5.2 Hz, 1H), 1.73 - 1.57 (m, 5H), 1.60 - 1.55 (m, 1H), 1.57 - 1.51 (m, 1H), 1.47 - 1.39 (m, 2H), 1.40 (td, *J* = 2.1, 1.1 Hz, 1H), 1.41 - 1.31 (m, 2H), 1.34 - 1.25 (m, 1H), 1.20 (s, 2H), 1.08 (t, *J* = 1.6 Hz, 5H), 0.99 - 0.90 (m, 9H), 0.91 (d, *J* = 2.0 Hz, 2H). LRMS (ESI): 629.4[M+H]⁺.

### Example 225 (3S,5S,8R,9R,10S,13R,14R,17S,18R,19R)-Betulinic acid-28-6"-O-methyl-β-D-glucurouopyranoside (A225)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-methoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl betulinic acid. Other required raw materials, reagents and preparation methods are the same as those in Example 10 to obtain A225(yield 50%). ¹H NMR (500 MHz, CDCl₃) δ 5.42 (d, *J* = 5.9 Hz, 1H), 4.89 (d, *J* = 6.4 Hz, 1H), 4.83 (h, *J* = 1.4 Hz, 1H), 4.77 (d, *J* = 6.3 Hz, 1H), 4.67 (h, *J* = 1.6 Hz, 1H), 4.51 (dd, *J* = 8.8, 2.6 Hz, 1H), 3.89 - 3.80 (m, 1H), 3.73 (s, 2H), 3.71 - 3.57 (m, 2H), 3.00 (dddt, *J* = 10.3, 5.0, 3.3, 1.6 Hz, 1H), 2.53 - 2.41 (m, 2H), 1.86 - 1.34 (m, 26H), 1.13 - 1.05 (m, 7H), 0.96 (dd, *J* = 12.7, 1.5 Hz, 6H), 0.85 (t, *J* = 1.4 Hz, 3H). LRMS (ESI): 629.4[M+H]⁺.

### Example 226 (5S,8R,9R,10S,14R,17R,18S)-3-Carbonyl betulinic acid-17-(1'R)-(hydroxy)methyl-6"-O-methyl-β-D-glucurouopyranoside (A226)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-methoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl betulinic acid. Other required raw materials, reagents and preparation methods are the same as those in Example 2 to obtain A226(yield 53%). 1H NMR (500 MHz, CDCl₃) δ 4.99 (d, J = 5.3 Hz, 1H), 4.87 (d, J = 6.4 Hz, 1H), 4.81 (h, J = 1.4 Hz, 1H), 4.69 (d, J = 5.3 Hz, 1H), 4.67 - 4.60 (m, 2H), 4.09 (d, J = 7.5 Hz, 1H), 3.91 - 3.80 (m, 2H), 3.75 - 3.66 (m, 3H), 3.66 - 3.52 (m, 2H), 2.53 - 2.41 (m, 2H), 2.13 (dddd, J = 10.4, 5.5, 3.5, 1.7 Hz, 1H), 1.83 (ddd, J = 5.7, 2.8, 1.4 Hz, 1H), 1.83 - 1.76 (m, 1H), 1.73 (q, J = 1.4 Hz, 3H), 1.63 - 1.28 (m, 20H), 1.13 - 1.05 (m, 7H), 0.97 (d, J = 1.5 Hz, 3H), 0.92 (d, J = 1.5 Hz, 3H), 0.85 (t, J = 1.4 Hz, 3H). LRMS (ESI): 631.4[M+H]⁺.

### Example 227 (5S,8R,9R,10S,14R,17R,18S)-3-Carbonyl betulinic acid-17-(1'R)-(hydroxy)methyl-6"-O-methyl-β-D-glucurouopyranoside (A227)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-isopropoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A227(yield 53%). ¹H NMR (500 MHz, CDCl₃) δ 5.42 (d, *J* = 5.9 Hz, 1H), 5.33 (tdd, *J* = 5.1, 1.8, 1.1 Hz, 1H), 5.03 (hept, *J* = 5.8 Hz, 1H), 4.95 (d, *J* = 6.4 Hz, 1H), 4.77 (d, *J =* 6.3 Hz, 1H), 4.51 (dd, *J* = 8.7, 2.6 Hz, 1H), 4.07 (d, *J* = 7.9 Hz, 1H), 3.86 (tdd, *J* = 8.3, 6.4, 1.8 Hz, 1H), 3.72 - 3.57 (m, 2H), 2.48 (ddd, *J* = 12.4, 6.5, 4.0 Hz, 1H), 2.40 (ddd, *J* = 12.5, 6.6, 4.0 Hz, 1H), 2.17 (tq, *J =* 5.1, 1.0 Hz, 1H), 2.04 (dddd, *J* = 12.7, 6.4, 5.1, 1.0 Hz, 1H), 1.95 - 1.87 (m, 1H), 1.91 - 1.83 (m, 1H), 1.83 (dddd, *J* = 7.0, 2.7, 1.9, 1.1 Hz, 2H), 1.83 - 1.78 (m, 1H), 1.72 - 1.29 (m, 14H), 1.25 (d, *J* = 5.7 Hz, 3H), 1.20 (d, *J* = 5.9 Hz, 3H), 1.15 (s, 2H), 1.08 (t, *J* = 1.6 Hz, 5H), 0.97 (d, *J* = 1.5 Hz, 3H), 0.95 - 0.90 (m, 8H). LRMS (ESI): 631.4[M+H]⁺.

### Example 228 (5S,8R,9R,10S,14R,17R,18S)-3-Carbonyl oleanolic acid-28-6"-O-cyclopropylmethyl-β-D-glucurouopyranoside (A228)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-cyclopropylmethoxy-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A228(yield54%). ¹H NMR (500 MHz, CDCl₃) δ 5.42 (d, *J* = 5.8 Hz, 1H), 5.33 (tdd, *J* = 5.1, 1.8, 1.1 Hz, 1H), 4.95 (d, *J =* 6.4 Hz, 1H), 4.77 (d, *J* = 6.3 Hz, 1H), 4.51 (dd, *J* = 8.7, 2.7 Hz, 1H), 4.12 - 4.05 (m, 3H), 3.85 (tdd, *J* = 8.2, 6.3, 1.8 Hz, 1H), 3.71 - 3.57 (m, 2H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.83 (m, 2H), 1.86 - 1.78 (m, 3H), 1.72 - 1.66 (m, 1H), 1.69 - 1.63 (m, 2H), 1.66 - 1.62 (m, 1H), 1.65 - 1.47 (m, 6H), 1.50 - 1.41 (m, 1H), 1.44 - 1.36 (m, 2H), 1.36 (ddd, *J* = 6.4, 3.4, 1.6 Hz, 1H), 1.36 - 1.29 (m, 1H), 1.15 (s, 2H), 1.08 (t, *J* = 1.6 Hz, 5H), 0.97 (d, *J* = 1.5 Hz, 3H), 0.95 - 0.90 (m, 8H), 0.58 - 0.44 (m, 4H). LRMS (ESI): 669.4[M+H]⁺.

### Example 229 (5S,8R,9R,10S,14R,17R,18S)-3-Carbonyl oleanolic acid-28-6"-N-ethyl-β-D-glucurouopyranoside (A229)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-ethylamino-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A229(yield58%). ¹H NMR (500 MHz, CDCl₃) δ 7.28 (t, *J* = 3.9 Hz, 1H), 5.46 - 5.39 (m, 1H), 5.33 (tdd, *J* = 5.1, 1.8, 1.1 Hz, 1H), 4.89 (d, *J* = 6.1 Hz, 1H), 4.78 - 4.71 (m, 1H), 4.50 - 4.43 (m, 1H), 4.09 (d, *J* = 7.5 Hz, 1H), 3.81 - 3.72 (m, 1H), 3.68 - 3.57 (m, 2H), 3.23 (qd, *J* = 6.2, 3.9 Hz, 2H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.84 (m, 2H), 1.86 - 1.81 (m, 1H), 1.84 - 1.78 (m, 2H), 1.72 - 1.29 (m, 14H), 1.21 - 1.13 (m, 5H), 1.08 (t, *J* = 1.6 Hz, 6H), 0.99 - 0.92 (m, 6H), 0.93 (d, *J* = 6.6 Hz, 5H). LRMS (ESI): 642.4[M+H]⁺.

### Example 230 (5S,8R,9R,10S,14R,17R,18S)-3-Carbonyl oleanolic acid-28-6"-N-2"'-fluoroethyl-β-D-glucurouopyranoside (A230)

2,3,4,6-O-Tetrabenzyl-1-(pyridin-1-yl)sulfonyl-β**-**D-thioglucopyranoside was replaced by 2,3,4-O-tribenzyl-1-(pyridin-1-yl)sulfonyl-6-2-fluoroethylamino-β-D-thioglucurouopyranoside. Compound (3S,5S,8R,9R,10S,14R,17R,18S)-28-O-benzyl-3-aldehyde oleanolic acid was replaced by (5S,8R,9R,10S,14R,17R,18S)-3-carbonyl-17-formyl oleanolic acid. Other required raw materials, reagents and preparation methods are the same as those in Example 10 to obtain A230(yield51%). ¹H NMR (500 MHz, CDCl₃) δ 7.80 (t, *J* = 4.9 Hz, 1H), 5.46 - 5.39 (m, 1H), 5.33 (tdd, *J* = 5.1, 1.8, 1.1 Hz, 1H), 4.89 (d, *J* = 6.1 Hz, 1H), 4.78 - 4.71 (m, 1H), 4.58 (td, *J* = 3.4, 2.3 Hz, 1H), 4.53 - 4.43 (m, 2H), 4.05 (d, *J* = 7.4 Hz, 1H), 3.81 - 3.72 (m, 1H), 3.68 - 3.57 (m, 2H), 3.44 (dtd, *J* = 4.6, 3.4, 1.2 Hz, 1H), 3.39 (dtd, *J* = 4.8, 3.4, 1.1 Hz, 1H), 2.48 (ddd, *J* = 12.5, 6.5, 4.0 Hz, 1H), 2.40 (ddd, *J* = 12.5, 6.6, 4.0 Hz, 1H), 2.17 (tq, *J* = 5.1, 1.0 Hz, 1H), 2.04 (dddd, *J* = 12.6, 6.4, 5.1, 1.0 Hz, 1H), 1.95 - 1.87 (m, 1H), 1.91 - 1.84 (m, 1H), 1.86 - 1.81 (m, 1H), 1.84 - 1.78 (m, 2H), 1.72 - 1.29 (m, 14H), 1.15 (s, 2H), 1.08 (t, *J* = 1.6 Hz, 6H), 0.99 - 0.92 (m, 6H), 0.93 (d, *J* = 6.6 Hz, 5H). LRMS (ESI): 650.4[M+H]⁺.

### Examples of pharmacological activity assays

### Example 1 Determination of in vivo hypoglycemic pharmacodynamic activity of the compounds of the invention

### 1. Pharmacodynamic experiment of compound on oral glucose tolerance in ICR mice

### i. Experimental materials

**Table 6: Use of Animals**

| | |
|---|---|
| **Strain** | ICR mice |
| **Grade** | SPF |
| **Animal weight** | 20∼25g |
| **Gender** | Male |
| **Supplier** | Shanghai Slake |
| **Methods of animal identification** | Animal rearing in single cage, cage label identification |
| **Animal number** | 114 |

After the animals arrived at WuXi AppTec facilities, they were kept in the animal feeding room with strictly controlled environmental conditions. The temperature was kept at 20-24 °C and the humidity was kept at 30-70% in the feeding room. The temperature and humidity of the feeding room were monitored in real time through the temperature and humidity meter, and the temperature and humidity were recorded twice a day (once in the morning and once in the afternoon). The lighting in the animal feeding room was controlled by an electronic timing lighting system, which was turned on for 12 hours every day and turned off for 12 hours (on at 7:00 a.m. and off at 19:00 p.m.). During the experiment, the animals were raised in a single cage and toys were provided for mice in each cage. During the experiment, the animals freely took food (growth/reproduction feed for rats and mice) and water.

After the animals arrived, they can adapt to the environment for 1-2 weeks before conducting the experiment.

### ii. Drug preparation and administration

**Table 3: Reference solvent information**

| | |
|---|---|
| **Name:** | **0.5% MC solution** |
| **Supplier:** | prepared when to be used |
| **Physical characteristics:** | clear solution |
| **Storage condition:** | 4 °C |

1) Preparation of liquid medicine: each compound was prepared on the first day of the experiment, and then stored in a 4 °C refrigerator for use on the next day.
2) Administration: After grouping, animals in each group were administered 0.5 hours before sugar administration.
3) Oral glucose tolerance experiment: sugar was administrated to the animals by gavage 0.5 hour after the end of the animal administration, and the time of sugar administration was recorded as 0 min. The oral dose of glucose was 5 g/kg, 10 ml/kg. The blood glucose of animals was detected before administration, before sugar administration, and 15, 30, 60, 90, and 120 minutes after sugar administration. The blood glucose was detected with a blood glucose meter and the corresponding blood glucose test paper.

### iii. Experimental results

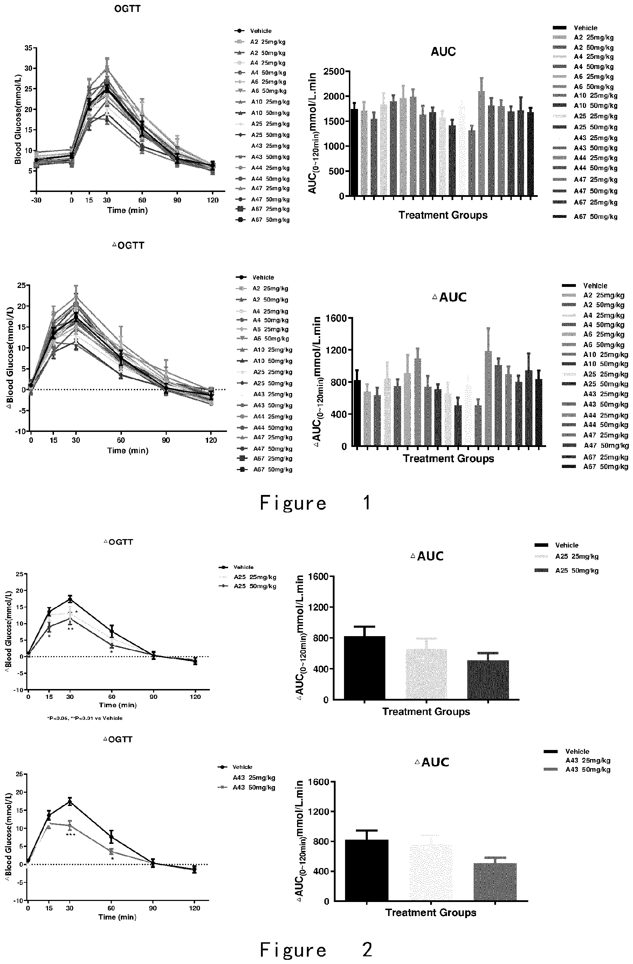

The results showed that oral administration of pentacyclic triterpenoid carbon glycosides could effectively reduce the postprandial blood glucose level of ICR mice (Figure 1), among which A25 and A43 have the best hypoglycemic activity, with obvious hypoglycemic effect at 25 mg/kg and further enhanced hypoglycemic effect at 50 mg/kg (Figure 2).

### Example 2 Determination of in vitro GLP-1 secretion activity of the compounds of the invention

### 1. Experimental materials

Cell line: intestinal endocrine cell line STC-1 cell
Negative control: DMSO
Positive control: INT777 (TGR5 receptor agonist)
Test tool: HTRF kit
Others: KRBH buffer, BSA, DPP4 inhibitor

### 2. Experimental method

STC-1 cells were seeded with a certain density. After the cells adhered to the wall overnight, they were starved with KRBH buffer, while BSA was used to maintain nutrition, and degradation of GLP-1 was reduced with DPP4 inhibitors. After 1h, they were replaced with glucose free or glucose containing KRBH buffer to stimulate GLP-1 secretion. At the same time, compounds of certain concentrations were added. After 1h of treatment, supernatant was collected and tested for GLP-1 concentration with HTRF kit. The secretion of LDH was also detected to indicate the toxicity of the compounds.

### 3. Experimental results

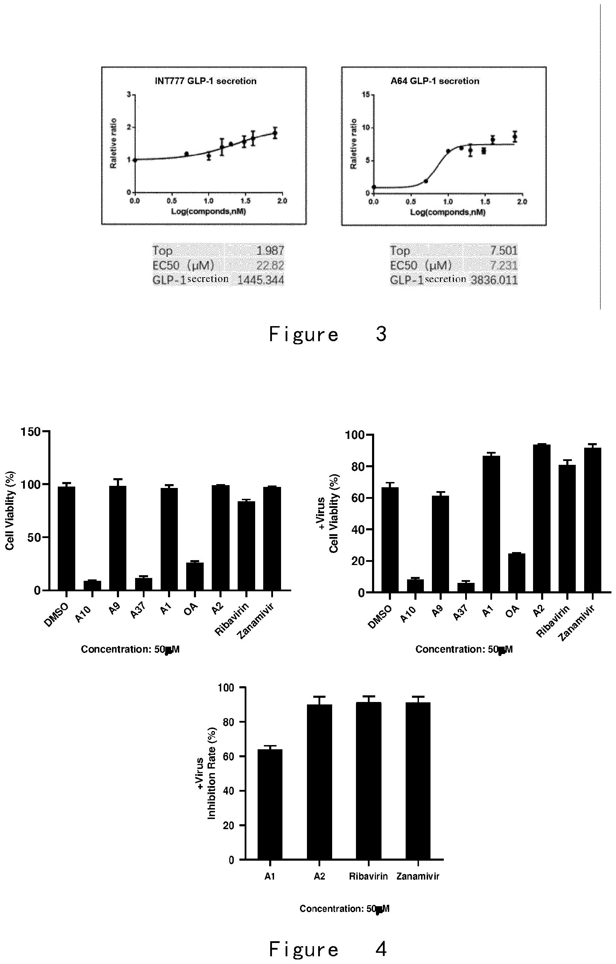

The experimental results showed that A64 had a good *in vitro* effect of promoting GLP-1 secretion, and its *in vitro* EC₅₀ was 7.231 µM, which is obviously superior to the positive compound INT777 (EC₅₀=22.82 µM) (Figure 3).

### Example 3. Determination of anti influenza virus pharmacodynamic activity in vitro of the compound of the invention

### 1. Experimental materials

Cell lines: MDCK cell
Negative control: DMSO
Positive control: Zanamivir/Oseltamivir(50 µ M)
CellTiter-Glo^{®} Luminescent Cell Viability Assay
Preparation of the compound mother liquor: diluting compounds 1-7 to 10 mM/L; Screening concentration: 50 µM

### 2. Experimental method

MDCK cells were laid with 96 well plates (1×10⁴/well) and incubated in 37°C and 5% CO₂ incubators for 24 h, then the medicine was added.

After dosing, the 96 well plate was put in the 37°C, 5% CO₂ incubator for 36h, and detected with the microplate reader.

### 3. Experimental results

Formula of inhibition rate: I(inhibition rate)=[1-(Xi-Xj)/(Yi-Yj)]×100%
Xi: number of surviving cells without virus in the test group; Xj: number of surviving cells with virus in the test group
Yi: number of surviving cells without virus in DMSO group; Yj: number of surviving cells with virus in DMSO group

The results showed that pentacyclic triterpenoid carbon glycosides can produce effective pharmacological activity against influenza virus, in which A2 has the same virus inhibition rate and low cytotoxicity as the positive drug zanamivir, similar virus inhibition rate to that of the positive drug ribavirin, but lower cytotoxicity than that of ribavirin (Figure 4).

### Example 4. Determination of affinity between the compound of the invention with coronavirus N protein in vitro

### 1. Experimental materials

N protein diluent: 20 µg/mL
Buffer solution: 10 mM HEPES, pH 7.4, 150 mM NaCl, 3.0 mM EDTA, and 0.005% (v/v) surfactant P20, 5% DMSO.
Instrument: Biacore 8K (GE Healthcare)

### 2. Test method

Biacore 8K (GE Healthcare) was used to test the affinity between the compound with N protein by surface plasmon resonance (SPR). Firstly, the N proteins were diluted to 20 µg/mL, standard amino coupling method was used to couple the full length N proteins to CM5 chip respectively at 25 °C, with the signal value of 10,000 signal units (RU). The running buffer used was 10 mM HEPES, pH 7.4, 150 mM NaCl, 3.0 mM EDTA, and 0.005% (v/v) surfactant P20, 5% DMSO. 10 mM of DMSO stock solution of the compound was diluted to 7 gradient concentrations (1.56 µM-100 µM) with running buffer. During the test, the instrument was initialized for three times using the running buffer, then the test compounds were injected and flown through the chip in turn, and the binding and dissociation time were set as 100 seconds. After one cycle, 50% DMSO was used to wash the remaining compounds on the chip and continued with the next cycle. Finally, solvent correction was carried out with running buffer solution containing 4.5%-5.8%. According to the generated spectrum, the value of the control group was deducted and solvent correction was conducted. The data was generated using the Biacore 8K data processing software and KD values were calculated by the static affinity model according to the fixed Rmax value.

### 3. Experiment conclusion

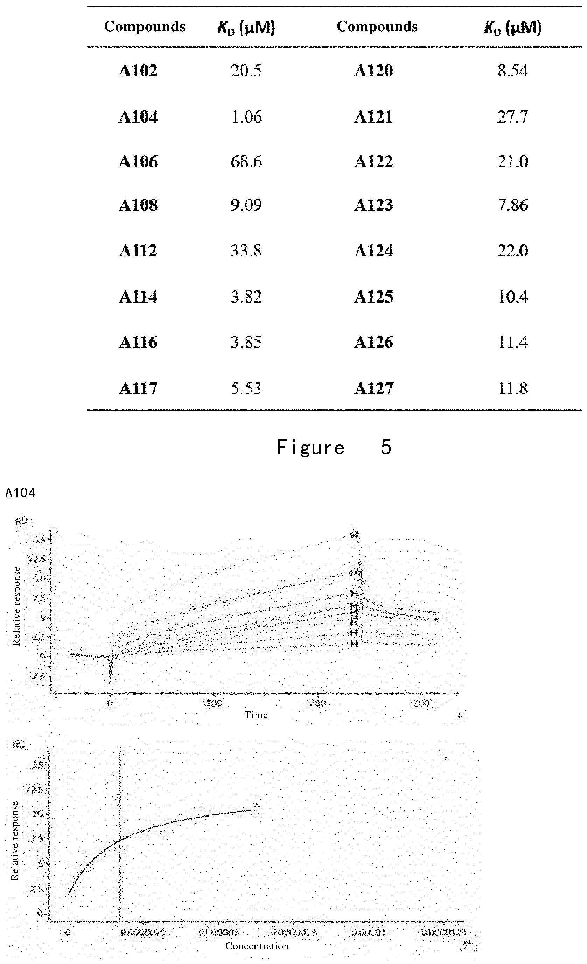

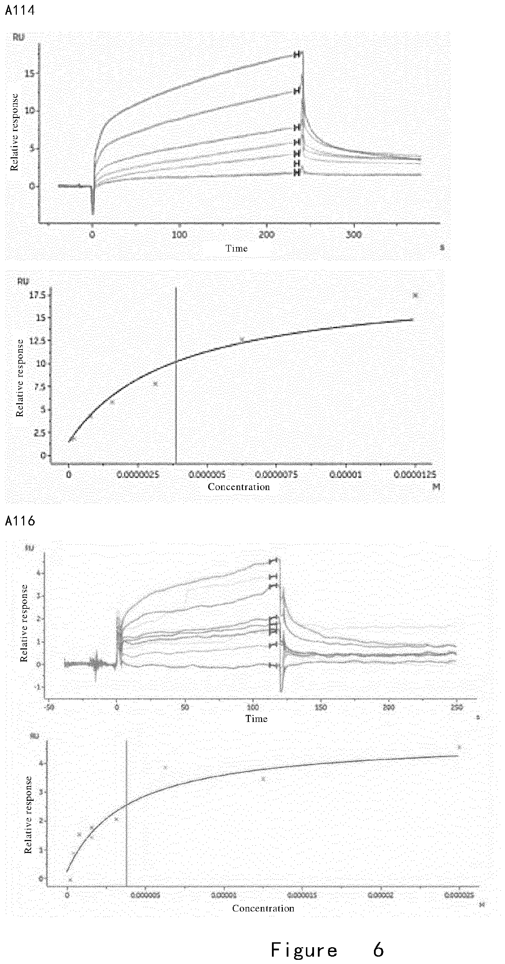

The results show that pentacyclic triterpenoid carbon glycosides have enhanced affinity with N protein, and Ki of some compounds were less than 10 µM. (Figures 5 and 6)

### Example 5: Determination of the in vitro pharmacodynamic activity of the compound of the invention against SARS Cov-2 virus

### 1. Experimental materials

Vero E6 cells (ATCC-1586)
48well plate (50000 cells/ well); 96 well plate (20000 cells/well)
2019-nCoV (nCoV-2019BetaCoV/Wuhan/WIV04/2019)

### 2. Experimental method

Vero E6 cells (ATCC-1586) were laid in a 48 well plate (50000 cells/well), and 100 µL/well of culture medium containing gradient concentration compounds was added, then 2019-nCoV (nCoV-2019BetaCoV/Wuhan/WIV04/2019) was added, whose multiple infection index (MOI) is 0.05. After the mixture was incubated for 1 hour, the supernatant was suck out, the cells were washed and 200 µL/well of medium containing gradient concentration compound was added again, and the mixture was incubated at 37 °C for 24 hours. After 24 hours, the cell supernatant was collected, the viral RNA in the supernatant was extracted, and the viral copy number in the supernatant was detected by real-time fluorescent quantitative PCR. The inhibition rate of the compound was calculated according to the viral copy number, and EC₅₀ of the compound was calculated by using prism 6.0.

Vero E6 cells (ATCC-1586) were laid in a 96 well plate (20000 cells/well), and 100 µL/well of culture medium containing gradient concentration compounds was added. After 24 hours, CCK8 detection kit was used to detect the effect of compounds on cell activity, and CC₅₀ of the compound was calculated by using prism 6.0.

### 3. Experiment conclusion

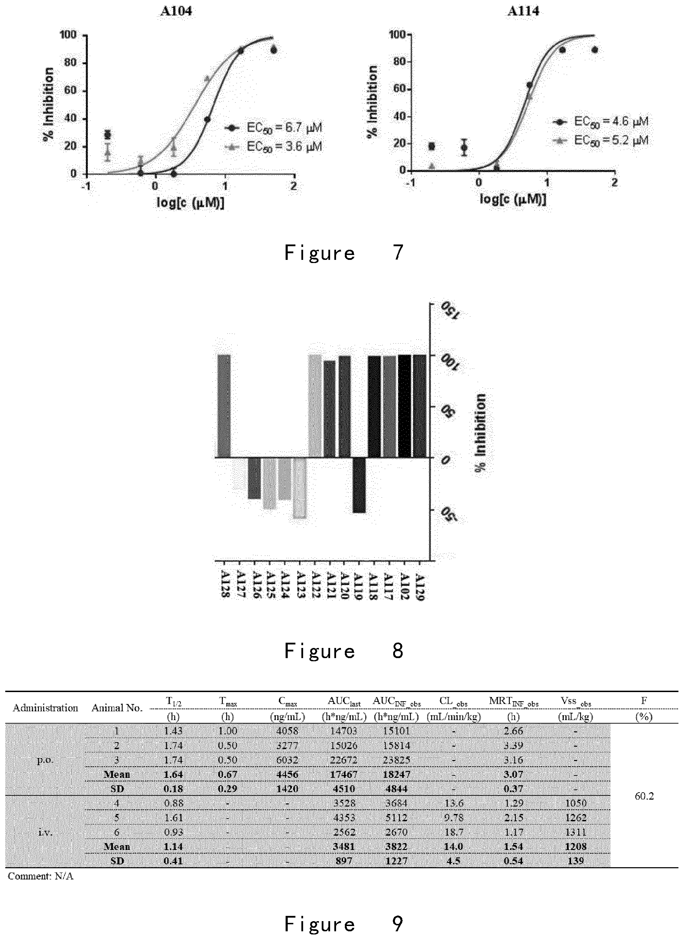

The results show that pentacyclic triterpenoid carbon glycosides could effectively inhibit the replication of SARS Cov-2 virus, among which the EC₅₀ of A104 and A114 was 3.6 µM and 4.6 µM and inhibition rate of some compounds at 10 µM level was 100% (Figures 7 and 8).

### Example 6: Determination of PK property of the compound of the invention in mice

### 1. Experimental materials

### Animal condition:

Species/strain: ICR (CD-1) mice
Gender/number of rats: male (n=6)
Weight (g): male (20-24 g)
Type of diet: standard rodent diet
   freely drink
   Fasted for 12 hours before administration and keeping fasting for 2 hours.
Feeding: animal room environment control (target conditions: temperature 18 to 29 °C, relative humidity 30 to 70%. Temperature and relative humidity were monitored every day. The electronic time control lighting system was used to provide 12 hours of light/12 hours of dark cycle.

### 2. Test method

Dosage for adminstration was shown in the table below:

| Administration route | Dose (mg/kg) | Dosing volume (mL/kg) | Carrier |
|---|---|---|---|
| P0 | 25 | 10 | DMS0/0. 5% HPMC (5/95, v/v/) |
| IV | 3 | 5 | DMSO/EtOH/PEG300/0.9%NaCl (5/5/40/50, v/v/v/v) |

### 3. Experiment conclusion

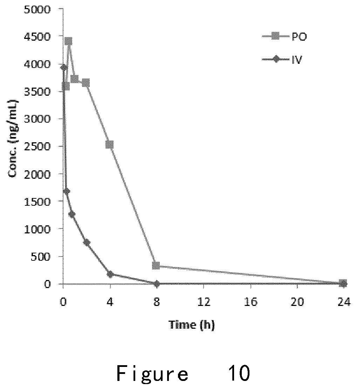

The results show that the pentacyclic triterpenoid carbon glycosides of the invention have good pharmacokinetic properties, and the specific conclusions are shown in Figure 9 and Figure 10. Figure 9 shows that the compound of the invention can reach the required drug exposure within the experimental time after oral and intestinal administration. Figure 10 shows that the compound of the invention was rapidly released within 8 hours after administration and completely released within 24 hours.

All documents mentioned herein are incorporated by reference in the present invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A pentacyclic triterpenoid carbon glycoside derivative compound with the structure shown in the following general formula I, or racemates, R-isomers, S-isomers, pharmaceutically acceptable salts thereof, or the mixtures thereof: 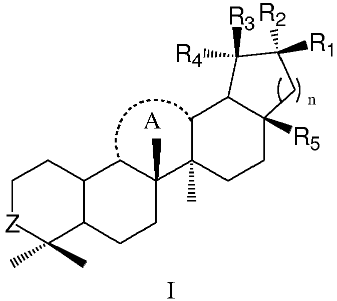 wherein,
ring A is selected from the group consisting of 6-membered saturated carbon ring or unsaturated carbon ring;
R₁, R₂ and R₃ are each independently selected from the group consisting of hydrogen, and methyl; R₄ is selected from the group consisting of hydrogen, and isopropenyl;
R₅ is selected from the group consisting of hydrogen, deuterium, tritium, halogen, cyano, amino, nitro, hydroxyl, sulfydryl, aldehyde group, carboxyl, sulfonyl, phosphate group, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, substituted or unsubstituted C₁-C₆ amido, and Y-R₇; wherein,
Y is selected from the group consisting of -(CH₂)ₘCHR₉-, carbonyl, -CONH-, and -COO-;
wherein m is 0 or 1;
R₇ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₂-C₁₀ ester group, substituted or unsubstituted C₅-C₉ furanosyl, and substituted or unsubstituted C₅-C₉ pyranosyl;
Z is selected from the group consisting of carbonyl, -CH(R₉)₂, C=N-R₈, C=N-NH-R₈, and -X-R₆;
R₈ is selected from the group consisting of hydrogen, deuterium, tritium, halogen, cyano, amino, nitro, hydroxyl, sulfydryl, aldehyde group, carboxyl, sulfonyl, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, and substituted or unsubstituted C₁-C₆ amido;
R₉ is selected from the group consisting of hydrogen, deuterium, tritium, halogen, cyano, amino, nitro, hydroxyl, and sulfydryl;
R₆ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
X is selected from the group consisting of -CHR₉-, carbonyl, S, -NHC(O)-Rs, -NHS(O)₂-R₈, - NHC(O)NH-Rs, - NHC(S)NH-R₈, -COO-, and -O-S(O)₂-R₈;
n is 1 or 2;
unless otherwise specified, the "substituted" in the above formulas means that the hydrogen atom on the corresponding group, or the hydroxyl group on the glycosyl ring is substituted by one or more substituents which are selected from the group consisting of deuterium, tritium, halogen, hydroxyl, carboxyl, sulfydryl, benzyl, C₁-C₁₂ alkoxycarbonyl, C₁-C₆ aldehyde group, amino, C₁-C₆ amide, nitro, cyano, unsubstituted or halogenated C₁-C₆ alkyl, C₂-C₁₀ alkenyl, C₁-C₆ alkoxy, C₁-C₆ alkylamino, C₆-C₁₀ aryl, five or six membered heteroaryl, five or six membered non-aromatic heterocyclyl, -O-(C₆-C₁₀ aryl), -O-(five or six membered heteroaryl), C₁-C₁₂ alkaminocarbonyl, substituted or unsubstituted C₂-C₁₀ acyl, sulfonyl (-SO₂-OH), phosphoryl (-PO₃-OH), C₅-C₉ furanosyl, and C₅-C₉ pyranosyl.

2. The compound or racemates, R-isomers, S-isomers, medicinal salts thereof, or the mixtures thereof according to claim 1, wherein the compound of formula I has the structure shown in the following general formula II: 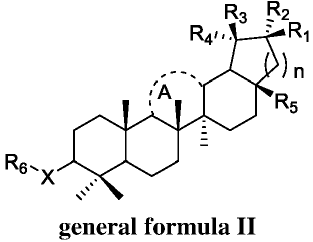 wherein,
R₅ is selected from the group consisting of hydrogen, deuterium, tritium, halogen, cyano, amino, nitro, hydroxyl, sulfydryl, aldehyde group, carboxyl, sulfonyl, phosphate group, substituted or unsubstituted C₁-C₆ alkyl, substituted or unsubstituted C₁-C₆ alkoxy, substituted or unsubstituted C₆-C₁₀ aryl, substituted or unsubstituted 5-7 membered heterocycle containing 1-3 heteroatoms selected from oxygen, sulfur and nitrogen, substituted or unsubstituted C₁-C₆ alkyl-phenyl, substituted or unsubstituted C₃-C₁₂ cycloalkyl, substituted or unsubstituted C₂-C₁₀ acyl, substituted or unsubstituted C₂-C₁₀ ester group, substituted or unsubstituted C₆-C₁₀ aryloxy, and substituted or unsubstituted C₁-C₆ amido;
X is selected from the group consisting of -CHR₉-, and carbonyl;
R₆ is selected from the group consisting of substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
R₉ is selected from the group consisting of hydrogen, deuterium, tritium, halogen, cyano, amino, nitro, hydroxyl, and sulfydryl;
ring A is selected from the group consisting of 6-membered saturated carbon ring or unsaturated carbon ring;
n is 1 or 2.

3. The compound or racemates, R-isomers, S-isomers, medicinal salts thereof, or the mixtures thereof according to claim 1, wherein the compound of formula I has the structure shown in the following general formula III: 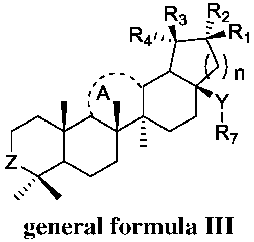 wherein,
R₁, R₂ and R₃ are each independently selected from the group consisting of hydrogen, and methyl;
R₄ is selected from the group consisting of hydrogen, and isopropenyl;
R₇ is selected from the group consisting of hydrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
Y is selected from the group consisting of -(CH₂)ₘCHR₉-, and carbonyl;
m is 0 or 1;
R₉ is selected from the group consisting of hydrogen, deuterium, tritium, halogen, cyano, amino, nitro, hydroxyl, and sulfydryl;
Z is selected from the group consisting of carbonyl, -CH(R₉)₂, =N-Rs, and -X-R₆;
R₉ is selected from the group consisting of hydrogen, halogen, cyano, amino, nitro, hydroxyl, and sulfydryl;
X is selected from the group consisting of -CHR₉-, carbonyl, S, -NHC(O)-Rs, -NHS(O)₂-R₈, - NHC(O)NH-Rs, -NHC(S)NH-R₈, -COO-, and -O-S(O)₂-R₈;
R₆ is selected from the group consisting of hydrogen, substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl;
ring A is selected from the group consisting of 6-membered saturated carbon ring or unsaturated carbon ring;
n is 1 or 2.

4. The compound or racemates, R-isomers, S-isomers, medicinal salts thereof, or the mixtures thereof according to claim 1, wherein the compound of formula I has the structure shown in the following general formulas IV, V, and VI:

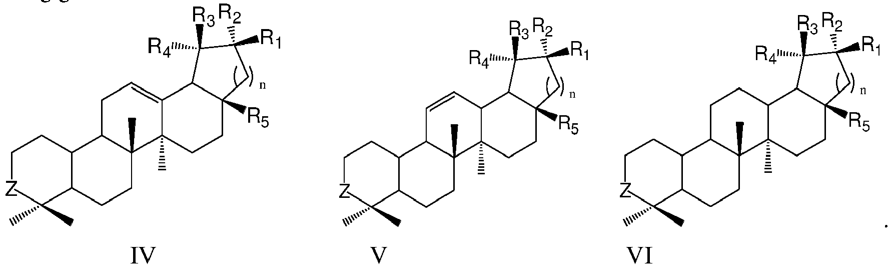

5. The compound or racemates, R-isomers, S-isomers, medicinal salts thereof, or the mixtures thereof according to claim 2, wherein R₅ is selected from the group consisting of hydrogen, halogen, hydroxyl, carboxyl, substituted or unsubstituted C₁-C₆ alkyl, substituted or unsubstituted C₁-C₆ alkoxy, substituted or unsubstituted C₂-C₁₀ acyl, substituted or unsubstituted C₂-C₁₀ ester group, and substituted or unsubstituted C₁-C₆ amido.

6. The compound or racemates, R-isomers, S-isomers, medicinal salts thereof, or the mixtures thereof according to claim 3, wherein R₉ is selected from the group consisting of hydrogen, hydroxyl, aldehyde group, sulfydryl, and -X-R₆;
X is selected from the group consisting of -CHR₉-, carbonyl, S, -NHC(O)-Rs, -NHS(O)₂-R₈, - NHC(O)NH-Rs, -NHC(S)NH-R₈, -COO-, and -O-S(O)₂-R₈;
R₆ is selected from the group consisting of substituted or unsubstituted C₁-C₆ alkyl, substituted or unsubstituted C₂-C₆ alkenyl, substituted or unsubstituted 5-7 membered heterocycle containing 1-3 heteroatoms selected from oxygen, sulfur and nitrogen, substituted or unsubstituted C₅-C₉ furanosyl, and substituted or unsubstituted C₅-C₉ pyranosyl; the "substituted" means that one or more hydrogens or hydroxyls on the glycosyl ring are substituted by substituents which are selected from the group consisting of hydrogen, deuterium, tritium, halogen, cyano, amino, nitro, sulfydryl, aldehyde group, carboxyl, benzyl, substituted or unsubstituted C₁-C₁₂ alkoxycarbonyl, substituted or unsubstituted C₂-C₁₂ alkaminocarbonyl, substituted or unsubstituted C₂-C₁₀ acyl, sulfonyl, phosphoryl, C₅-C₉ furanosyl, and C₅-C₉ pyranosyl.

7. The compound or racemates, R-isomers, S-isomers, medicinal salts thereof, or the mixtures thereof according to claim 1, wherein the pentacyclic triterpenoid carbon glycoside compounds are selected from the group consisting of:
| Number | Structure | Name |
|---|---|---|
| A1 | 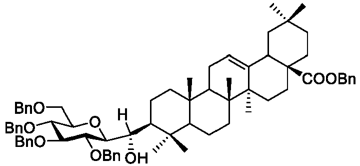 | (3S,5S,8R,9R,10S,14R,17R,18 S)-28-O-Benzyloleanolicacid-3-(1'R)-(hydroxyl)methyl-2",3",4",6"-O-tetrabenzyl-β-D-glucopyranoside |
| A2 | 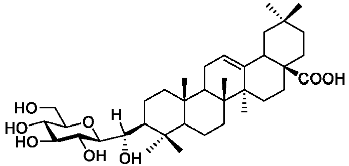 | (3S,5S,8R,9R,10S,14R,17R,18 S)-Oleanolicacid-3-(1'R)-(hydroxyl)methyl-β-D-glucopyranoside |
| A3 | 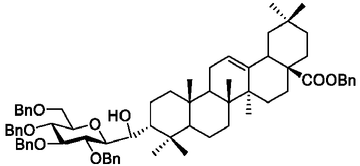 | (3R,5S,8R,9R,10S,14R,17R,18 S)-28-O-Benzyloleanolicacid-3-(1'R)-(hydroxyl)methyl-2",3",4",6"-O-tetrabenzyl-β-D-glucopyranoside |
| A4 | 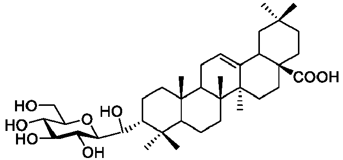 | (3R,5S,8R,9R, 10S, 14R, 17R, 18 S)-Oleanolicacid-3-(1'R)-(hydroxyl)methyl-β-D-glucopyranoside |
| A5 | 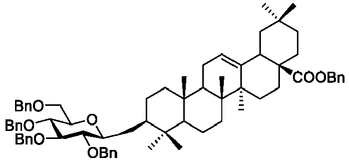 | (3S,5S,8R,9R,10S,14R,17R,18 S)-28-O-Benzyloleanolicacid-3-methyl-2",3",4",6"-O-tetrabenzyl-β-D-glucopyranoside |
| A6 | 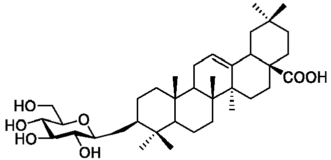 | (3S,5S,8R,9R,10S,14R,17R,18 S)-Oleanolicacid-methyl-β-D-glucopyranoside |
| A7 | 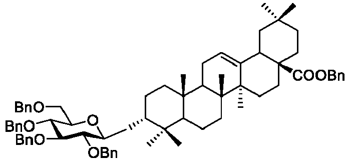 | (3R,5S,8R,9R,10S, 14R, 17R, 18 S)-28-O-Benzyloleanolicacid-3-methyl-2",3",4",6"-O-tetrabenzyl-β-D-glucopyranoside |
| A8 | 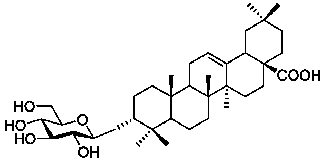 | (3R,5S,8R,9R,10S,14R,17R,18 S)-Oleanolicacid-3-methyl-β-D-glucopyranoside |
| A9 | 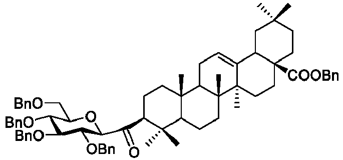 | (3S,5S,8R,9R,10S,14R,17R,18 S)-28-O-Benzyloleanolicacid-3-carbonyl-2",3",4",6"-O-tetrabenzyl-β-D-glucopyranoside |
| A10 | 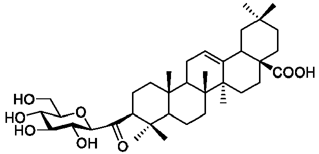 | (3S,5S,8R,9R,10S,14R,17R,18 S)-Oleanolicacid-3-carbonyl-β-D-glucopyranoside |
| A11 | 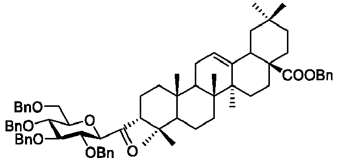 | (3R,5S,8R,9R,10S,14R,17R,18 S)-28-O-Benzyloleanolicacid-3-carbonyl-2",3",4",6"-O-tetrabenzyl-β-D-glucopyranoside |
| A12 | 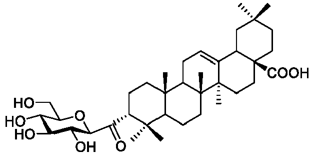 | (3R,5S,8R,9R,10S,14R,17R,18 S)-Oleanolicacid-3-carbonyl-β-D-glucopyranoside |
| A13 | 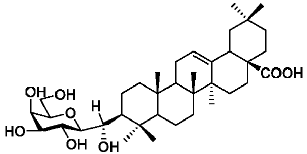 | (3S,5S,8R,9R,10S,14R,17R,18 S)-Oleanolicacid-3-(1'R)-(hydroxyl)methyl-β-D-galactopyranoside |
| A14 | 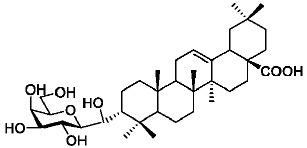 | (3R,5S,8R,9R,10S,14R,17R,18 S)-Oleanolicacid-3-(1'R)-(hydroxyl)methyl-β-D-galactopyranoside |
| A15 | 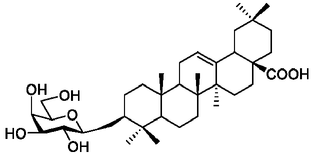 | (3S,5S,8R,9R,10S,14R,17R,18 S)-Oleanolicacid-3-methyl-β-D-galactopyranoside |
| A16 | 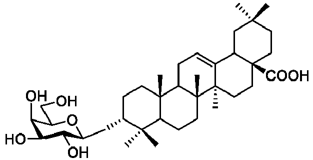 | (3R,5S,8R,9R,10S,14R,17R,18 S)-Oleanolicacid-3-methyl-β-D-galactopyranoside |
| A17 | 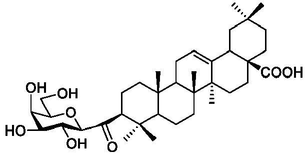 | (3S,5S,8R,9R,10S,14R,17R,18 S)-Oleanolicacid-3-carbonyl-β-D-galactopyranoside |
| A18 | 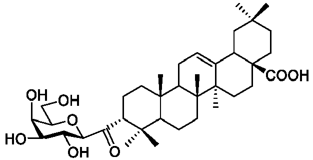 | (3R,5S,8R,9R, 10S, 14R, 17R, 18 S)-Oleanolicacid-3-carbonyl-β-D-galactopyranoside |
| A19 | 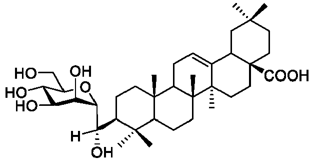 | (3S,5S,8R,9R,10S,14R,17R,18 S)-Oleanolicacid-3-(1'S)-(hydroxyl)methyl-α-D-mannopyranoside |
| A20 | 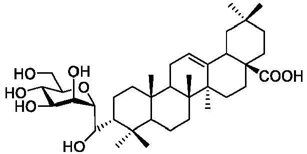 | (3R,5S,8R,9R, 10S, 14R, 17R, 18 S)-Oleanolicacid-3-(1'S)-(hydroxyl)methyl-α-D-mannopyranoside |
| A21 | 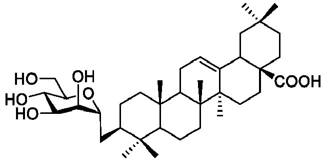 | (3S,5S,8R,9R,10S,14R,17R,18 S)-Oleanolicacid-3-methyl-α-D-mannopyranoside |
| A22 | 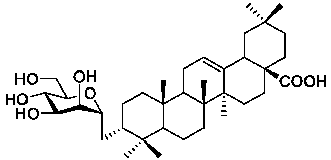 | (3R,5S,8R,9R,10S,14R,17R,18 S)-Oleanolicacid-3-methyl-α-D-mannopyranoside |
| A23 | 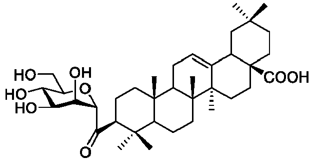 | (3S,5S,8R,9R,10S,14R,17R,18 S)-Oleanolicacid-3-carbonyl-α-D-mannopyranoside |
| A24 | 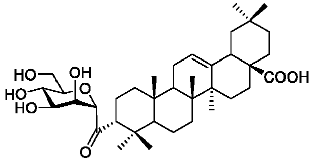 | (3R,5S,8R,9R, 10S, 14R, 17R, 18 S)-Oleanolicacid-3-carbonyl-α-D-mannopyranoside |
| A25 | 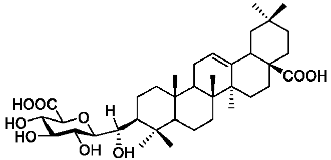 | (3S,5S,8R,9R,10S,14R,17R,18 S)-Oleanolicacid-3-(1'R)-(hydroxyl)methyl-β-D-glucurouopyranoside |
| A26 | 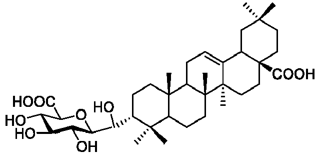 | (3R,5S,8R,9R, 10S, 14R, 17R, 18 S)-Oleanolicacid-3-(1'R)-(hydroxyl)methyl-β-D-glucurouopyranoside |
| A27 | 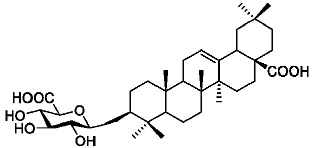 | (3S,5S,8R,9R,10S,14R,17R,18 S)-Oleanolicacid-3-methyl-β-D-glucurouopyranoside |
| A28 | 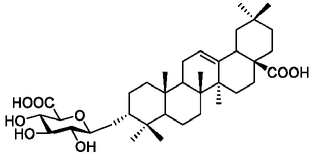 | (3R,5S,8R,9R,10S,14R,17R,18 S)-Oleanolicacid-3-methyl-β-D-glucurouopyranoside |
| A29 | 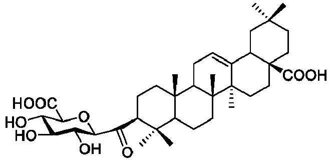 | (3S,5S,8R,9R,10S,14R,17R,18 S)-Oleanolicacid-3-carbonyl-β-D-glucurouopyranoside |
| A30 | 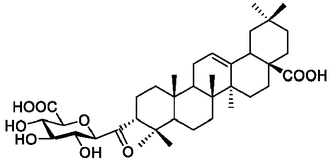 | (3R,5S,8R,9R, 10S, 14R, 17R, 18 S)-Oleanolicacid-3-carbonyl-β-D-glucurouopyranoside |
| A31 | 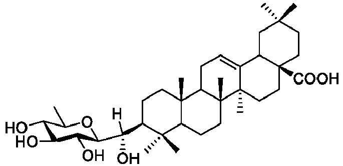 | (3S,5S,8R,9R,10S,14R,17R,18 S)-Oleanolicacid-3-(1'R)-(hydroxyl)methyl-6"-deoxy-β-D-glucopyranoside |
| A32 | 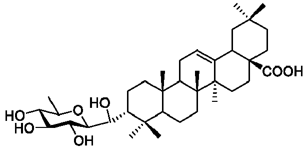 | (3R,5S,8R,9R,10S,14R,17R,18 S)-Oleanolicacid-3-(1'R)-(hydroxyl)methyl-6"-deoxy-β-D-glucopyranoside |
| A33 | 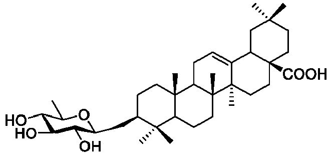 | (3S,5S,8R,9R,10S,14R,17R,18 S)-Oleanolicacid-3-methyl-6"-deoxy-β-D-glucopyranoside |
| A34 | 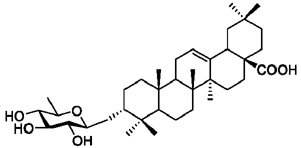 | (3R,5S,8R,9R,10S,14R,17R,18 S)-Oleanolicacid-3-methyl-6"-deoxy-β-D-glucopyranoside |
| A35 | 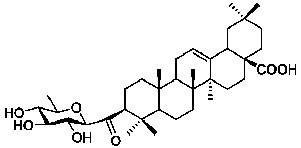 | (3S,5S,8R,9R,10S,14R,17R,18 S)-Oleanolicacid-3-carbonyl-6"-deoxy-β-D-glucopyranoside |
| A36 | 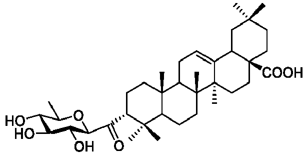 | (3R,5S,8R,9R,10S,14R,17R,18 S)-Oleanolicacid-3-carbonyl-6"-deoxy-β-D-glucopyranoside |
| A37 | 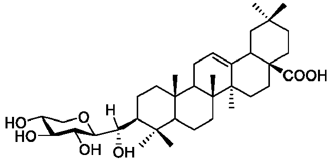 | (3S,5S,8R,9R,10S,14R,17R,18 S)-Oleanolicacid-3-(1'R)-(hydroxyl)methyl-β-D-xylopyranoside |
| A38 | 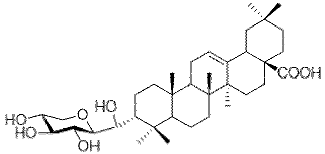 | (3R,5S,8R,9R,10S,14R,17R,18 S)-Oleanolicacid-3-(1'R)-(hydroxyl)methyl-β-D-xylopyranoside |
| A39 | 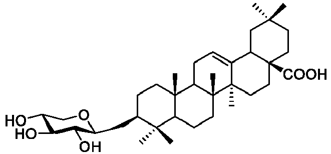 | (3S,5S,8R,9R,10S,14R,17R,18 S)-Oleanolicacid-3-methyl-β-D-xylopyranoside |
| A40 | 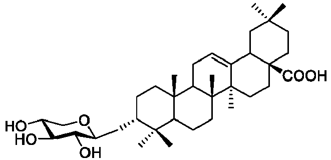 | (3R,5S,8R,9R,10S,14R,17R,18 S)-Oleanolicacid-3-methyl-β-D-xylopyranoside |
| A41 | 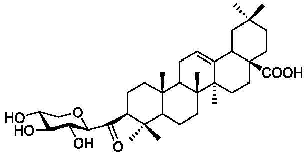 | (3S,5S,8R,9R,10S,14R,17R,18 S)-Oleanolicacid-3-carbonyl-β-D-xylopyranoside |
| A42 | 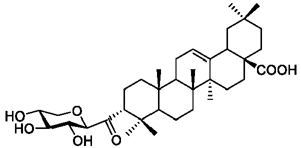 | (3R,5S,8R,9R,10S,14R,17R,18 S)-Oleanolicacid-3-carbonyl-β-D-xylopyranoside |
| A43 | 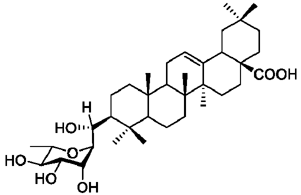 | (3S,5S,8R,9R,10S,14R,17R,18 S)-Oleanolicacid-3-(1'S)-(hydroxyl)methyl-α-L-rhamnopyrano side |
| A44 | 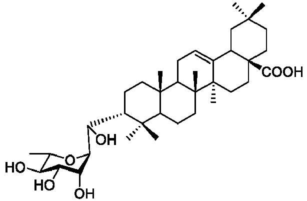 | (3R,5S,8R,9R,10S,14R,17R,18 S)-Oleanolicacid-3-(1'S)-(hydroxyl)methyl-α-L-rhamnopyrano side |
| A45 | 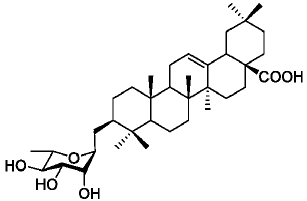 | (3S,5S,8R,9R,10S,14R,17R,18 S)-Oleanolicacid-3-(1' S)-methyl-α-L-rhamnopyranoside |
| A46 | 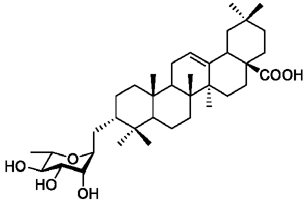 | (3R,5S,8R,9R,10S,14R,17R,18 S)-Oleanolicacid-3-(1'S)-methyl-α-L-rhamnopyranoside |
| A47 | 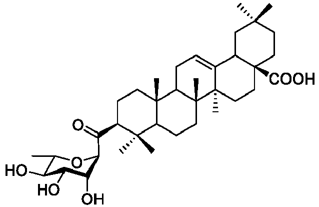 | (3S,5S,8R,9R,10S,14R,17R,18 S)-Oleanolicacid-3-(1'S)-carbonyl-α-L-rhamnopyrano side |
| A48 | 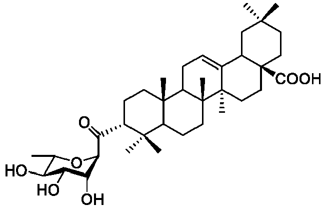 | (3R,5S,8R,9R,10S,14R,17R,18 S)-Oleanolicacid-3-(1'S)-carbonyl-α-L-rhamnopyrano side |
| A49 | 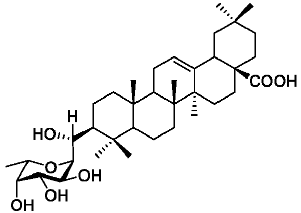 | (3S,5S,8R,9R,10S,14R,17R,18 S)-Oleanolicacid-3-(1'S)-(hydroxyl)methyl-α-L-fucopyranoside |
| A50 | 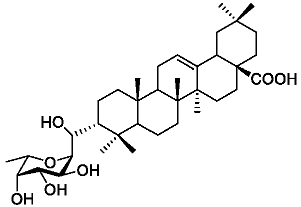 | (3R,5S,8R,9R,10S,14R,17R,18 S)-Oleanolicacid-3-(1'S)-(hydroxyl)methyl-α-L-fucopyranoside |
| A51 | 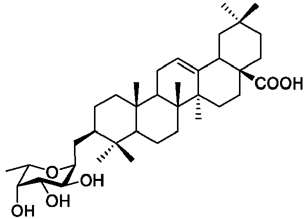 | (3S,5S,8R,9R,10S,14R,17R,18 S)-Oleanolicacid-3-(1'S)-methyl-α-L-fucopyranoside |
| A52 | 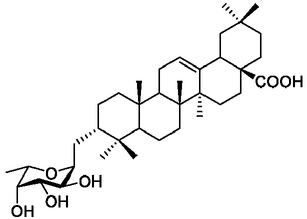 | (3R,5S,8R,9R,10S,14R,17R,18 S)-Oleanolicacid-3-(1'S)-methyl-α-L-fucopyranoside |
| A53 | 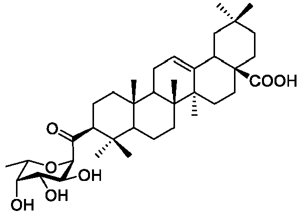 | (3S,5S,8R,9R,10S,14R,17R,18 S)-Oleanolicacid-3-(1'S)-carbonyl-α-L-fucopyranoside |
| A54 | 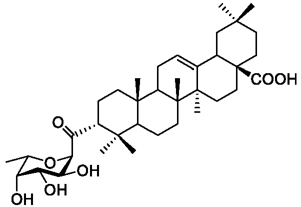 | (3R,5S,8R,9R,10S,14R,17R,18 S)-Oleanolicacid-3-(1' S)-carbonyl-α-L-fucopyranoside |
| A55 | 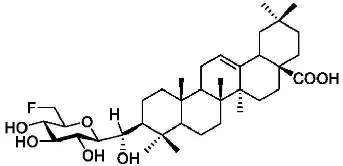 | (3S,5S,8R,9R,10S,14R,17R,18 S)-Oleanolicacid-3-(1'R)-(hydroxyl)methyl-6"-deoxy-6"-fluoro-β-D-glucopyranoside |
| A56 | 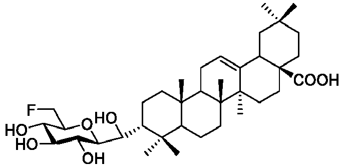 | (3R,5S,8R,9R,10S,14R,17R,18 S)-Oleanolicacid-3-(1'R)-(hydroxyl)methyl-6"-deoxy-6"-fluoro-β-D-glucopyranoside |
| A57 | 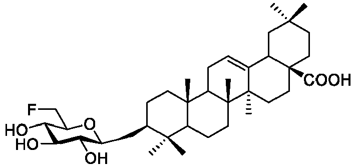 | (3S,5S,8R,9R,10S,14R,17R,18 S)-Oleanolicacid-3-methyl-6"-deoxy-6"-fluoro-β-D-glucopyranoside |
| A58 | 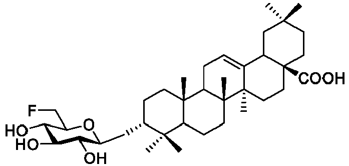 | (3R,5S,8R,9R, 10S, 14R, 17R, 18 S) -Oleanolic acid- 3 -methyl-6"-deoxy-6"-fluoro-β-D-glucopyranoside |
| A59 | 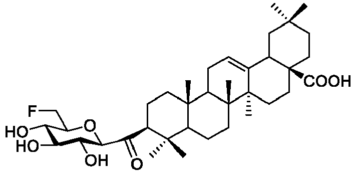 | (3S,5S,8R,9R, 10S, 14R, 17R, 18 S) -Oleanolic acid- 3 -carbonyl-6"-deoxy-6"-fluoro-β-D-glucopyranoside |
| A60 | 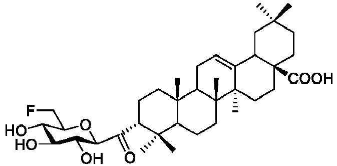 | (3R,5S,8R,9R,10S,14R,17R,18 S)-Oleanolic acid-3-carbonyl-6"-deoxy-6"-fluoro-β-D-glucopyranoside |
| A61 | 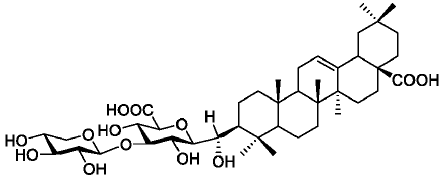 | (3S,5S,8R,9R,10S,14R,17R,18 S)-Oleanolicacid-3-(1'R)-(hydroxyl)methyl-β-D-xylopyranosyl(1→3)-β-D-glucurouopyranoside |
| A62 | 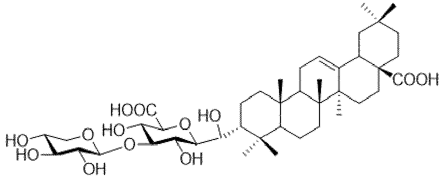 | (3R,5S,8R,9R,10S,14R,17R,18 S)-Oleanolicacid-3-(1'R)-(hydroxyl)methyl-β-D-xylopyranosyl(1→3)-β-D-glucurouopyranoside |
| A63 | 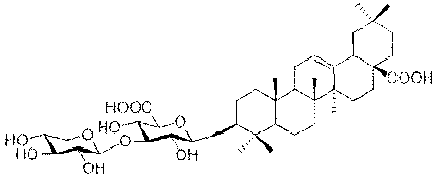 | (3S,5S,8R,9R,10S,14R,17R,18 S)-Oleanolicacid-3-methyl-β-D-xylopyranosyl(1→3)-β-D-glucurouopyranoside |
| A64 | 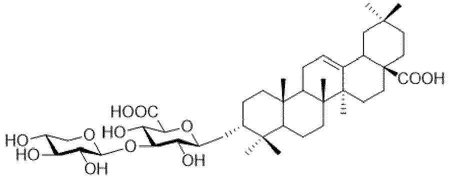 | (3R,5S,8R,9R,10S,14R,17R,18 S)-Oleanolicacid-3-methyl-β-D-xylopyranosyl(1→3)-β-D-glucurouopyranoside |
| A65 | 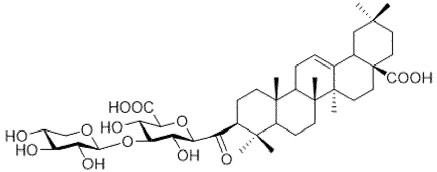 | (3S,5S,8R,9R,10S,14R,17R,18 S)-Oleanolicacid-3-carbonyl-β-D-xylopyranosyl(1→3)-β-D-glucurouopyranoside |
| A66 | 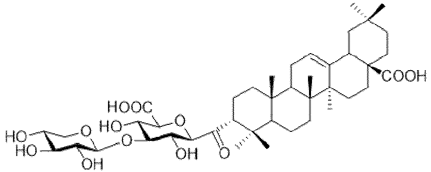 | (3R,5S,8R,9R,10S,14R,17R,18 S)-Oleanolicacid-3-carbonyl-β-D-xylopyranosyl(1→3)-β-D-glucurouopyranoside |
| A67 | 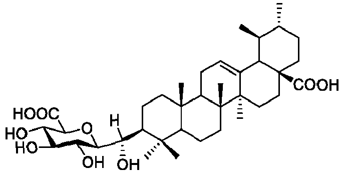 | (3S,5S,8R,9R,10S,14R,17R,18 S,19S,20R)-Ursolicacid-3-(1'R)-(hydroxyl)methyl-β-D-glucurouopyranoside |
| A68 | 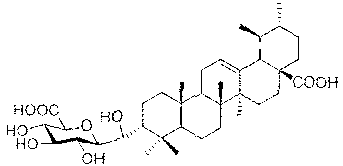 | (3R,5S,8R,9R,10S,14R,17R,18 S,19S,20R)-Ursolicacid-3-(1'R)-(hydroxyl)methyl-β-D-glucurouopyranoside |
| A69 | 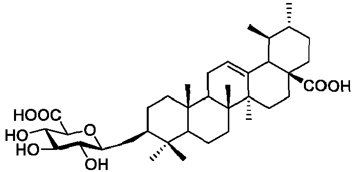 | (3S,5S,8R,9R,10S,14R,17R,18 S,19S,20R)-Ursolicacid-3-methyl-β-D-glucurouopyranoside |
| A70 | 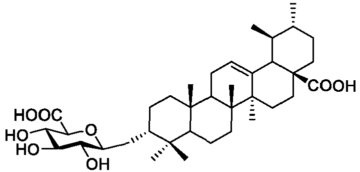 | (3R,5S,8R,9R,10S,14R,17R,18 S,19S,20R)-Ursolicacid-3-methyl-β-D-glucurouopyranoside |
| A71 | 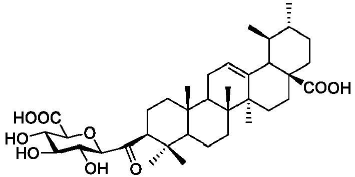 | (3S,5S,8R,9R,10S,14R,17R,18 S,19S,20R)-Ursolicacid-3-carbonyl-β-D-glucurouopyranoside |
| A72 | 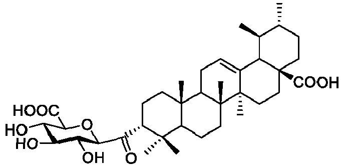 | (3R,5S,8R,9R,10S,14R,17R,18 S,19S,20R)-Ursolicacid-3-carbonyl-β-D-glucurouopyranoside |
| A73 | 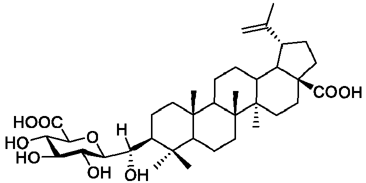 | (3S,5S,8R,9R,10S,13R,14R,17 S,18R,19R)-Betulinicacid-3-(1'R)-(hydroxyl)methyl-β-D-glucurouopyranoside |
| A74 | 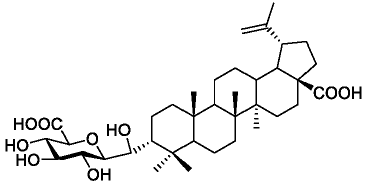 | (3S,5S,8R,9R,10S,13R,14R,17 S,18R,19R)-Betulinicacid-3-(1'R)-(hydroxyl)methyl-β-D-glucurouopyranoside |
| A75 | 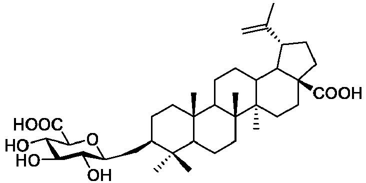 | (3S,5S,8R,9R,10S,13R,14R,17 S,18R,19R)-Betulinicacid-3-methyl-β-D-glucurouopyranoside |
| A76 | 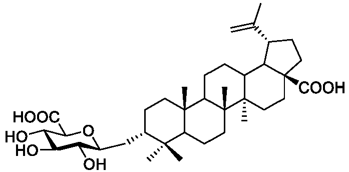 | (3S,5S,8R,9R,10S,13R,14R, 17 S,18R,19R)-Betulinicacid-3-methyl-β-D-glucurouopyranoside |
| A77 | 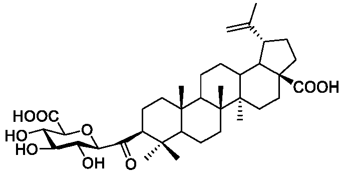 | (3S,5S,8R,9R,10S,13R,14R,17 S,18R,19R)-Betulinicacid-3-carbonyl-β-D-glucurouopyranoside |
| A78 | 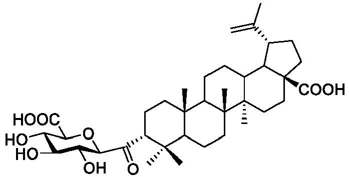 | (3S,5S,8R,9R,10S,13R,14R,17 S,18R,19R)-Betulinicacid-3-carbonyl-β-D-glucurouopyranoside |
| A79 | 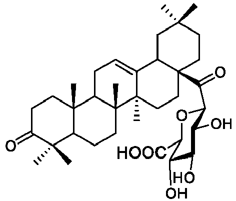 | (5S,8R,9R,10S,14R,17R,18S)-3-Carbonyloleanolicacid-28-β-D-glucurouopyranoside |
| A80 | 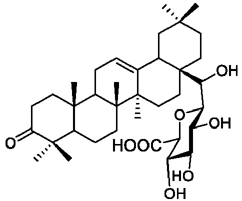 | (5S,8R,9R,10S,14R,17R,18S)-3-Carbonyloleanolicacid-17-(1'R)-(hydroxyl)methyl-β-D-glucurouopyranoside |
| A81 | 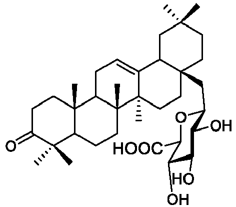 | (5S,8R,9R,10S,14R,17R,18S)-3-Carbonyloleanolicacid-17-methyl-β-D-glucurouopyranoside |
| A82 | 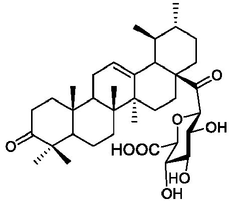 | (5S,8R,9R,10S,14R,17R,18S1 9S,20R)-3-Carbonylursolicacid-28-β-D-glucurouopyranoside |
| A83 | 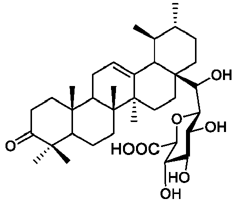 | (5S,8R,9R,10S,14R,17R,18S1 9S,20R)-3-Carbonylursolicacid-17-(1'R)-(hydroxyl)methyl-β-D-glucurouopyranoside |
| A84 | 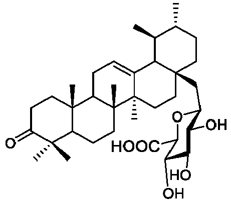 | (5S,8R,9R,10S,14R,17R,18S,1 9S,20R)-3-Carbonylursolicacid-17-methyl-β-D-glucurouopyranoside |
| A85 | 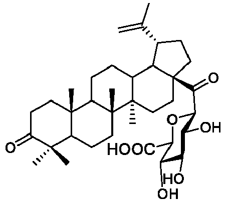 | (5S,8R,9R,10S,13R,14R,17S,1 8R,19R)-3-Carbonylbetulinicacid-28-(3-D-glucurouopyranoside |
| A86 | 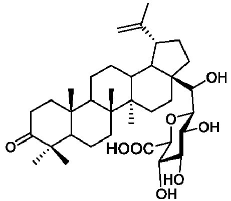 | (5S,8R,9R,10S,13R,14R,17S,1 8R,19R)-3-Carbonylbetulinicacid-17-(1'R)-(hydroxyl)methyl-β-D-glucurouopyranoside |
| A87 | 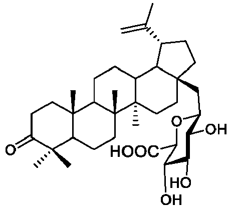 | (5S,8R,9R,10S,13R,14R,17S,1 8R,19R)-3-Carbonylbetulinicacid-17-methyl-β-D-glucurouopyranoside |
| A88 | 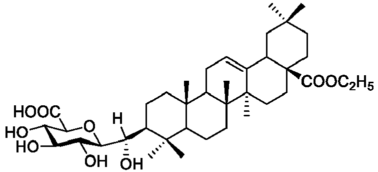 | (3S,5S,8R,9R,10S,14R,17R,18 S)-28-O-Ethyloleanolicacid-3-(1'R)-(hydroxyl)methyl-β-D-glucurouopyranoside |
| A89 | 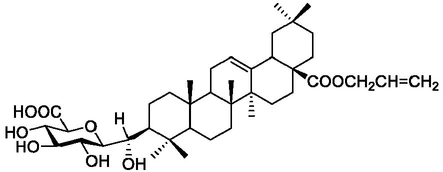 | (3S,5S,8R,9R,10S,14R,17R,18 S)-28-O-Allyloleanolicacid-3-(1'R)-(hydroxyl)methyl-β-D-glucurouopyranoside |
| A90 | 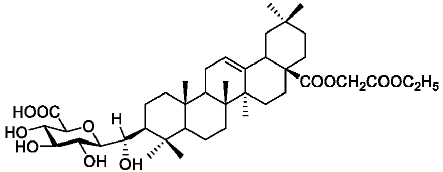 | (3S,5S,8R,9R,10S,14R,17R,18 S)-28-O-(O-Ethyl)carboxymethyloleanolic acid-3-(1'R)-(hydroxyl)methyl-β-D-glucurouopyranoside |
| A91 | 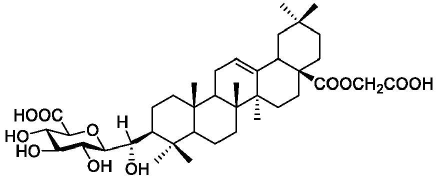 | (3S,5S,8R,9R,10S,14R,17R,18 S)-28-O-Acetoxyoleanolicacid-3-(1'R) - (hydroxyl)methyl-β-D-glucurouopyranoside |
| A92 | 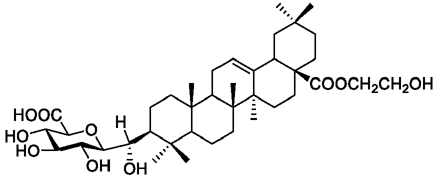 | (3S,5S,8R,9R,10S,14R,17R,18 S)-28-O-Hydroxyethyloleanolicacid-3-(1'R)-(hydroxyl)methyl-β-D-glucurouopyranoside |
| A93 | 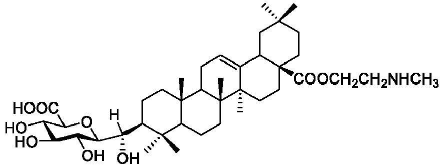 | (3S,5S,8R,9R,10S,14R,17R,18 S)-28-O-(N-Methyl)aminoethyloleanolicac id-3-(1'R)-(hydroxyl)methyl-β-D-glucurouopyranoside |
| A94 | 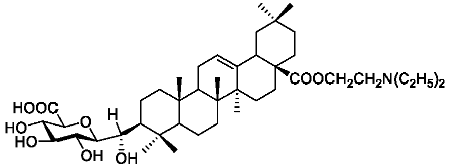 | (3S,5S,8R,9R,10S,14R,17R,18 S)-28-O-(N,N-Diethyl)aminoethyloleanolicac id-3-(1'R)-(hydroxyl)methyl-β-D-glucurouopyranoside |
| A95 | 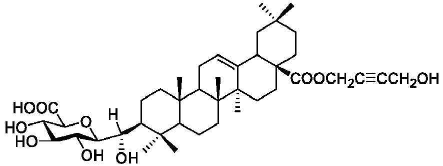 | (3S,5S,8R,9R,10S,14R,17R,18 S)-28-O-Hydroxylbut-2" "-ynyloleanolicacid-3-(1'R)-(hydroxyl)methyl-β-D-glucurouopyranoside |
| A96 | 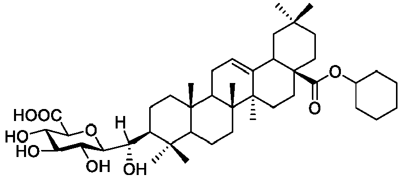 | (3S,5S,8R,9R,10S,14R,17R,18 S)-28-O-Cyclohexyloleanolicacid-3-(1'R)-(hydroxyl)methyl-β-D-glucurouopyranoside |
| A97 | 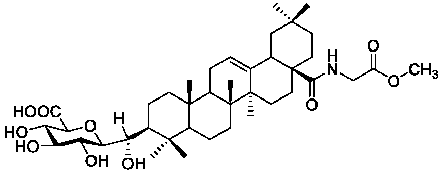 | (3S,5S,8R,9R,10S,14R,17R,18 S)-28-(O-Methyl)carboxymethylaminool eanolicacid-3-(1'R)-(hydroxyl)methyl-β-D-glucurouopyranoside |
| A98 | 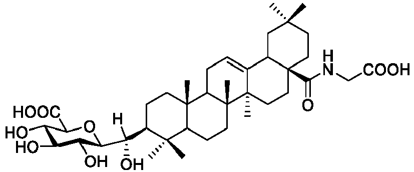 | (3S,5S,8R,9R,10S,14R,17R,18 S)-28-Carboxymethylaminooleanolic acid-3-(1'R)-(hydroxyl)methyl-β-D-glucurouopyranoside |
| A99 | 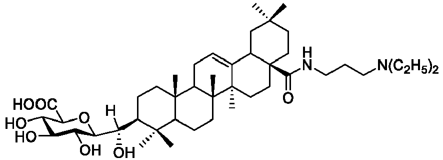 | (3S,5S,8R,9R,10S,14R,17R,18 S)-28-(N,N-Diethyl)aminopropylaminoole anolicacid-3-(1'R)-(hydroxyl)methyl-β-D-glucurouopyranoside |
| A100 | 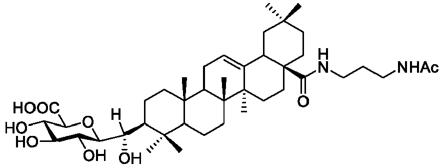 | (3S,5S,8R,9R,10S,14R,17R,18 S)-28-(N-Acetyl)aminopropylaminoolea nolicacid-3-(1'R)-(hydroxyl)methyl-β-D-glucurouopyranoside |
| A101 | 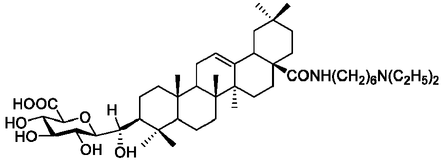 | (3S,5S,8R,9R,10S,14R,17R,18 S)-28-(N,N-Diethyl)aminohexylaminoolea nolicacid-3-(1'R)-(hydroxyl)methyl-β-D-glucurouopyranoside |
| A102 | 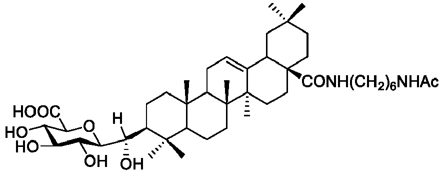 | (3S,5S,8R,9R,10S,14R,17R,18 S)-28-(N-Acetyl)aminohexylaminoolean olicacid-3-(1'R)-(hydroxyl)methyl-β-D-glucurouopyranoside |
| A103 | 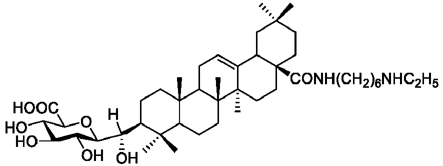 | (3S,5S,8R,9R,10S,14R,17R,18 S)-28-(N-Ethyl)aminohexylaminooleano licacid-3-(1'R)-(hydroxyl)methyl-β-D-glucurouopyranoside |
| A104 | 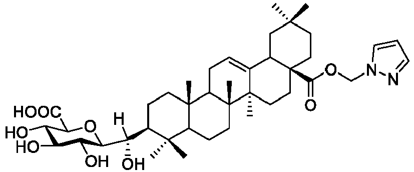 | (3S,5S,8R,9R,10S,14R,17R,18 S)-28-O-(Pyrazol-1"-yl)methyloleanolicacid-3-(1'R)-(hydroxyl)methyl-β-D-glucurouopyranoside |
| A105 | 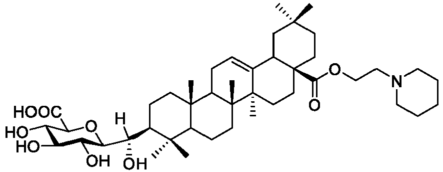 | (3S,5S,8R,9R,10S,14R,17R,18 S)-28-O-(Piperidin-1"-yl)methyloleanolicacid-3-(1'R)-(hydroxyl)methyl-β-D-glucurouopyranoside |
| A106 | 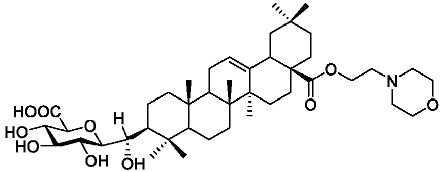 | (3S,5S,8R,9R,10S,14R,17R,18 S)-28-O-(Morpholin-4"-yl)methyloleanolicacid-3-(1'R)-(hydroxyl)methyl-β-D-glucurouopyranoside |
| A107 | 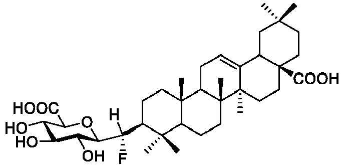 | (3S,5S,8R,9R,10S,14R,17R,18 S)-Oleanolicacid-3-(1'R)-fluoromethyl-β-D-glucurouopyranoside |
| A108 | 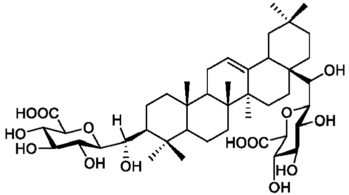 | (3S,5S,8R,9R,10S,14R,17R,18 S)-Oleanolicacid-3-(1'R)-(hydroxyl)methyl-28-(1"R)-(hydroxyl)methyl-β-D-diglucurouopyrano side |
| A109 | 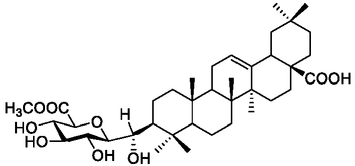 | (3S,5S,8R,9R,10S,14R,17R,18 S)-Oleanolicacid-3-(1'R)-(hydroxyl)methyl-6"-O-methyl-β-D-glucurouopyranoside |
| A110 | 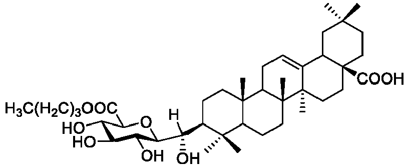 | (3S,5S,8R,9R,10S,14R,17R,18 S)-Oleanolicacid-3-(1'R)-(hydroxyl)methyl-6"-O-n-butyl-β-D-glucurouopyranoside |
| A111 | 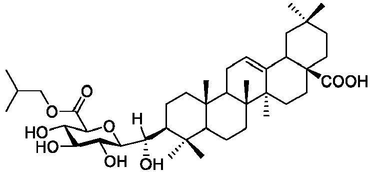 | (3S,5S,8R,9R,10S,14R,17R,18 S)-Oleanolicacid-3-(1'R)-(hydroxyl)methyl-6"-O-isobutyl-β-D-glucurouopyranoside |
| A112 | 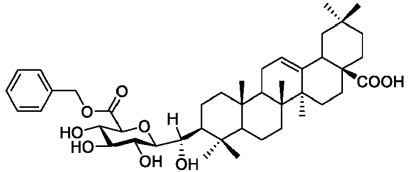 | (3S,5S,8R,9R,10S,14R,17R,18 S)-Oleanolicacid-3-(1'R)-(hydroxyl)methyl-6"-O-benzyl-β-D-glucurouopyranoside |
| A113 | 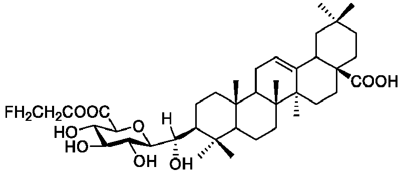 | (3S,5S,8R,9R,10S,14R,17R,18 S)-Oleanolicacid-3-(1'R)-(hydroxyl)methyl-6"-O-(1-fluoroacetyl)-β-D-glucurouopyranoside |
| A114 | 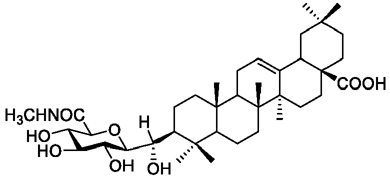 | (3S,5S,8R,9R,10S,14R,17R,18 S)-Oleanolicacid-3-(1'R)-(hydroxyl)methyl-6"-methylamino-β-D-glucurouopyranoside |
| A115 | 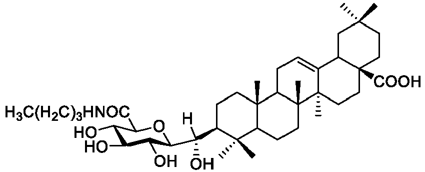 | (3S,5S,8R,9R,10S,14R,17R,18 S)-Oleanolicacid-3-(1'R)-(hydroxyl)methyl-6"-n-butylamino-β-D-glucurouopyranoside |
| A116 | 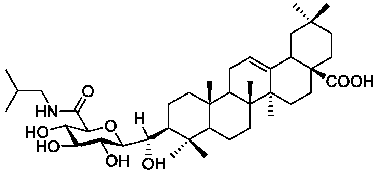 | (3S,5S,8R,9R,10S,14R,17R,18 S)-Oleanolicacid-3-(1'R)-(hydroxyl)methyl-6"-isobutylamino-β-D-glucurouopyranoside |
| A117 | 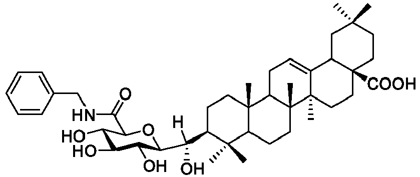 | (3S,5S,8R,9R,10S,14R,17R,18 S)-Oleanolicacid-3-(1'R)-(hydroxyl)methyl-6"-benzylamino-β-D-glucurouopyranoside |
| A118 | 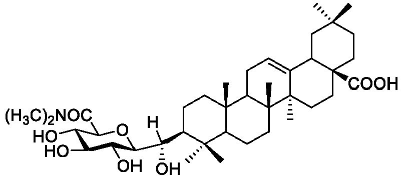 | (3S,5S,8R,9R,10S,14R,17R,18 S)-Oleanolicacid-3-(1'R)-(hydroxyl)methyl-6"-dimethylamino-β-D-glucurouopyranoside |
| A119 | 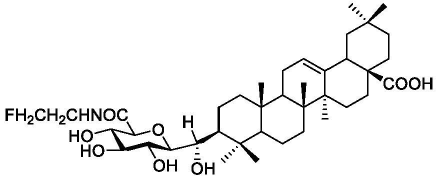 | (3S,5S,8R,9R,10S,14R,17R,18 S)-Oleanolicacid-3-(1'R)-(hydroxyl)methyl-6"-(1-fluoroethylamino)-β-D-glucurouopyranoside |
| A120 | 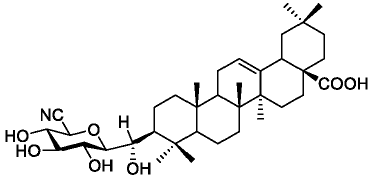 | (3S,5S,8R,9R,10S,14R,17R,18 S)-Oleanolicacid-3-(1'R)-(hydroxyl)methyl-5"-cyano-β-D-xylopyranoside |
| A121 | 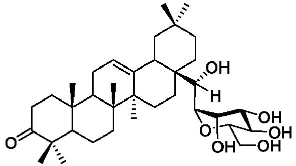 | (5S,8R,9R,10S,14R,17R,18S)-3-Carbonyloleanolicacid-17-(1'S)-(hydroxyl)methyl-β-D-mannopyranoside |
| A122 | 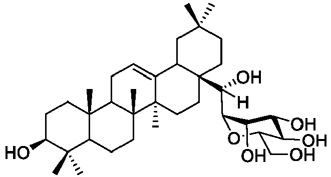 | (3S,5S,8R,9R,10S,14R,17R,18 S)-Oleanolicacid-17-(1'S)-(hydroxyl)methyl-β-D-mannopyranoside |
| A123 | 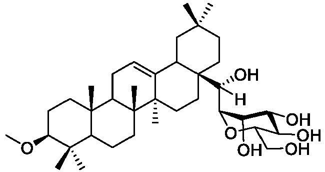 | (3S,5S,8R,9R,10S,14R,17R,18 S)-3-Methoxyoleanolicacid-17-(1'S)-(hydroxyl)methyl-β-D-mannopyranoside |
| A124 | 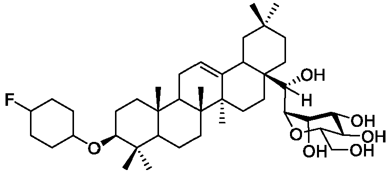 | (3S,5S,8R,9R,10S,14R,17R,18 S)-3-(4-Fluorocyclohexoxy)oleanolica cid-17-(1'S)-(hydroxyl)methyl-β-D-mannopyranoside |
| A125 | 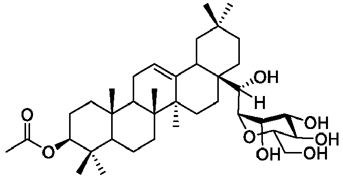 | (3S,5S,8R,9R,10S,14R,17R,18 S)-3-Acetoxyoleanolicacid-17-(1'S)-(hydroxyl)methyl-β-D-mannopyranoside |
| A126 | 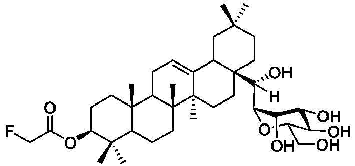 | (3S,5S,8R,9R,10S,14R,17R,18 S)-3-Fluorocetoxyoleanolicacid-17-(1'S)-(hydroxyl)methyl-β-D-mannopyranoside |
| A127 | 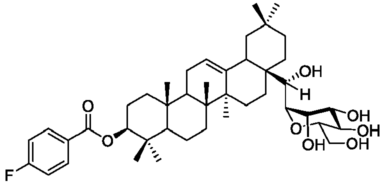 | (3S,5S,8R,9R,10S,14R,17R,18 S)-3-(4-fluorophenylacetoxy)oleanolic acid-17-(1'S)-(hydroxyl)methyl-β-D-mannopyranoside |
| A128 | 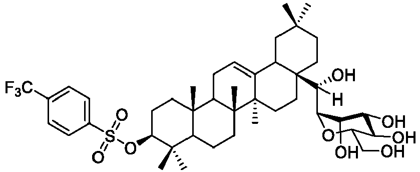 | (3S,5S,8R,9R,10S,14R,17R,18 S)-3-(4-Trifluoromethylphenylsulfonyl oxy)oleanolicacid-17-(1'S)-(hydroxyl)methyl-β-D-mannopyranoside |
| A129 | 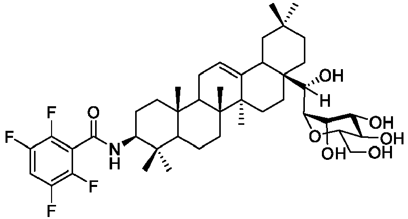 | (3S,5S,8R,9R,10S,14R,17R,18 S)-3-(2,3,5,6-Tetrafluorobenzamide)oleanoli cacid-17-(1'S)-(hydroxyl)methyl-β-D-mannopyranoside |
| A130 | 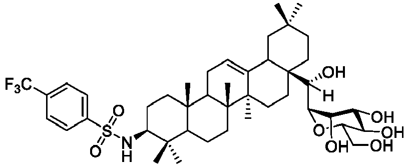 | (3S,5S,8R,9R,10S,14R,17R,18 S)-3-(4-Trifluoromethylbenzenesulfon amido)oleanolicacid-17-(1'S)-(hydroxyl)methyl-β-D-mannopyranoside |
| A131 | 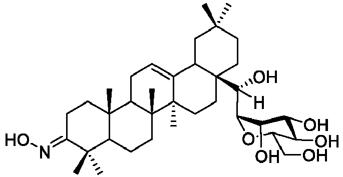 | (5S,8R,9R,10S,14R,17R,18S)-3 -Oximidooleanolicacid-17-(1'S)-(hydroxyl)methyl-β-D-mannopyranoside |
| A132 | 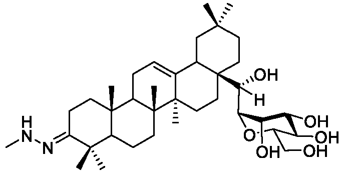 | (5S,8R,9R,10S,14R,17R,18S)-3-Methylhydrazinylideneoleanol icacid-17-(1'S)-(hydroxyl)methyl-β-D-mannopyranoside |
| A133 | 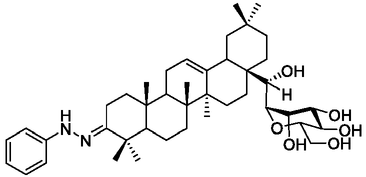 | (5S,8R,9R,10S,14R,17R,18S)-3-Phenylhydrazinylideneoleanoli cacid-17-(1'S)-(hydroxyl)methyl-β-D-mannopyranoside |
| A134 | 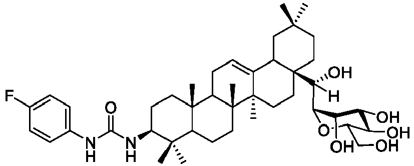 | (3S,5S,8R,9R,10S,14R,17R,18 S)-3-(4-Fluorophenylureido)oleanolica cid-17-(1'S)-(hydroxyl)methyl-β-D-mannopyranoside |
| A135 | 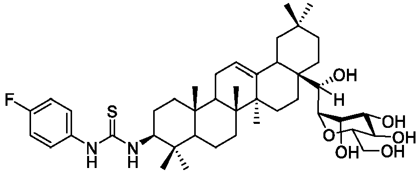 | (3S,5S,8R,9R,10S,14R,17R,18 S)-3-(4-Fluorophenylthioureido)oleano licacid-17-(1'S)-(hydroxyl)methyl-β-D-mannopyranoside |
| A136 | 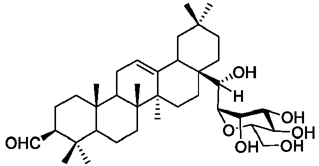 | (3S,5S,8R,9R,10S,14R,17R,18 S)-3-Formyloleanolicacid-17-(1'S)-(hydroxyl)methyl-β-D-mannopyranoside |
| A137 | 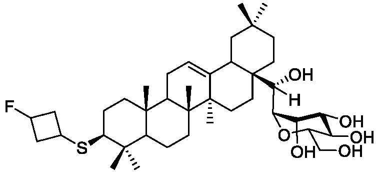 | (3S,5S,8R,9R,10S,14R,17R,18 S)-3-(3-Fluorocyclobutylthio)oleanolic acid-17-(1'S)-(hydroxyl)methyl-β-D-mannopyranoside |
| A138 | 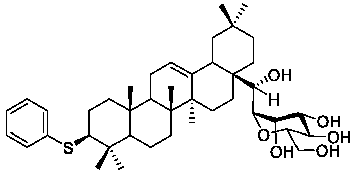 | (3S,5S,8R,9R,10S,14R,17R,18 S)-3-Phenylthiooleanolicacid-17-(1'S)-(hydroxyl)methyl-β-D-mannopyranoside |
| A139 | 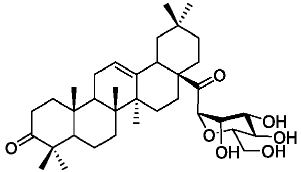 | (5S,8R,9R,10S,14R,17R,18S)-3-Carbonyloleanolicacid-28-β-D-mannopyranoside |
| A140 | 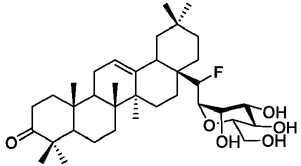 | (5S,8R,9R,10S,14R,17R,18S)-3-Carbonyloleanolicacid-17-(1'S)-fluoromethyl-β-D-mannopyranoside |
| A141 | 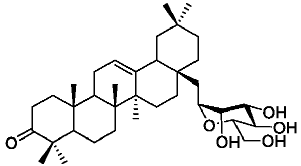 | (5S,8R,9R,10S,14R,17R,18S)-3-Carbonyloleanolicacid-17-methyl-β-D-mannopyranoside |
| A142 | 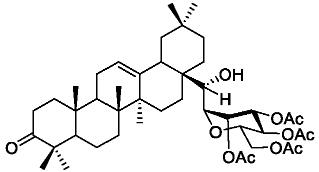 | (5S,8R,9R,10S,14R,17R,18S)-3-Carbonyloleanolicacid-17-(1'S)-(hydroxyl)methyl-2",3",4",6"-O-Tetraacetyl-β-D-mannopyranoside |
| A143 | 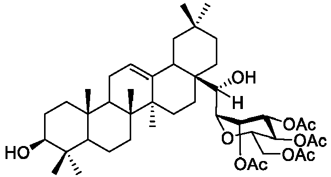 | (3S,5S,8R,9R,10S,14R,17R,18 S)-Oleanolicacid-17-(1'S)-(hydroxyl)methyl-2",3",4",6"-O-tetraacetyl-β-D-mannopyranoside |
| A144 | 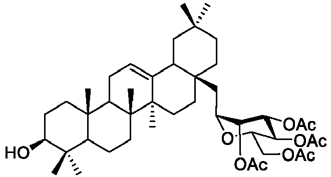 | (5S,8R,9R,10S,14R,17R,18S)-3-Carbonyloleanolicacid-17-methyl-2",3",4",6"-O-tetraacetyl-β-D-mannopyranoside |
| A145 | 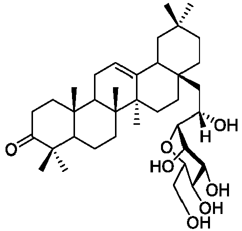 | (5S,8R,9R,10S,14R,17R,18S)-3-Carbonyloleanolicacid-17-(2'S)-(hydroxyl)ethyl-β-D-mannopyranoside |
| A146 | 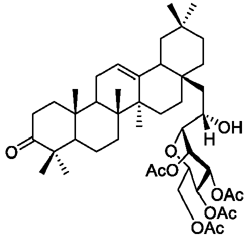 | (5S,8R,9R,10S,14R,17R,18S)-3-Carbonyloleanolicacid-17-(2'S)-(hydroxyl)ethyl-2",3",4",6"-O-Tetraacetyl-β-D-mannopyranoside |
| A147 | 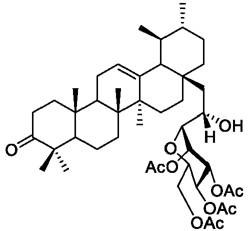 | (5S,8R,9R,10S,14R,17R,18S)-3-Carbonylursolicacid-17-(2' S)-(hydroxyl)ethyl-2",3",4",6"-O-tetraacetyl-β-D-mannopyranoside |
| A148 | 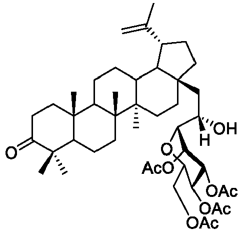 | (5S,8R,9R,10S,14R,17R,18S)-3-C arbonylbetulinic acid-17-(2' S)-(hydroxyl)ethyl-2",3",4",6"-O-tetraacetyl-β-D-mannopyranoside |
| A149 | 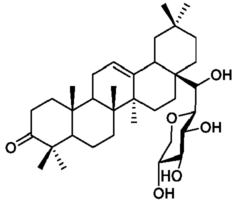 | (5S,8R,9R,10S,14R,17R,18S)-3-Carbonyloleanolicacid-17-(1'R)-(hydroxyl)methyl-β-D-xylopyranoside |
| A150 | 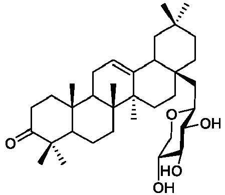 | (5S,8R,9R,10S,14R,17R,18S)-3-Carbonyloleanolicacid-17methyl-β-D-xylopyranoside |
| A151 | 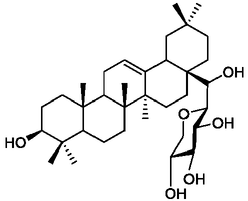 | (3S,5S,8R,9R,10S,14R,17R,18 S)-Oleanolicacid-17-(1'R)-(hydroxyl)methyl-β-D-xylopyranoside |
| A152 | 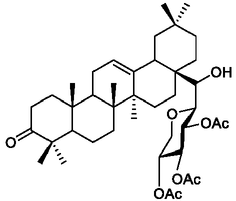 | (5S,8R,9R,10S,14R,17R,18S)-3-Carbonyloleanolicacid-17-(1'R)-(hydroxyl)methyl-2",3",4"-O-triacetyl-β-D-xylopyranoside |
| A153 | 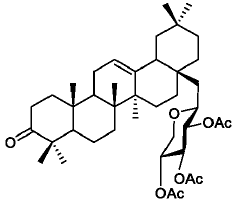 | (5S,8R,9R,10S,14R,17R,18S)-3-Carbonyloleanolicacid-17-methyl-2",3",4"-O-triacetyl-β-D-xylopyranoside |
| A154 | 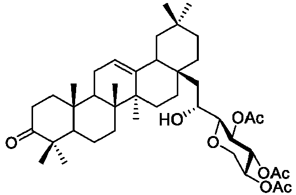 | (5S,8R,9R,10S,14R,17R,18S)-3-Carbonyloleanolicacid-17-(2'R)-(hydroxyl)ethyl-2",3",4"-O-triacetyl-β-D-xylopyranoside |
| A155 | 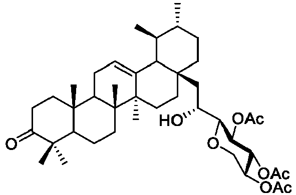 | (5S,8R,9R,10S,14R,17R,18S)-3-Carbonylursolicacid-17-(2'R)-(hydroxyl)ethyl-2",3",4"-O-triacetyl-β-D-xylopyranoside |
| A156 | 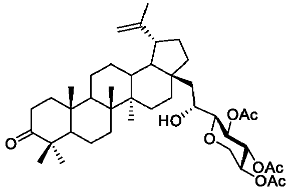 | (5S,8R,9R,10S,14R,17R,18S)-3-C arbonylbetulinic acid-17-(2'R)-(hydroxyl)ethyl-2",3",4"-O-triacetyl-β-D-xylopyranoside |
| A157 | 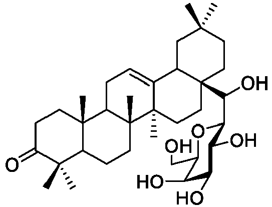 | (5S,8R,9R,10S,14R,17R,18S)-3-Carbonyloleanolicacid-17-(1'R)-(hydroxyl)methyl-β-D-galactopyrano side |
| A158 | 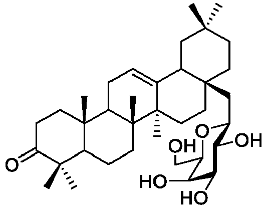 | (5S,8R,9R,10S,14R,17R,18S)-3-Carbonyloleanolicacid-17-methyl-β-D-galactopyranoside |
| A159 | 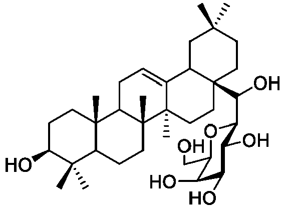 | (3S,5S,8R,9R,10S,14R,17R,18 S)-Oleanolicacid-17-(1'R)-(hydroxyl)methyl-β-D-galactopyrano side |
| A160 | 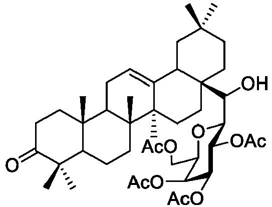 | (5S,8R,9R,10S,14R,17R,18S)-3-Carbonyloleanolicacid-17-(1'R)-(hydroxyl)methyl-2",3",4",6"-O-tetraacetyl-β-D-galactopyranoside |
| A161 | 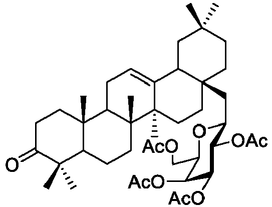 | (5S,8R,9R,10S,14R,17R,18S)-3-Carbonyloleanolicacid-17-methyl-2",3",4",6"-O-tetraacetyl-β-D-galactopyrano side |
| A162 | 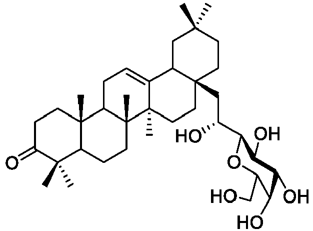 | (5S,8R,9R,10S,14R,17R,18S)-3-Carbonyloleanolicacid-17-(2'R)-(hydroxyl)ethyl-(3-D-galactopyrano side |
| A163 | 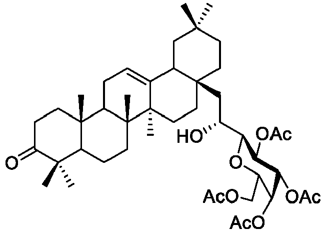 | (5S,8R,9R,10S,14R,17R,18S)-3-Carbonyloleanolicacid-17-(2'R)-(hydroxyl)ethyl-2",3",4",6"-O-tetraacetyl-β-D-galactopyranoside |
| A164 | 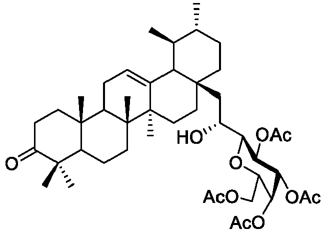 | (5S,8R,9R,10S,14R,17R,18S)-3-Carbonylursolicacid-17-(2'R)-(hydroxyl)ethyl-2",3",4",6"-O-tetraacetyl-β-D-galactopyranoside |
| A165 | 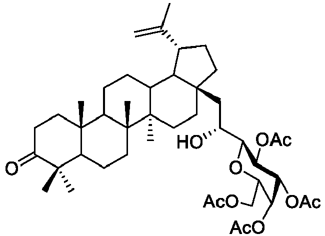 | (5S,8R,9R,10S,14R,17R,18S)-3-C arbonylbetulinic acid-17-(2'R)-(hydroxyl)ethyl-2",3",4",6"-O-tetraacetyl-β-D-galactopyranoside |
| A166 | 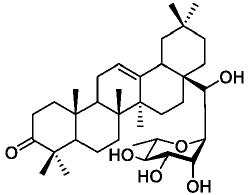 | (5S,8R,9R,10S,14R,17R,18S)-3-Carbonyloleanolicacid-17-(1'R)-(hydroxyl)methyl-α-L-rhamnopyrano side |
| A167 | 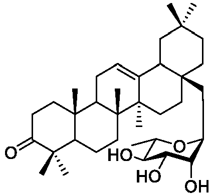 | (5S,8R,9R,10S,14R,17R,18S)-3-Carbonyloleanolicacid-17-methyl-α-L-rhamnopyranoside |
| A168 | 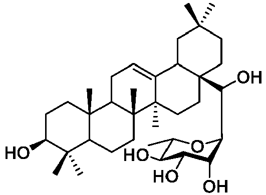 | (3S,5S,8R,9R,10S,14R,17R,18 S)-Oleanolicacid-17-(1'R)-(hydroxyl)methyl-α-L-rhamnopyrano side |
| A169 | 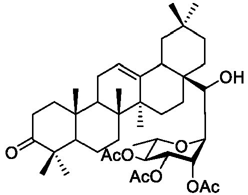 | (5S,8R,9R,10S,14R,17R,18S)-3-Carbonyloleanolicacid-17-(1 'R)-(hydroxyl)methyl-2",3",4"-O-triacetyl-α-L-rhamnopyrano side |
| A170 | 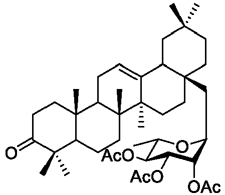 | (5S,8R,9R,10S,14R,17R,18S)-3-Carbonyloleanolicacid-17-methyl-2",3",4"-O-triacetyl-α-L-rhamnopyranoside |
| A171 | 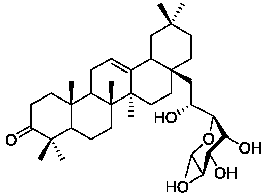 | (5S,8R,9R,10S,14R,17R,18S)-3-Carbonyloleanolicacid-17-(2'R)-(hydroxyl)ethyl-α-L-rhamnopyrano side |
| A172 | 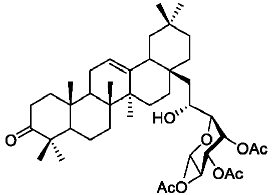 | (5S,8R,9R,10S,14R,17R,18S)-3-Carbonyloleanolicacid-17-(2'R)-(hydroxyl)ethyl-2",3",4"-O-triacetyl-α-L-rhamnopyranoside |
| A173 | 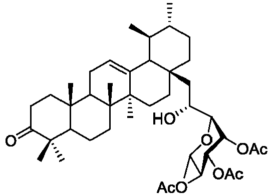 | (5S,8R,9R,10S,14R,17R,18S)-3-Carbonylursolicacid-17-(2'R)-(hydroxyl)ethyl-2",3",4"-O-triacetyl-α-L-rhamnopyrano side |
| A174 | 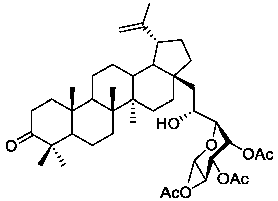 | (5S,8R,9R,10S,14R,17R,18S)-3-C arbonylbetulinic acid-17-(2'R)-(hydroxyl)ethyl-2",3",4"-O-triacetyl-α-L-rhamnopyrano side |
| A175 | 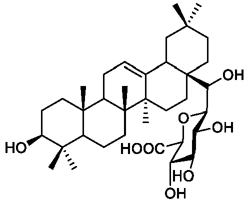 | (3S,5S,8R,9R,10S,14R,17R,18 S)-Oleanolicacid-17-(1'R)-(hydroxyl)methyl-β-D-glucurouopyranoside |
| A176 | 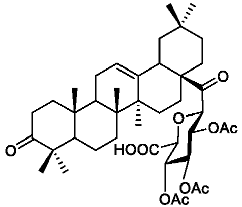 | (5S,8R,9R,10S,14R,17R,18S)-3-Carbonyloleanolicacid-17-carbonyl-2",3",4"-O-triacetyl-β-D-glucurouopyranoside |
| A177 | 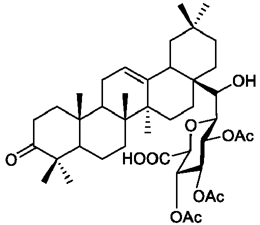 | (5S,8R,9R,10S,14R,17R,18S)-3-Carbonyloleanolicacid-17-(1'R)-(hydroxyl)methyl-2",3",4"-O-triacetyl-β-D-glucurouopyranoside |
| A178 | 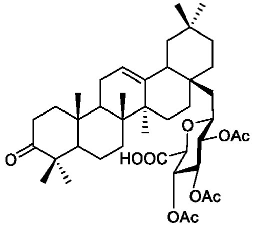 | (5S,8R,9R,10S,14R,17R,18S)-3-Carbonyloleanolicacid-17-methyl-2",3",4"-O-triacetyl-β-D-glucurouopyranoside |
| A179 | 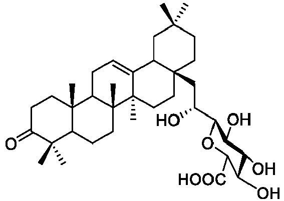 | (5S,8R,9R,10S,14R,17R,18S)-3-Carbonyloleanolicacid-17-(2'R)-(hydroxyl)ethyl-(3-D-glucurouopyranoside |
| A180 | 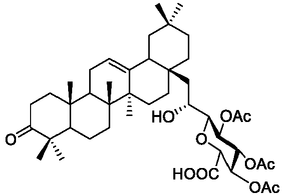 | (5S,8R,9R,10S,14R,17R,18S)-3-Carbonyloleanolicacid-17-(2'R)-(hydroxyl)ethyl-2",3",4"-O-triacetyl-β-D-glucurouopyranoside |
| A181 | 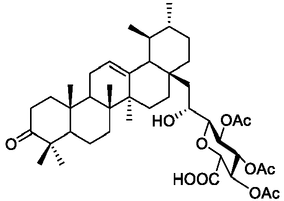 | (5S,8R,9R,10S,14R,17R,18S)-3-Carbonylursolicacid-17-(2'R)-(hydroxyl)ethyl-2",3",4"-O-triacetyl-β-D-glucurouopyranoside |
| A182 | 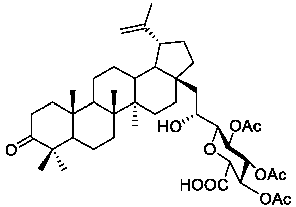 | (5S,8R,9R,10S,14R,17R,18S)-3-C arbonylbetulinic acid-17-(2'R)-(hydroxyl)ethyl-2",3",4"-O-triacetyl-β-D-glucurouopyranoside |
| A183 | 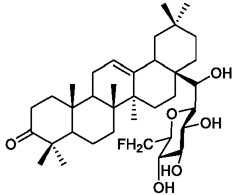 | (5S,8R,9R,10S,14R,17R,18S)-3-Carbonyloleanolicacid-17-(1'R)-(hydroxyl)methyl-6"-deoxy-6"-fluoro-β-D-glucopyranoside |
| A184 | 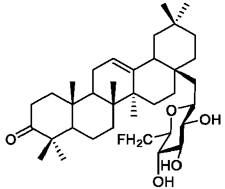 | (5S,8R,9R,10S,14R,17R,18S)-3-Carbonyloleanolicacid-17methyl-6"-deoxy-6"-fluoro-β-D-glucopyranoside |
| A185 | 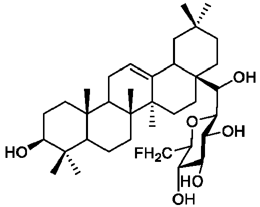 | (3S,5S,8R,9R, 10S, 14R, 17R, 18 S)-Oleanolicacid-17-(1'R)-(hydroxyl)methyl-6"-deoxy-6"-fluoro-β-D-glucopyranoside |
| A186 | 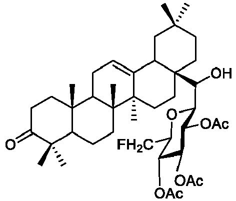 | (5S,8R,9R,10S,14R,17R,18S)-3-Carbonyloleanolicacid-17-(1'R)-(hydroxyl)methyl-2",3",4"-O-triacetyl-6"-deoxy-6"-fluoro-β-D-glucopyranoside |
| A187 | 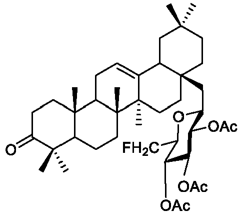 | (5S,8R,9R,10S,14R,17R,18S)-3-Carbonyloleanolicacid-17-methyl-2",3",4"-O-triacetyl-6"-deoxy-6"-fluoro-β-D-glucopyranoside |
| A188 | 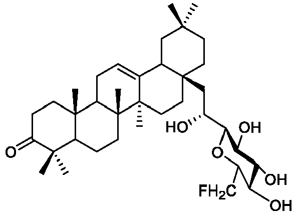 | (5S,8R,9R,10S,14R,17R,18S)-3-Carbonyloleanolicacid-17-(2'R)-(hydroxyl)ethyl-6"-deoxy-6"-fluoro-β-D-glucopyranoside |
| A189 | 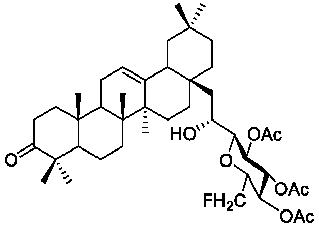 | (5S,8R,9R,10S,14R,17R,18S)-3-Carbonyloleanolicacid-17-(2'R)-(hydroxyl)ethyl-2",3",4"-O-triacetyl-6"-deoxy-6"-fluoro-β-D-glucopyranoside |
| A190 | 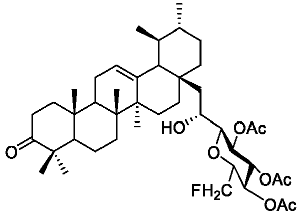 | (5S,8R,9R,10S,14R,17R,18S)-3-Carbonylursolicacid-17-(2'R)-(hydroxyl)ethyl-2",3 ",4"-O-triacetyl-6"-deoxy-6"-fluoro-β-D-glucopyranoside |
| A191 | 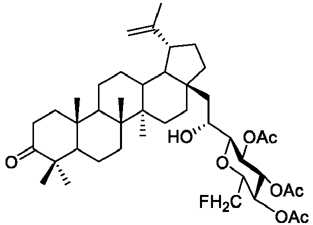 | (5S,8R,9R,10S,14R,17R,18S)-3-Carbonylbetulinicacid-17-(2'R)-(hydroxyl)ethyl-2",3",4"-O-triacetyl-6"-deoxy-6"-fluoro-β-D-glucopyranoside |
| A192 | 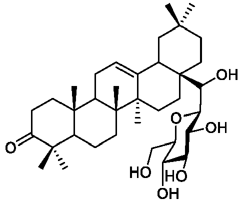 | (5S,8R,9R,10S,14R,17R,18S)-3-Carbonyloleanolicacid-17-(1'R)-(hydroxyl)methyl-β-D-glucopyranoside |
| A193 | 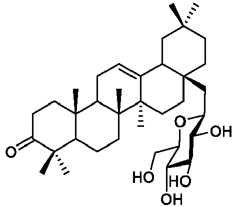 | (5S,8R,9R,10S,14R,17R,18S)-3-Carbonyloleanolicacid-17methyl--β-D-glucopyranoside |
| A194 | 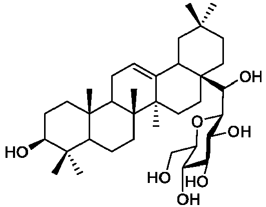 | (3S,5S,8R,9R,10S,14R,17R,18 S)-Oleanolicacid-17-(1'R)-(hydroxyl)methyl-β-D-glucopyranoside |
| A195 | 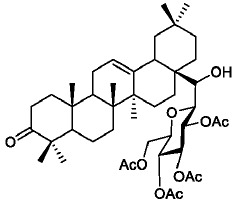 | (5S,8R,9R,10S,14R,17R,18S)-3-Carbonyloleanolicacid-17-(1 'R)-(hydroxyl)methyl-2",3",4",6"-O-tetraacetyl-β-D-glucopyranoside |
| A196 | 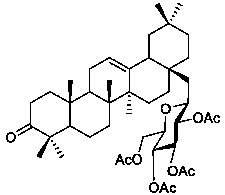 | (5S,8R,9R,10S,14R,17R,18S)-3-Carbonyloleanolicacid-17-methyl-2",3",4",6"-O-tetraacetyl-β-D-glucopyranoside |
| A197 | 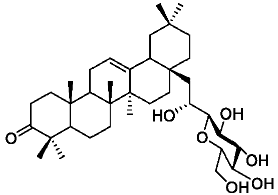 | (5S,8R,9R,10S,14R,17R,18S)-3-Carbonyloleanolicacid-17-(2'R)-(hydroxyl)ethyl-P-D-glucopyranoside |
| A198 | 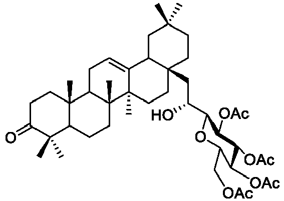 | (5S,8R,9R,10S,14R,17R,18S)-3-Carbonyloleanolicacid-17-(2'R)-(hydroxyl)ethyl-2",3",4",6"-O-tetraacetyl-β-D-glucopyranoside |
| A199 | 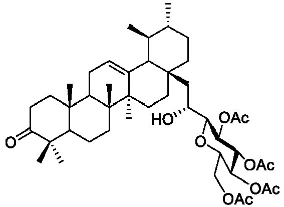 | (5S,8R,9R,10S,14R,17R,18S)-3-Carbonylursolicacid-17-(2'R)-(hydroxyl)ethyl-2",3",4",6"-O-tetraacetyl-β-D-glucopyranoside |
| A200 | 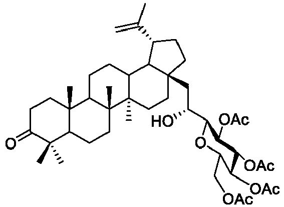 | (5S,8R,9R,10S,14R,17R,18S)-3-C arbonylbetulinic acid-17-(2'R)-(hydroxyl)ethyl-2",3",4",6"-O-tetraacetyl-β-D-glucopyranoside |
| A201 | 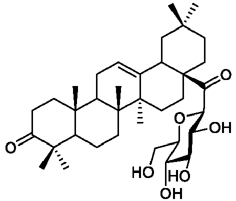 | (5S,8R,9R,10S,14R,17R,18S)-3-Carbonyloleanolicacid-28-β-D-glucopyranoside |
| A202 | 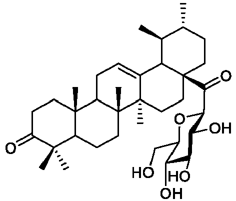 | (5S,8R,9R,10S,14R,17R,18S)-3-Carbonylursolicacid-28-β-D-glucopyranoside |
| A203 | 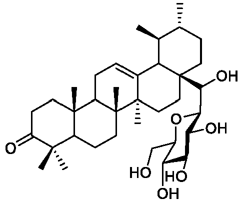 | (5S,8R,9R,10S,14R,17R,18S)-3-Carbonylursolicacid-17-(1'R)-(hydroxyl)methyl-β-D-glucopyranoside |
| A204 | 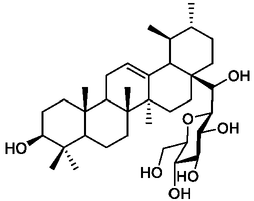 | (3S,5S,8R,9R,10S,14R,17R,18 S)-Ursolicacid-17-(1'R)-(hydroxyl)methyl-β-D-glucopyranoside |
| A205 | 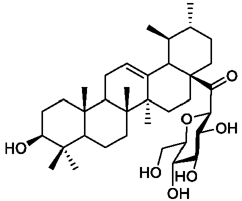 | (3S,5S,8R,9R,10S,14R,17R,18 S)-Ursolicacid-28-β-D-glucopyranoside |
| A206 | 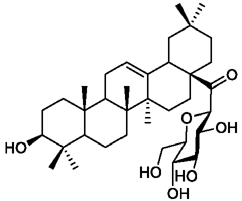 | (3S,5S,8R,9R,10S,14R,17R,18 S)-Oleanolicacid-28-β-D-glucopyranoside |
| A207 | 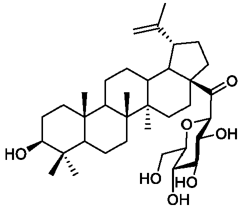 | (3S,5S,8R,9R,10S,13R,14R,17 S,18R,19R)-Betulinicacid-28-β-D-glucopyranoside |
| A208 | 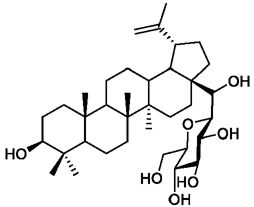 | (3S,5S,8R,9R,10S,13R,14R,17 S,18R,19R)-Betulinicacid-17-(1'R)-(hydroxyl)methyl-β-D-glucopyranoside |
| A209 | 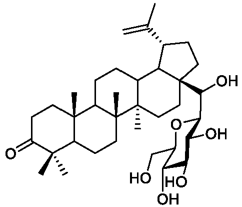 | (5S,8R,9R,10S,13R,14R,17S,1 8R,19R)-3-Carbonylbetulinicacid-17-(1'R)-(hydroxyl)methyl-β-D-glucopyranoside |
| A210 | 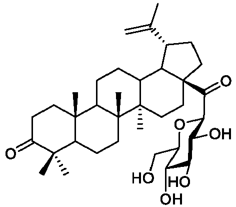 | (5S,8R,9R,10S,13R,14R,17S,1 8R,19R)-3-Carbonylbetulinicacid-28-β-D-glucopyranoside |
| A211 | 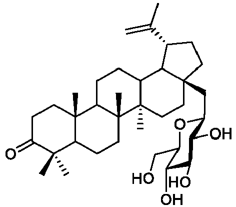 | (5S,8R,9R,10S,13R,14R,17S,1 8R,19R)-3-Carbonylbetulinicacid-17-methyl-β-D-glucopyranoside |
| A212 | 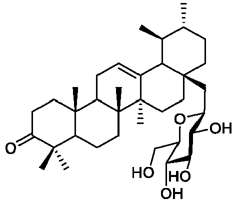 | (5S,8R,9R,10S,14R,17R,18S)-3-Carbonylursolicacid-17-methyl-β-D-glucopyranoside |
| A213 | 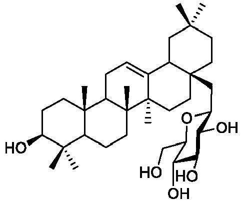 | (3S,5S,8R,9R,10S,14R,17R,18 S)-Oleanolicacid-17-methyl-β-D-glucopyranoside |
| A214 | 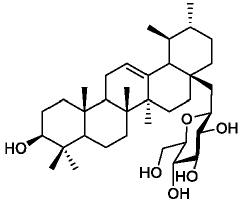 | (3S,5S,8R,9R,10S,14R,17R,18 S)-3-Carbonylursolicacid-17-methyl-β-D-glucopyranoside |
| A215 | 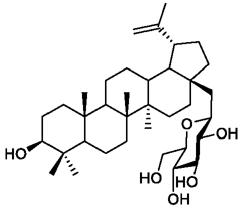 | (3S,5S,8R,9R,10S,13R,14R,17 S,18R,19R)-Betulinicacid-17-methyl-β-D-glucopyranoside |
| A216 | 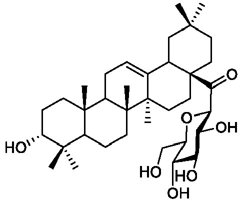 | (3R,5S,8R,9R,10S,14R,17R,18 S)-Oleanolicacid-28-β-D-glucopyranoside |
| A217 | 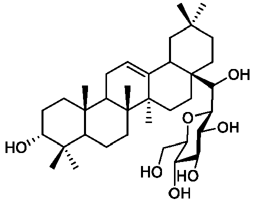 | (3R,5S,8R,9R,10S,14R,17R,18 S)-Oleanolicacid-17-(1'R)-(hydroxyl)methyl-β-D-glucopyranoside |
| A218 | 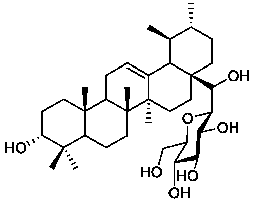 | (3R,5S,8R,9R,10S,14R,17R,18 S)-Ursolicacid-17-(1'R)-(hydroxyl)methyl-β-D-glucopyranoside |
| A219 | 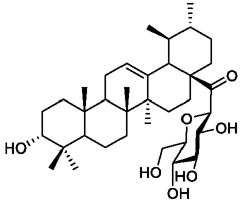 | (3R,5S,8R,9R,10S,14R,17R,18 S)-Ursolicacid-28-β-D-glucopyranoside |
| A220 | 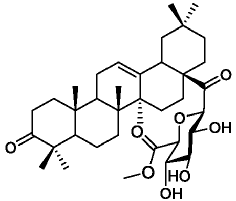 | (5S,8R,9R,10S,14R,17R,18S)-3-Carbonyloleanolicacid-28-6"-O-methyl-β-D-glucurouopyranoside |
| A221 | 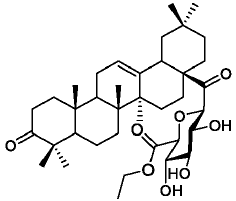 | (5S,8R,9R,10S,14R,17R,18S)-3-Carbonyloleanolicacid-28-6"-O-ethyl-β-D-glucurouopyranoside |
| A222 | 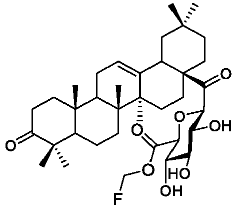 | (5S,8R,9R,10S,14R,17R,18S)-3-Carbonyloleanolicacid-28-6"-O-2‴-fluoroethyl-β-D-glucurouopyranoside |
| A223 | 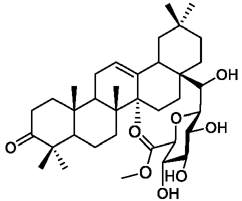 | (5S,8R,9R,10S,14R,17R,18S)-3-Carbonyloleanolicacid-17-(1'R)-(hydroxyl)methyl-6"-O-methyl-β-D-glucurouopyranoside |
| A224 | 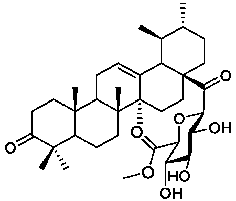 | (5S,8R,9R,10S,14R,17R,18S)-3-Carbonylursolicacid-28-6" - O-methyl-β-D-glucurouopyranoside |
| A225 | 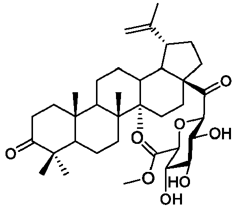 | (3S,5S,8R,9R,10S,13R,14R,17 S,18R,19R)-Betulinicacid-28-6"-O-methyl-β-D-glucurouopyranoside |
| A226 | 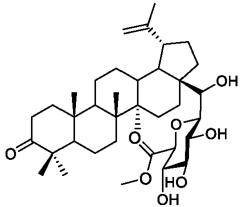 | (5S,8R,9R,10S,14R,17R,18S)-3-C arbonylbetulinic acid-17-(1'R)-(hydroxyl)methyl-6"-O-methyl-β-D-glucurouopyranoside |
| A227 | 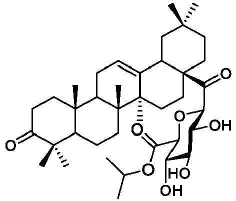 | (5S,8R,9R,10S,14R,17R,18S)-3-Carbonyloleanolicacid-28-6"-O-isopropyl-β-D-glucurouopyranoside |
| A228 | 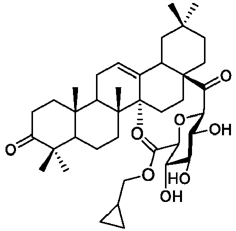 | (5S,8R,9R,10S,14R,17R,18S)-3-Carbonyloleanolicacid-28-6"-O-cyclopropylmethyl-β-D-glucurouopyranoside |
| A229 | 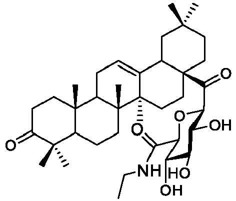 | (5S,8R,9R,10S,14R,17R,18S)-3-Carbonyloleanolicacid-28-6"-N-ethyl-β-D-glucurouopyranoside |
| A230 | 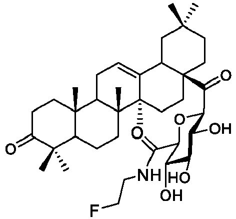 | (5S,8R,9R,10S,14R,17R,18S)-3-Carbonyloleanolicacid-28-6"-N-2"'-fluoroethyl-β-D-glucurouopyranoside |

8. A preparation method of the compound according to claim 1, wherein the preparation method includes the following scheme 1 or scheme 2: 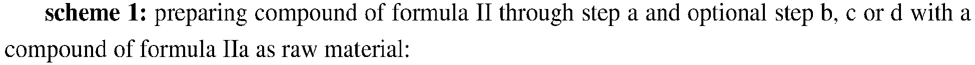 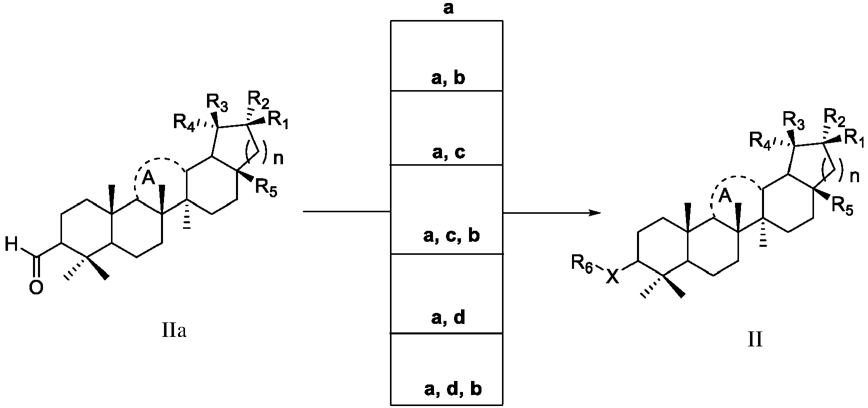 wherein, each group is defined as described in claim 1;
step a: reacting compound IV with compound III to obtain the product of step a;
step b: reducing the product of step a to obtain the product of step b;
step c: alkylating the product of step b to obtain the product of step c;
step d: reacting the product of step c with Des Martin reagent to obtain a compound of formula II;
wherein the structure of compound IV is:

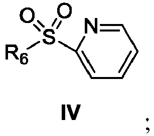

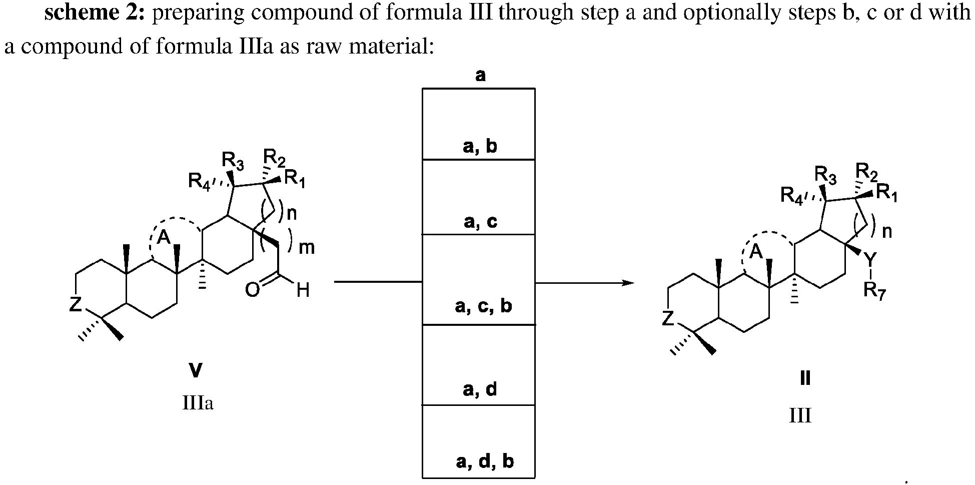

9. A pharmaceutical composition, wherein the pharmaceutical composition contains the therapeutically effective amount of one or more of the compound of formula I, or pharmaceutical salts, racemates, R-isomers and S-isomers thereof according to claim 1, as well as one or more pharmaceutical carriers, excipients, adjuvants, accessories and/or diluents.

10. A use of a compound of formula I and formula II, racemates, R-isomers, S-isomers or pharmaceutical salts thereof according to claim 1 for preparing a medicament for treating or preventing metabolic diseases related to diabetes and viral diseases; preferably, the diseases are selected from the group consisting of diabetes, influenza, obesity, liver fibrosis, metabolic diseases, and viral diseases.
